(19) Europäisches Patentamt
European Patent Office
Office européen des brevets

(11) Numéro de publication : 0 361 990 B1

# (12) FASCICULE DE BREVET EUROPEEN

(45) Date de publication du fascicule du brevet : **24.02.93 Bulletin 93/08**

(51) Int. Cl.$^5$ : **C07C 211/28,** C07C 255/42

(21) Numéro de dépôt : **89402198.9**

(22) Date de dépôt : **02.08.89**

## (54) Benzylamines disubstituées, leur procédé de préparation, leur utilisation comme médicament et leurs intermédiaires de synthèse.

(30) Priorité : **01.09.88 FR 8811450**

(43) Date de publication de la demande : **04.04.90 Bulletin 90/14**

(45) Mention de la délivrance du brevet : **24.02.93 Bulletin 93/08**

(84) Etats contractants désignés : **AT BE CH DE ES FR GB GR IT LI LU NL SE**

(56) Documents cités :
**SYNTHESIS, vol. 2, fevrier 1979, pages 127-129; H. AHLBRECHT et al, "Dehydrocyanation of Alpha-Aminonitriles; A Versatile andConvenient Enamine and Dieneamine Synthesis"**
**JOURNAL OF THE CHEMICAL SOCIETY, Perkin Transactions I, 1980, pages 1450-1457; R. W. JEMISON et al, "Base Catalyse-dRearrangements Involving Ylide Intermediates. Part 2. The Stevens (1,2) and (3,2) Sigmatropic Rearrangements of Allylic AmmoniumYlides"**
**JOURNAL OF THE CHEMICAL SOCIETY, Perkins Transactions I, 1980, pages 1458-1461; R. W. JEMISON et al, "Base CatalysedRearrangements involving Ylide Intermediates. Part 3. A Novel Thermal (1,3) Sigmatropic Rearrangement"**

(56) Documents cités :
**CHEMICAL ABSTRACTS, vol. 70, no. 9, 3 mars 1969, page 367, colonne 2, abrégé no. 37897u, Columbus, Ohio, USA; L. MIGINIAC etal, "Reaction of substituted alpha,beta-unsaturated organometallic compounds with aldimines"**
**CHEMICAL ABSTRACTS, vol. 79, no. 15, 15 octobre 1973, page 358, colonne 1, abrégé no. 91230v, Columbus, Ohio, USA; B MAUZE etal, "Reversibility of the reaction between alpha-ethylenic organometallics and simple aldimines"**

(73) Titulaire : **JOUVEINAL S.A.**
**Tour Maine Montparnasse 33 Avenue du Maine**
**F-75755 Paris Cedex 15 (FR)**

(72) Inventeur : **Aubard, Gilbert**
**7 Chemin de la Savetière**
**F-91120 Palaiseau (FR)**
Inventeur : **Calvet, Alain**
**56 Avenue du Colonel de Rochebrune**
**F-92380 Garches (FR)**
Inventeur : **Gouret, Claude**
**34, rue Croix du Val**
**F-92190 Meudon (FR)**
Inventeur : **Grouhel, Agnès**
**2, rue des Peupliers**
**F-92190 Meudon (FR)**
Inventeur : **Jacobelli, Henri**
**65 Avenue du Général de Gaulle**
**F-91550 Paray vieille Poste (FR)**
Inventeur : **Junien, Jean-Louis**
**36 Avenue Eiffel**
**F-92310 Sevres (FR)**

(74) Mandataire : **Bourgognon, Jean-Marie et al**
**Cabinet Flechner 22, Avenue de Friedland**
**F-75008 Paris (FR)**

EP 0 361 990 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles benzylamines α, α-disubstituées, leur procédé de préparation et leur utilisation sous forme de médicaments en thérapeutique, ainsi que des intermédiaires utiles pour leur synthèse.

Ces benzylamines répondent à la formule :

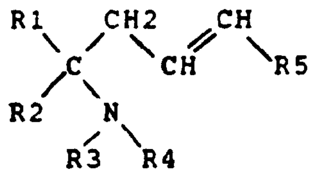

(I)

dans laquelle:

- R1 est phényle éventuellement mono ou di-substitué par des atomes d'halogène, par des radicaux alkyle inférieurs, haloalkyle inférieurs ou alkoxy inférieurs,
- R2 est alkyle inférieur,
- R3 et R4, identiques ou différents, sont l'hydrogène, des radicaux alkyle inférieurs ou alkènyle inférieurs,
- R5 est phényle éventuellement mono, di- ou tri-substitué par des atomes d'halogène ou par des radicaux alkoxy inférieurs.

Dans les définitions précédentes, le qualificatif "inférieur" comprend les radicaux ayant de 1 à 5 atomes de carbone en chaîne linéaire ou ramifiée. Par ailleurs, les significations des radicaux R1 à R5 qui suivent sont particulièrement appropriées aux composés (I) :

- R1 est phényle pouvant être mono ou di substitué par des atomes d'halogène comme le chlore, des radicaux alkyle inférieurs tels que méthyle, haloalkyle inférieurs comme trifluorométhyle ou alkoxy inférieurs comme méthoxy,
- R3 et R4 identiques ou différents sont l'hydrogène, alkyle inférieur comme méthyle, éthyle, propyle ou encore alkènyle inférieur comme allyle
- R5 est phényle éventuellement mono à trisubstitué par des atomes d'halogène comme le chlore, ou par des radicaux alkoxy inférieurs comme méthoxy.

L'invention comprend également les sels d'addition des benzylamines de formule (I) avec les acides minéraux ou organiques. Parmi ces sels, ceux obtenus par réaction des benzylamines avec des acides thérapeutiquement acceptables sont préférés. Par exemple, ceux préparés avec les acides acétique, benzènesulfonique, camphosulfonique, citrique, éthane sulfonique, fumarique, bromhydrique, chlorhydrique, lactique, maléique, malique, méthanesulfonique, mucique, nitrique, pamoïque, phosphorique, salicylique, stéarique, succinique, sulfurique, tartrique.

La formule (I) présente un atome de carbone asymétrique qui a pour conséquence l'existence pour chaque composé de formes racémiques et d'énantiomères optiquement actifs qui font partie intégrante de l'invention.

De même, d'autres structures isomères pouvant être consécutives à des significations particulières des radicaux R1 à R5 ou à leur association font également partie de l'invention de même que les formes salifiées de l'ensemble de ces composés ainsi que leurs formes solvatées.

L'ensemble des produits (I) de l'invention sont peu toxiques chez l'animal et présentent d'intéressantes propriétés psychotropes qui justifient leur utilité en thérapeutique sous forme de médicaments.

Toutefois, pour cette application, les composés préférés sont ceux dans lesquels R1 est phényle, R5 est phényle, R2 est éthyle, R3 et R4 semblables ou différents sont l'hydrogène ou méthyle et plus particulièrement ce sont les benzylamines (I) suivantes :

- α-cinnamyl-α-éthyl-benzylamine, racémique (±/-),lévogyre (-), dextrogyre (+) et leurs sels,
- α-cinnamyl-α-éthyl-N-méthyl-benzylamine racémique (±/-), lévogyre (-), dextrogyre (+) et leurs sels,
- α-cinnamyl-N,N-diméthyl-α-éthyl-benzylamine racémique (±/-), lévogyre (-), dextrogyre (+) et leurs sels,

Par ailleurs, il est également distingué un groupe de composés (IA) de formule (I) dans laquelle au moins un des radicaux R1 et R5 est un phényle substitué et R3 et R4 qui sont identiques ou différents sont l'hydrogène ou alkyle inférieur, R3 et R4 n'étant pas tous deux l'hydrogène qui, outre les propriétés psychotropes annoncées, montre "in vitro" une affinité pour les récepteurs opiacés et plus particulièrement les récepteurs mu et kappa. Cette affinité, se traduit "in vivo" chez l'animal par une action sur la motricité de la vessie qui font que ces produits (IA) ont une utilité aux traitements des dysfonctionnements urinaires.

Les benzylamines préférées de ce groupe sont

- α-cinnamyl-N,N-diméthyl-α-éthyl-p.chlorobenzylamine

- α-cinnamyl-N,N-diméthyl-α-éthyl-p.méthylbenzylamine
- α-(3',4'-dichloro)cinnamyl-N,N-diméthyl-α-éthyl-p.méthylbenzylamine

L'invention vise également un procédé de préparation des benzylamines de formule (I), caractérisé en ce qu'il consiste essentiellement et tel que décrit au schéma 1 :

- pour obtenir une benzylamine de formule (I.1), répondant à la formule (I), dans laquelle R3 et R4 sont l'hydrogène, à hydrolyser un isocyanate de formule :

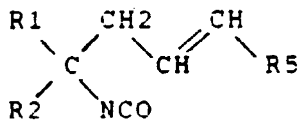
(II.1)

- pour obtenir une benzylamine de formule (I.2), répondant à la formule (I) dans laquelle R3 est méthyle et R4 est l'hydrogène,
  i) à réduire par un hydrure métallique un isocyanate de formule (II.1) ou,
  ii) à acyler une benzylamine (I.1) par l'acide formique en présence de carbonyldiimidazole en un composé intermédiaire N-formyl :

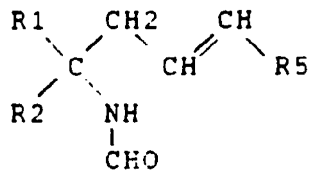

  puis à réduire cet intermédiaire par un hydrure métallique,
- pour obtenir une benzylamine de formule (I.2), répondant à la formule (I) dans laquelle R3 est alkyle inférieur différent de méthyle ou alkényle inférieur et R4 est l'hydrogène,
  i) à alkyler une benzylamine de formule (I.1) par un halogénure Z1R3 dans lequel R3 a les significations précédemment énoncées et Z1 est le chlore, le brome ou l'iode, ou
  ii) à acyler une benzylamine (I.1) par un réactif (R6-CO)n Z2 dans lequel R6 est l'homologue carboné directement inférieur à R3 (R3=-CH2R6) et n a pour valeur 1 lorsque Z2 est un halogène comme le chlore ou le brome ou lorsque Z2 est un radical hydroxyle, et n a pour valeur 2 lorsque Z2 représente un atome d'oxygène pour obtenir un carboxamide intermédiaire de formule

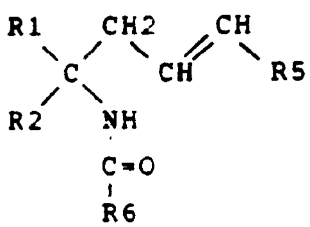
(II.2.1)

  puis à réduire par un hydrure métallique la fonction carboxamide de l'intermédiaire
- pour obtenir une benzylamine de formule (I.3) dans laquelle R3 et R4 identiques sont méthyle, à diméthyler une benzylamine (I.1) en la faisant réagir avec le formaldéhyde et, soit l'acide formique, soit un hydrure réducteur métallique ou organo-métallique
- pour obtenir une benzylamine de formule (I.3), répondant à la formule (I) dans laquelle R3 est différent de l'hydrogène et R4 est alkyle inférieur excepté méthyle ou est alkényle inférieur.
  i) à acyler une benzylamine de formule (I.2) dans laquelle R3 est différent de l'hydrogène par un agent d'acylation de formule R7CO Z5 dans laquelle R7 est l'homologue immédiatement inférieur de R4 (R4 = -CH2-R7) et Z5 représente le brome ou le chlore pour obtenir un carboxamide intermédiaire :

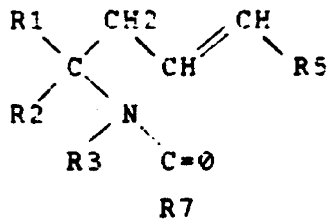

(II.2.2.)

puis à effectuer une réduction de la fonction carboxamide par un hydrure métallique,

ii) ou a faire réagir un réactif organomagnésien R2MgZ3 dans lequel R2 est alkyle inférieur et Z3 un atome de chlore, de brome, ou d'iode sur un amino-nitrile de formule :

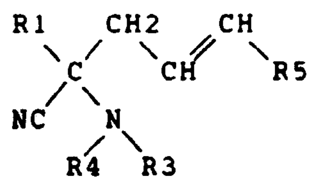

(II.3)

dans lequel R3 et R4 identiques ou différents sont alkyle ou alkényle inférieurs.

- pour obtenir une benzylamine de formule (I.3) répondant à la formule (I) dans laquelle R3 est alkyle ou alkényle inférieur et R4 est méthyle, à N-méthyler une benzylamine de formule (I.2.) dans laquelle R3 est alkyle ou alkényle inférieur par l'aldéhyde formique en présence d'un réducteur tel qu'un hydrure métallique ou organo-métallique.

La présente invention concerne également au titre de composés intermédiaires utiles à la préparation des benzylamines (I), les composés de formules II.1, II.2 et II.3 :

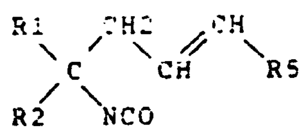

(II.1)

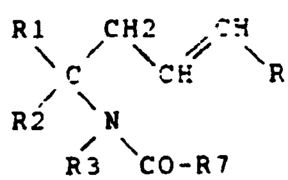

(II.2)

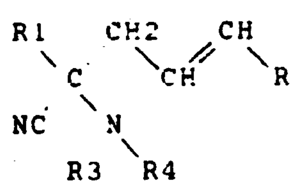

(II.3)

dans lesquelles:

- pour les isocyanates (II.1) les significations de R1, R2, R5 sont identiques à celles définies pour la formule (I),
- pour les carboxamides (II.2), les significations de R1, R2, R3, R5 sont identiques à celles définies pour (I), R7 étant l'hydrogène, un radical alkyle inférieur, alkènyle inférieur,
- pour les nitriles (II.3), R1, R5 ont les significations identiques à celles définies pour (I), R3 et R4 également, excepté l'hydrogène.

Le procédé de préparation des composés intermédiaires consiste :

- pour préparer les composés (II.1), à alkyler un composé (VIII) R1-CH2-W dans lequel R1 est tel que défini pour (I) et W est un radical nitrile (-CN) ou carboxyle (-COOH) par un halogènure d'alkyle de formule R2Z6,

R2 étant tel que défini pour (I) et Z6 étant un halogène, pour obtenir, pour W = -COOH un acide de formule (VI) R1(R2)-CH-COOH.

et, pour W = -CN, obtenir un nitrile de formule (VII) R1(R2)-CH-CN qui est hydrolysé en acide (VI), puis à alkyler l'acide (VI) par un halogènure d'alkényle (V) de formule 27-CH2-CH=CH-R5, Z7 étant un halogène et R5 tel que défini pour (I) pour obtenir les acides (III) R1(R2)C(COOH)CH2- CH=CH-R5 puis à préparer les isocyanates (II.1) par la réaction de Curtius a.2) à partir de ces acides.

- pour préparer les composés (II.2),
  - i) dans lesquels R3 est l'hydrogène et qui correspondent alors aux composés de formule (II.2.1) déjà présentés, à acyler une benzylamine de l'invention de formule (I.1) par un réactif (R6-CO)nZ2 déjà défini afin d'obtenir le carboxamide intermédiaire (II.2) dans lequel R3 est l'hydrogène et R7 correspond à R6 du réactif précédemment cité, et,
  - ii) dans lesquels R3 est alkyle ou alkényle inférieur, et qui correspondent alors aux composés de formule (II.2.2), à acyler une benzylamine de formule

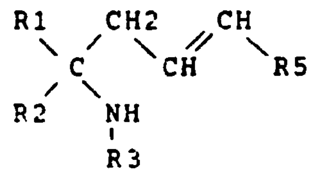

par un halogènure de formule R7COZ5, dans laquelle R7 est hydrogène, alkyle inférieur ou alkényle inférieur et Z5 est le brome ou le chlore et,

- pour préparer les composés (II.3) à faire réagir un aldéhyde de formule (XI) R1-CHO avec une amine de formule R3-NH-R4 et un cyanure de métal alcalin pour obtenir un aminonitrile intermédiaire de formule (X) R1(CN)CH-N(R3)R4 dans lequel R1 a les définitions présentées pour (I) R3 et R4 ayant également les définitions de (I), excepté l'hydrogène, puis à alkyler l'aminonitrile (X) par un halogénure d'alkényle (V) déjà décrit.

SCHEMA 1 :

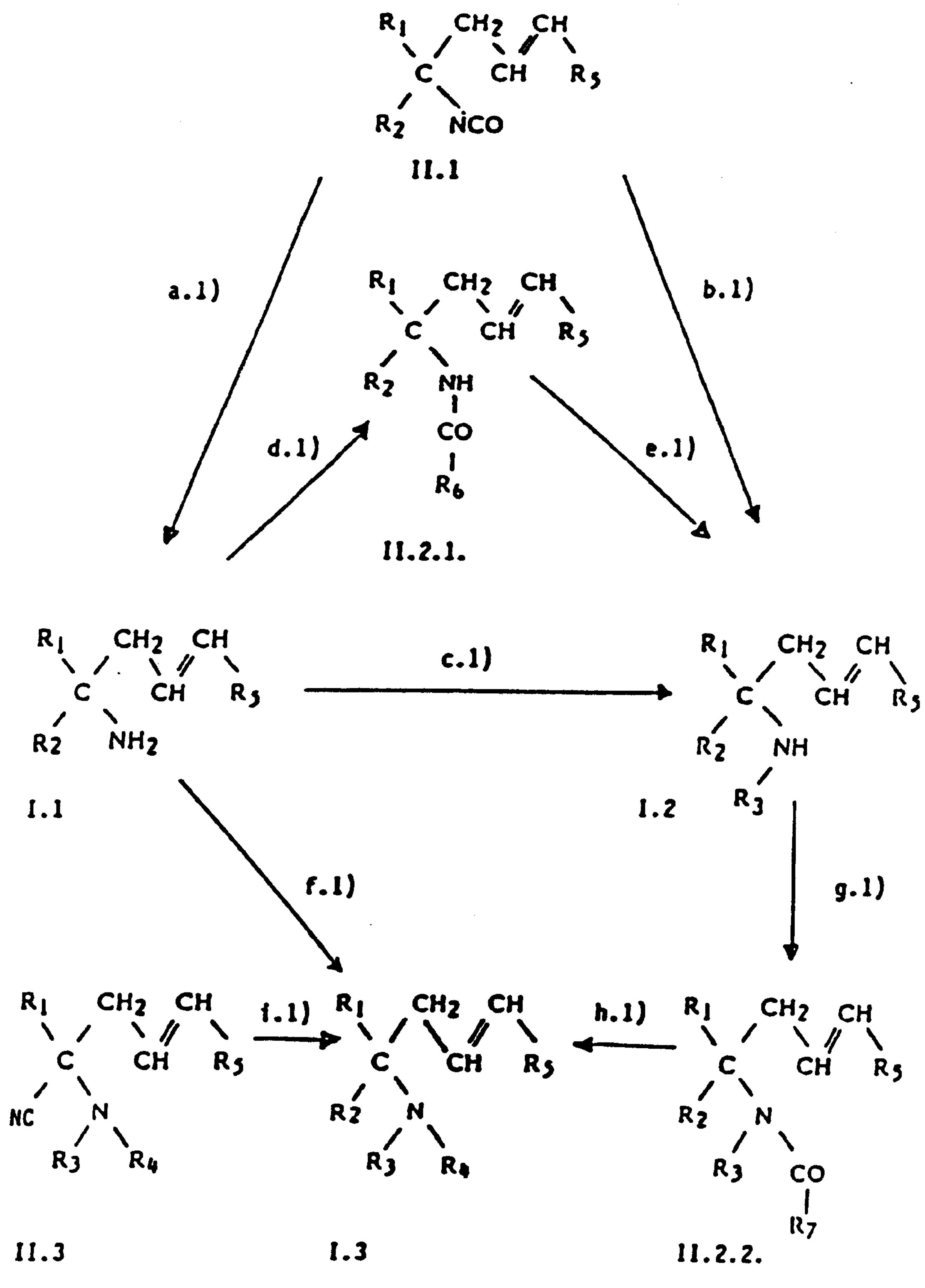


II.1

II.2.1.

I.1

I.2

II.3

I.3

II.2.2.

Les composés (I) de l'invention se differencient des composés connus les plus proches de par leur structure chimique et également de par leur application. Ainsi, L. Miginiac et B. Mauzé dans Bull. Soc. Chim. Fr., 1968, (9), p. 3832-44 puis Bull. Soc. Chim. Fr., 1973, (5) (Pt. 2), p. 1832-8 rapportent, au cours de l'étude de la réaction de dérivés organométalliques α-éthyléniques substitués sur des aldimines la préparation du N-méthylamino-1-diphényl-1,4-butène-3 de formule C6H5-CH-(NH-CH3)-CH2-CH=CH-C6H5 sans en indiquer d'application.

Par ailleurs, R.W. Jemison et coll. dans J. Chem. Soc., Perkin Trans. I., 1980, p. 1450-7 et 1458-61, au cours de l'étude de réarrangements de structures impliquant des intermédiaires de type "ylide" et catalysés par des bases, obtiennent et décrivent un produit f) à la page 1451 et à la page 1454, qui est le N,N-diméthylamino -1(p-nitrophényl)-1 phényl-4 butène-3 sans en indiquer d'application.

Ces produits diffèrent des benzylamines suivant l'invention par le fait que leur atome de carbone en position alpha dans la séquence benzylamine n'est que tri-substitué, alors que celui des benzylamines suivant l'invention portent, en plus, un radical alkyle. En outre, aucune activité pharmacologique susceptible d'application thérapeutique n'est rapportée pour ces produits.

SCHEMA 2 :

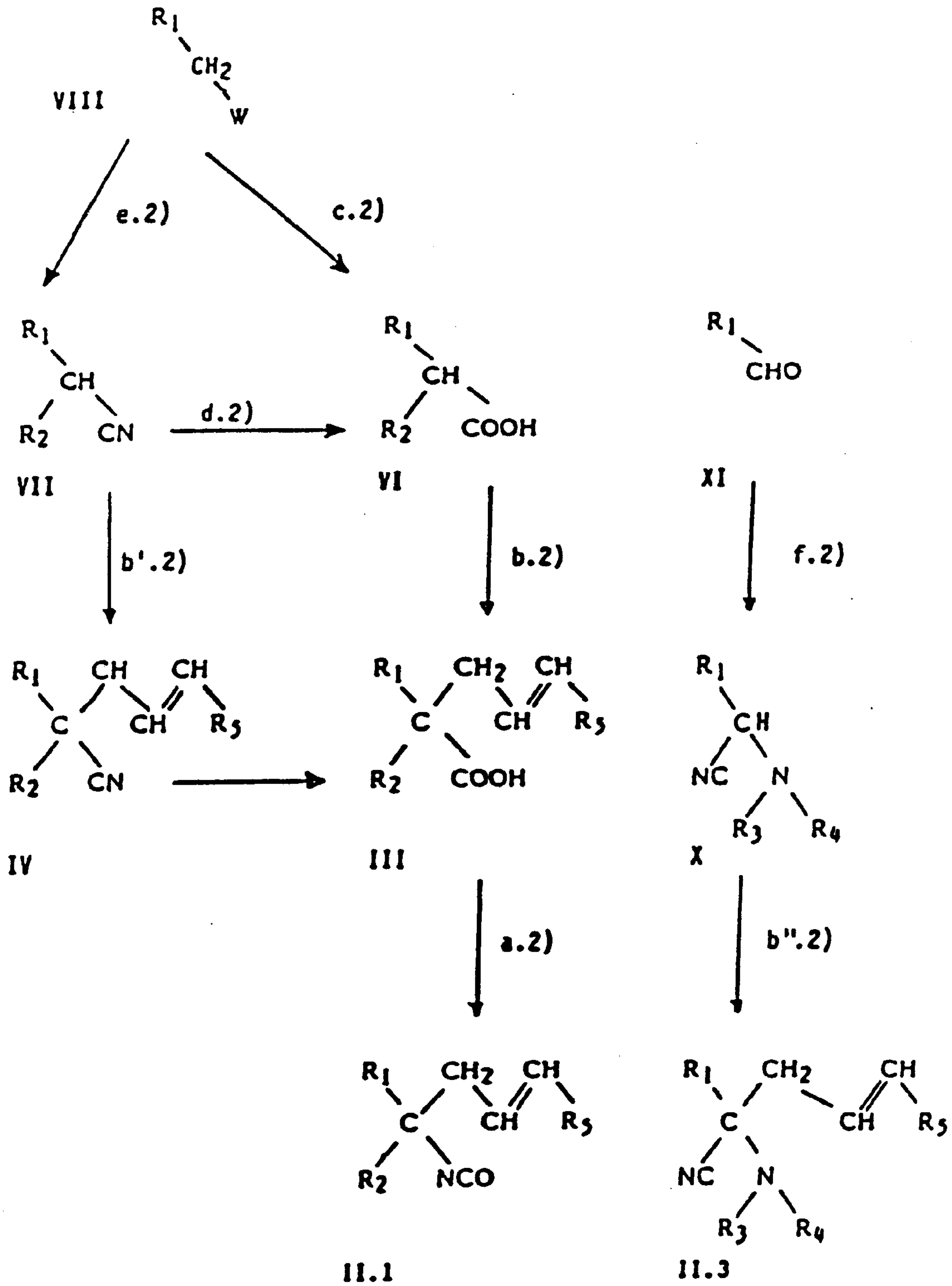


VIII, VII, VI, XI, IV, III, X, II.1, II.3

Indépendamment du procédé de préparation des composés de formule (I), l'invention vise egalement la

préparation des intermédiaires (II.1), (II.2) et (II.3).

Celle des composés (II.2) qui comprend les produits de formule (II.2.1) et (II.2.2) est indiquée au schéma 1. Le procédé de préparation de (II.1) et (II.3) est indiqué au schéma 2 précédent et consiste :

- à préparer les composés intermédiaires de formule II.1 à partir de composés (VIII) de formule R1-CH2-W dans lesquels R1 est défini comme pour (I) et W est un radical nitrile (-CN) ou encore le radical carboxyle (-COOH) qui sont soumis à deux alkylations successives d'abord par un halogénure d'alkyle inférieur R2Z6 puis par un halogénure d'alkényle (V) préparé tel qu'indiqué au schéma 3 et de formule Z7-CH2-CH=CH-R5 dans lesquels R5 a les significations décrites pour (I) et 27 est un atome d'halogène tel que le chlore ou le brome,
- à préparer les composés intermédiaires de formule (II.3) par cyano-amino deoxo substitution selon la synthèse de Strecker de benzaldéhydes R1-CHO (XI) et d'amines R3-NH-R4, composés dans lesquels R1 est un phényle tel que décrit pour (I), R3 et R4 sont les radicaux décrits pour (I) excepté l'hydrogène en présence d'un cyanure alcalin pour obtenir un amino nitrile (X) qui est ensuite soumis à une alkylation par un halogénure d'alkényle (V) pour obtenir l'intermédiaire (II.3) .

SCHEMA 3 :

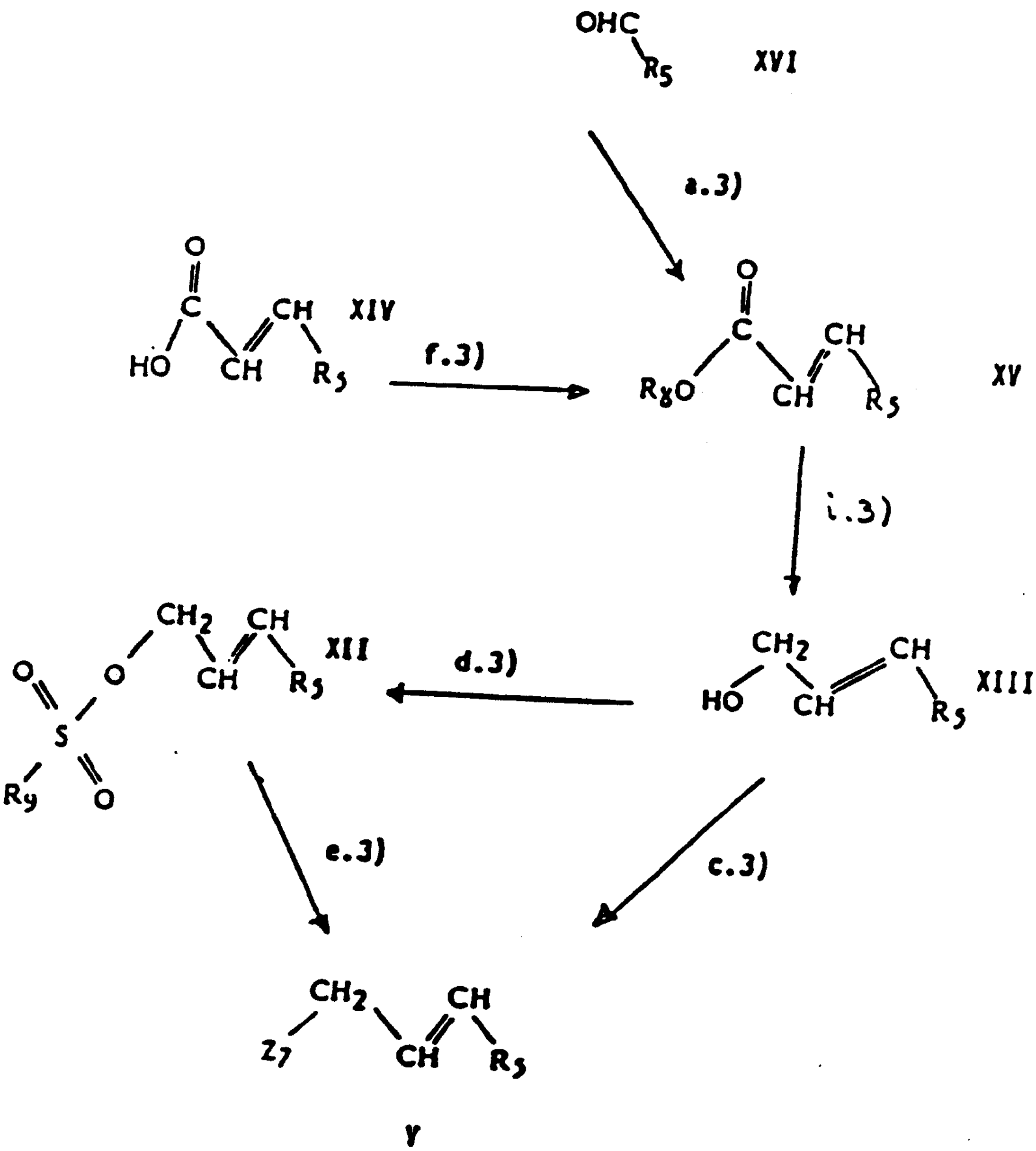


Le procédé de préparation des intermédiaires est illustré aux schémas 2 et 3 et est dans le détail décrit dans ce qui suit :

- pour obtenir comme il est indiqué au schéma 3, un composé (V) dans lequel R5 a les valeurs définies pour (I), et Z7 est un halogène tel que le brome ou le chlore, le procédé consiste :

i) à faire réagir un dérive carbonylé (XVI), R5-CHO dans lequel R5 a les valeurs définies pour (I), avec un phosphonoacétate de trialkyle en présence d'hydrure métallique selon la réaction a.3) de Wittig-Horner-Emmons pour obtenir un ester (XV) dans lequel R8 est alkyle inférieur.

Comme il est décrit dans "Organic Synthèses", Coll. vol. V p. 509, la réaction consiste à introduire à une température comprise entre 0° et 20°C et en une durée comprise entre 5 minutes et 1 h 30, un phosphonoacétate de trialkyle inférieur à une suspension d'hydrure métallique dans un solvant dipolaire, puis à ajouter le dérivé carbonylé (XVI) et à chauffer le milieu réactionnel de 2h à 8 jours puis à séparer l'ester insaturé (XV)

obtenu. La méthode préférée consiste pour une mole de (XVI) à utiliser de 0,8 à 1,2 moles de phosphonoacétate de triéthyle et 0,8 à 1,2 moles d'hydrure de sodium et ce en rapport équimoléculaire entre eux. Ainsi, selon a.3) le phosphonoacétate de triéthyle est ajouté entre 0° et 10°C à la suspension d'hydrure de sodium dans le 1,2-diméthoxyéthane, en 30 minutes environ, puis le dérivé carbonylé est introduit et le mélange porté sous agitation à une température voisine de 85°C durant 3 à 5 jours.

L'ester (XV) formé est isolé et purifié, puis réduit parun hydrure métallique ou métallo organique selon la réaction b.3) dans des solvants comme les éthers, les hydrocarbures aliphatiques ou aromatiques.

Les éléments métalliques de ces hydrures sont l'aluminium, le bore et les métaux alcalins sont le lithium et le sodium. Au titre d'agents réducteurs préférés on utilise l'hydrure de lithium-aluminium (LAH) en milieu éthéré dans le tetrahydrofurane (THF) ou l'hydrure de di-isobutyl-aluminium (DIBAL-H) (R) dans les hexanes, le THF ou encore le toluène qui est le solvant préféré pour cet agent. Ainsi pour réduire une mole d'ester (XV) il est utilisé la quantité suffisante d'hydrure contenant de 1,2 à 10 équivalents d'hydrogène actif, la réaction étant effectuée à une température comprise entre -70° et 40°C et en une à huit heures. De cette manière, une solution toluènique contenant 1,0 mole d'ester est refroidie à une température voisine de -50°C puis une solution toluènique contenant de 2,0 à 2,5 moles de DIBAL-H est introduite en une heure environ et en maintenant à cette température. La réaction est poursuivie durant deux heures environ à -50°C puis l'alcool (XIII) formé est isolé, purifié, et engagé dans les réactions de préparation du dérivé halogéné (V) par des méthodes classiques décrites par exemple dans "Advanced Organic Chemistry" J. March, 3ème ed. (Wiley) p.382-383, soit en une étape selon la réaction c.3) soit en deux étapes selon les réactions d.3) et e.3) avec pour intermédiaire l'ester sulfonique (XII).

La réaction c.3) peut consister à faire réagir sur l'alcool des haloacides ou des halogènures d'acides inorganiques. Il est également possible dans la même réaction de former un dérivé activé intermédiaire de l'alcool, dérivé non isolé qui, par réaction "in situ" avec un halogènure de métal alcalin comme par exemple le chlorure, bromure, iodure de lithium ou de sodium conduit au dérivé (V). Cette réaction consiste à faire réagir sur l'alcool (XIII), dans un solvant aprotique, un halogènure de trialkyle ou de triaryle silane pour former le dérivé activé qui réagit ensuite avec l'halogènure alcalin ; les réactifs préférés étant les chloro-trialkylsilanes et les halogènures de lithium. Ainsi la réaction c.3) consiste à introduire sous atmosphère d'azote et à la température du laboratoire, une mole d'alcool dans une solution d'acétonitrile contenant de 1,8 à 2,2 moles de bromure de lithium et de 2,35 à 2,75 mole de chloro triméthylsilane. La réaction est poursuivie au reflux du solvant de 2 à 24 heures puis le dérivé bromé (V) formé est isolé et éventuellement purifié.

La préparation des composés (V) par l'intermédiaire de l'ester sulfonique (XII) dans lequel R9 est alkyle aliphatique inférieur ou aryle éventuellement substitué et R5 est déja défini, par les réactions successives d.3) et e.3) consiste à préparer l'ester sulfonique (XII) selon d.3) en ajoutant à une mole d'alcool (XIII) en solution dans un solvant halogéné tel que le chlorure de méthylène, de 0,8 à 1,2 mole d'un agent accepteur d'acide, minéral ou organique, comme de préférence la triéthylamine ou la 4-diméthylaminopyridine, puis à une température comprise entre -20 et +30°C ajouter de 0,8 à 1,2 mole d'un halogènure d'acide sulfonique R9-SO2-Z4 dans lequel R9 est décrit plus haut et Z4 est un halogène qui est généralement le chlore et, après réaction et traitements, à isoler l'ester sulfonique (XII) et à le purifier par exemple par distillation sous pression réduite, puis selon la réaction e.3) à obtenir le dérivé (V) par halo-désulfonyloxy substitution qui consiste à faire réagir l'ester (XII) dans un solvant aprotique avec un halogènure dans lequel l'halogène est plus particulièrement le chlore ou le brome associé au sodium ou au lithium. Le mélange est porté au reflux du solvant de 4 h. à 7 jours pour obtenir une réaction complète. D'une manière préférée, pour une mole de composé (XII) en solution dans 2,5 à 3,5 l. d'acétone, on ajoute de 1,4 à 1,8 mole de bromure de lithium. Après 24 h. à 4 jours de réaction au reflux le dérivé bromé (V) obtenu est isolé et purifié par distillation.

ii) à partir d'un acide insaturé (XIV) à estérifier cet acide par un alcool R8OH dans lequel R8 est alkyle aliphatique inférieur pour obtenir un ester (XV).

La méthode d'estérification préférée f.3) consiste à utiliser comme catalyseur un acide de Lewis tel qu'un trihalogènure de bore et plus particulièrement le complexe trifluorure de bore - éther diéthylique. De façon habituelle à une mole d'acide en solution dans 1 à 10 l d'alcool R8OH, il est ajouté 0,75 à 1,50 mole de complexe BF3-éther. La solution est chauffée au reflux de 2 à 48 h puis traitée pour obtenir l'ester (XV) qui, éventuellement purifié, est ensuite engagé dans les réactions b.3) à e.3) déja décrites.

- pour obtenir, tel qu'il est montré au schéma 2, un intermédiaire de l'invention de formule (II.1) :

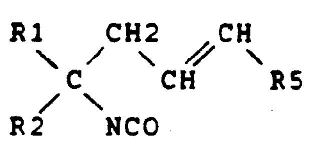

(II.1)

dans laquelle R1, R2, R5 ont les valeurs indiquées pour (I)

i) soit à monoalkyler par un halogènure d'alkyle R2-Z6 dans lequel Z6 est halogène, un acide phénylacétique R1-CH2-COOH (VIII) pour obtenir un acide (VI): R1-(R2)CH-COOH puis à effectuer une seconde alkylation avec un dérivé halogéné (V), précédemment décrit et défini pour obtenir l'acide phénylacétique dialkylé (III) de formule :

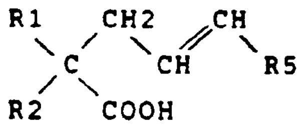

(III)

Les réactions d'alkylation sont réalisées par des méthodes connues comme celles présentées dans "Advanced Organic Chemistry" J. March, 3ème ed. ( Wiley ) p.421. qui consistent à alkyler les anions des acides obtenus par réaction de bases fortes sur les acides ou leurs sels.

Les bases fortes utilisées à cet effet peuvent être des dérivés métalliques ou organométalliques de métaux alcalins.

Ainsi, pour obtenir les acides (VI) et lorsque R1 ne comporte pas de substituant de nature halogènée comme des atomes de chlore ou des radicaux trifluorométhyl, à employer la méthode décrite dans "Journal of Organic Chemistry" 32, 9, p.2797-2803, 1967, qui consiste à préparer en solution dans le tetrahydrofurane le dianion de l'acide (VIII) par action de naphtalènate de sodium puis à faire réagir un halogènure qui est de préférence un dérivé iodé, pour obtenir selon la réaction c.2) l'acide (VI) puis, selon la réaction b.2) à réaliser la seconde alkylation de cet acide avec le dérivé (V) selon une méthode inspirée de celle décrite dans "Tetrahedron Lett." 1980, 21 (12) p.1169-72 qui consiste essentiellement pour former le dianion réactif a utiliser le diisopropylamide de lithium ( LDA ).

D'une manière plus précise, la première alkylation selon c.2) consiste à préparer d'abord le naphtalènate de sodium en milieu éthéré anhydre comme dans le THF par addition pour une mole de naphtalène en solution dans 0,5 à 1 l. de THF, de 0,9 à 1,1 mol de sodium puis à laisser la réaction se développer de 4 à 24 h. et plus favorablement de 12 à 18 h., à ajouter cette solution dans une autre solution de THF contenant de 0,3 à 0,5 mole d'acide (VIII) afin de former le dianion réactif par contact durant 1 à 24 h. à une température comprise entre I0 et 50°C. Habituellement la réaction est complète entre 3 et 5 h. à 20°C et il est introduit alors de 0,3 à 1,2 mole de dérivé halogéné R2-Z6 et plus précisément, de 0,45 à 0,75 mole de dérivé ou l'halogène Z6 est l'iode. La réaction est complète après agitation entre 1 et 48 h. à une température comprise entre 10 et 50°C. Plus favorablement le mélange est maintenu 16 à 20 h. à 20-30°C avant d'être traité pour obtenir le composé purifié (VI) attendu.

Ce composé est ensuite engagé dans la seconde réaction b.2) d'alkylation qui consiste à préparer "in situ" le LDA à partir de quantités équimoléculaires de diisopropylamine et de butyllithium puis, pour une mole de LDA ainsi préparé à ajouter dans le THF de 0,5 à 0,3 mole d'acide (VI) pour obtenir son dianion. Le dérivé halogéné (V) est ensuite introduit à une température comprise entre -10 et 50°C puis le mélange laissé à réagir de 2 à 48 h. selon la réactivité des composés.

Ainsi d'une manière préférée à une mole de diisopropylamine dans 500 ml de THF il est ajouté à -20°C environ de 0,95 à 1 mole de butyl lithium puis de 0,4 à 0,5 mole d'acide (VI) en solution dans environ 250 ml de THF. Après réaction de 1 à 2 heures entre 20 et 100°C pour former le dianion, le mélange est refroidi vers 0°C et on y ajoute de 0,4 à 0,5 mole du dérivé halogènè (V).

La réaction est développée durant 1 à 2 h. à température ambiante puis le mélange est traité pour isoler et purifier le dérivé (III) obtenu,

ii) soit à monoalkyler par un halogènure d'alkyle R2-Z6 précédemment décrit un phénylacétonitrile R1-CH2-CN (VIII) pour obtenir selon la réaction e.2) un phénylacétonitrile alkylé (VII) de formule R1(R2)-CH-CN dans lequel R1 et R2 ont les définitions décrites pour (I), Puis selon la réaction d'hydrolyse d.2) préparer l'acide (VI) qui est ensuite traité par la réaction b.2) comme décrit précédemment pour obtenir l'acide (III).

Cette préparation est préférée lorsque R1 est substitué par des atomes d'halogène en particulier de chlore ou par des radicaux haloalkyle inférieurs comme trifluorométhyle.

Pour ce faire, on utilise de préférence la méthode décrite dans "Il Farmaco" Ed. Sci. XXV (6) 1970, p.409-421 qui consiste selon la réaction e.2) à faire réagir un halogènure d'alkyle R2-Z6 sur un phénylacétonitrile (VIII) par une réaction utilisant un catalyseur dit de transfert de phase, en introduisant une mole d'acétonitrile dans une solution aqueuse de 2,5 à 3 moles de ce catalyseur comme le chlorure de benzyltriéthylammonium qui est préféré, puis 0,75 à 1 mol de dérivé R2-Z6 dans lequel Z6 est le brome ou le chlore.

Après réaction de 1 à 48 h. et, plus habituellement de 3 à 5 h. le mélange est traité et le phénylacétonitrile

monoalkylé est purifié, généralement par distillation sous pression réduite. Ce nitrile selon la réaction d.2) est hydrolysé, d'abord par l'acide bromhydrique en milieu éthanolique puis par une solution concentrée d'hydroxyde de sodium ainsi qu'il est décrit dans l'article cité.

iii) soit, selon la réaction b'.2), analogue à la réaction b.2) déjà décrite, préparer le dérivé dialkylé (IV) qui est ensuite hydrolysé pour obtenir l'acide (III).

La réaction d'alkylation b'.2) consiste essentiellement à n'utiliser que la moitié de la quantité de LDA engagée dans la réaction b.2), l'hydrolyse du nitrile (IV) obtenu par la réaction d.2) déja décrite conduit ensuite à l'acide (III).

Les produits intermédiaires de l'invention de formule (II.1) sont préparés, selon la réaction de Curtius a.2) à partir des acides (III) préparés tel qu'il vient d'être décrit en i), ii), et iii).

Des méthodes de réarrangement diverses (Hofman, Curtius et Lossen) permettent de préparer des isocyanates à partir de composés dérivés d'acides qui sont respectivement pour les réactions citées, les amides, les acides et les hydroxamates.

De façon préférée le procédé de préparation des composés II.1 de l'invention utilise la réaction de Curtius qui a été l'objet de publications indiquées par exemple dans la section "Organic Name Réactions" p.21 du "Merck Index" 10ème ed.. Elle consiste à partir d'un acide, à préparer successivement son chlorure puis l'azide correspondant et enfin par décomposition thermique de ce dernier à obtenir l'isocyanate souhaité.

La méthode avantageusement pratiquée permet de réaliser en une seule opération cette succession de réactions et consiste, dans un solvant halogéné apolaire à faire réagir l'acide (III) avec de l'azide de sodium en présence de dichlorophosphate d'alkyle ou d'aryle comme les dichlorophosphate d'éthyle ou de phényle et d'une trialkylamine comme la triéthylamine ou d'une amine aromatique comme la pyridine qui est préférée, puis à éliminer le solvant et procéder directement au réarrangement de l'azide formé par action de la chaleur.

Pratiquement, pour 1 mole d'acide en solution dans 3 à 20 litres de dichlorométhane on ajoute de 1 à 1,75 mol de dichlorophosphate de phényle puis de 2 à 3,5 mol d'azide de sodium et de pyridine en quantités équimoléculaires. La formation de l'azide est réalisée à une température comprise entre 10 et 40°C en agitant de 4 à 24 h selon la réactivité des produits.

Après traitements à l'eau et à l'acide chlorhydrique le dichlorométhane est éliminé par distillation tout en ajoutant un solvant inerte de point d'ébullition supérieur à 100°C apte à être utilisé comme solvant de la réaction de décomposition thermique de l'acide en isocyanate.

Plus favorablement, on utilise un solvant aromatique comme le toluène et la réaction de décomposition est effectuée à la température du reflux de ce solvant jusqu'à fin de dégagement gazeux ce qui nécessite une durée comprise entre 30 minutes et 8 heures.

L'isocyanate (II.1) est obtenu après évaporation du solvant puis éventuellement purifié.

- et, pour obtenir, tel qu'il est montré au schéma 1, les composés intermédiaires de l'invention de formule (II.2.1) et (II.2.2) répondant à la formule générale (II.2) :

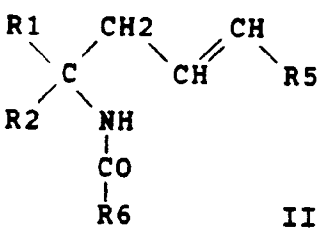

II.2.1.

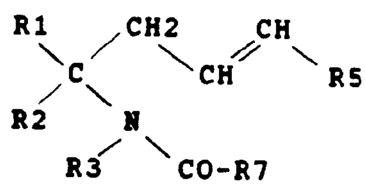

II.2.2

dans lesquelles R1, R2, R3, R5 ont les valeurs indiquées pour (I) et R6 et R7 sont l'hydrogène, alkyle ou alkènyle inférieur,

i) à acyler un composé de l'invention (I.1) dans lequel R3 et R4 sont l'hydrogène par la réaction d.1) pour obtenir un composé (II.2.1) dans lequel R4 est l'hydrogène et R6 un radical précédemment défini.

Cette acylation est réalisée par un agent de formule (R6CO)nZ2 dans lequel R6 déjà défini est l'homologue carboné directement inférieur au radical R3 (R3=CH2-R6) et dans lequel lorsque n=1 Z2 représente un atome d'halogène tel que le chlore ou brome, excepté lorsque R6 est hydrogène, ou Z2 représente un hydroxyle, et, lorsque n-2 Z2 représente un atome d'oxygène,

ii) ou à acyler par la réaction g.1) une benzylamine de l'invention (I.2) dans laquelle R3 ou R4 est l'hydrogène, l'autre étant alkyle ou alkènyle inférieur pour obtenir un composé (II.2.2), dans lequel R1, R2, R3 et R7 sont précédemment définis.

L'acylation est alors réalisée par un agent de formule R7C0Z5 dans lequel R7 est excepté l'hydrogène,

l'homologue carboné directement inférieur à R4 (R4=CH2-R7) et dans lequel Z5 représente un halogène tel que le chlore ou le brome.

Lorsque les réactions d'acylation d.1) et g.1) font intervenir un réactif dns lequel Z2 ou Z5 sont un halogène les réactions sont réalisées en milieu monophasique anhydre en présence d'une base organique ou en milieu biphasique en présence d'un hydroxyde de métal alcalin.

La réaction préférée s'effectue en milieu monophasique dans le toluène ou plus favorablement le dichlorométhane, et consiste à ajouter dans une solution contenant une mole de dérivé à acyler de 1,0 à 1,5 mol d'amine qui est généralement la triéthylamine, puis à ajouter un composé R6-COZ2 ou R7-COZ5 dans lequel Z2 ou Z5 sont halogène et ce en quantité équimoléculaire à la triéthylamine. La solution est ensuite maintenue de 3 à 48 h à une température comprise entre 15 et 30°C de façon à obtenir la réaction la plus complète possible.

Par ailleurs, pour obtenir des composés de formule (II.2.1) dans lesquels R6 est l'hydrogène on utilise de préférence la méthode d'acylation qui consiste à faire réagir sur les composés l'acide formique en présence de 1,1′-carbonyldiimidazole en rapport équimoléculaire par rapport à l'acide et dans un solvant apolaire comme le tetrahydrofurane.

Favorablement pour une mole de produit à acyler, on engage de 1 à 1,1 mole de chacun des réactifs, la réaction s'effectuant en milieu homogène à une température comprise entre 15 et 30°C et ce durant 1 à 48 h pour obtenir une réaction complète.

Lorsque la réaction d'acylation consiste à faire réagir un composé I.1 sur un agent dans lequel Z2 est hydroxyle, n est égal 1 et R6 est différent de l'hydrogène une méthode consiste à préparer in situ un anhydride mixte comprenant ce réactif puis à acyler l'amine I.1 par cet intermédiaire.

Favorablement, la réaction est effectuée dans des solvants apolaires anhydres de la classe des éthers oxydes. Le tétrahydrofurane est préféré et on forme d'abord l'anhydre mixte à une température comprise entre -40 et 0°C en ajoutant pour une mole de réactif de 1,0 à 1,5 mole d'amine tertiaire comme la N-méthylmorpholine puis de 0,9 à 1,2 mole de chloroformiate d'isobutyle, on ajoute ensuite une mole du dérivé (I.1) à acyler et laisse la réaction se développer de 1 à 48 heures à une température comprise entre 0 et 60°C. Habituellement, le résultat de la réaction est satisfaisant à une température comprise entre 10 et 25°C après une durée de 10 à 20 heures.

Dans des conditions voisines, il est aussi possible de préparer "in situ" un anhydre de l'agent d'acylation acide, par la dicyclohexylcarbodimide puis à laisser réagir l'anhydride formé sur le dérivé aminé (I.1).

Enfin, lorsque le réactif d'acylation est un anhydride c'est-à-dire lorsque n=2 et Z2 est l'oxygène, la réaction, lorsque le point d'ébullition de l'anhydride est inférieur à 140°C peut s'effectuer sans solvant, en faisant réagir le composé I.1 dans un large excès et à la température de reflux du réactif. La méthode préférée consiste cependant à réaliser la réaction en utilisant comme solvant la pyridine et en faisant réagir pour une mole de composé à acyler, de 1 à 5 mole d'anhydride.

Couramment, l'utilisation de 1,2 à 1,8 mole d'anhydride au reflux de la pyridine durant 1 à 3 heures conduit à des résultats convenables. Les dérivés (II.2.1) ou (II.2.2) obtenus sont, si leur état le nécessite, purifiés par les méthodes habituelles, ou engagés tels quels dans les réactions de réductions qui suivent pour conduire aux benzylamines de l'invention de formules (I.2) et (I.3) comme il est montré au schéma 1.

- et, pour préparer comme il est décrit au schéma 2 un amino nitrile produit intermédiaire de l'invention de structure (II.3) :

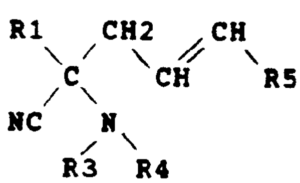

(II.3)

dans lequel R1 et R5 ont les valeurs définies pour I, R3 et R4 ayant également les valeurs définies pour I excepté l'hydrogène, à :

i) faire réagir selon la réaction f.2) un aldéhyde (XI) de formule R1-CHO avec une amine secondaire R3-NH-R4 pour obtenir l'amino nitrile (X) de formule NC(R1)-CH-N (R3)(R4).

Cette réaction est fréquemment utilisée pour la préparation d'amino-acides selon la méthode de Strecker, elle est appliquée à la synthèse des composés (X) et consiste à faire réagir 1 mole de benzaldéhyde (XI) avec 0,8 à 3,0 mol de cyanure de sodium ou de potassium et avec 0,8 à 3,0 mol d'un sel d'amine secondaire de formule R3-NH-R4 en milieu alcoolique ou hydro-alcoolique, à une température comprise entre 5°C et la température de reflux du milieu et pendant de 1 à 24 h.

Les sels d'amine secondaires utilisés de préférence sont hydrosolubles comme les chlorhydrate, bromhydrate, sulfate...

Le solvant réactionnel comprend un alcool de bas poids moléculaire et miscible en toutes proportions avec l'eau comme le méthanol ou l'éthanol. Dans le cas de milieux hydro alcooliques les proportions respectives sont comprises entre 95 et 10% d'alcool pour le complément à 100% par de l'eau, ce rapport permettant d'obtenir de façon favorable un milieu réactionnel homogène.

D'une manière courante à une solution de 1,1 à 1,3 mol de cyanure de sodium et de 1,1 à 1,3 mol de chlorhydrate d'amine secondaire en solution dans 150 à 400 ml d'eau il est ajouté 1,0 mol du benzaldéhyde (XI) en solution dans 75 à 200 ml de méthanol. Le mélange est agité durant 3 à 5 h à une température comprise entre 15 et 30°C puis traité pour isoler l'amino-nitrile (X) qui est si nécessaire purifié par distillation.

ii) puis à alkyler ce composé avec un dérivé halogéné de formule (V) déja décrit, pour obtenir selon la réaction b''.2) l'intermédiaire de l'invention (II.3).

Cette réaction b''.2) est analogue à la réaction b'.2) et fait appel aux mêmes réactifs qui sont utilisés dans des conditions opératoires identiques.

Bien que les procédés qui viennent d'être décrits fassent appel à des réactions connues, la préparation des principaux produits est décrite dans la partie expérimentale aux chapitres intitulés :

- "Produits utilisés dans la synthèse" pour les composés de formule (X), (VI), (V) et (III),
- "Exemples des produits de l'invention" décrits à l'exemple 1 pour les composés de formule (II.1) et à l'exemple 2 pour les produits de formule (II.3).

Au titre des procédés, l'invention vise particulièrement celui de préparation des composés de l'invention de formule (I) racémiques ou optiquement actifs qui peuvent être des benzylamines primaires (I.1) dans lesquelles R3 et R4 sont l'hydrogène ou secondaires (I.2) dans lesquelles R3 ou R4 sont l'hydrogène ou encore tertiaires (I.3) dans lesquelles R3 et R4 sont différents de l'hydrogène et ont les significations désignées pour la formule générale (I).

Le procédé de préparation des benzylamines de l'invention (I.1.) dans lequel R3 et R4 sont l'hydrogène consiste en l'hydrolyse, selon la réaction a.1) du schéma 1, des isocyanates intermédiaires de l'invention (II.1) précédemment décrits.

D'une manière générale, cette hydrolyse est catalysée par les acides ou les bases de préférence inorganiques tels que les acides bromhydrique, sulfurique, phosphorique et chlorhydrique qui est l'acide préféré ou les hydroxydes de métaux alcalins ou alcalino-terreux, les hydroxydes de sodium et de potassium étant préférés.

L'hydrolyse peut être pratiquée en milieu aqueux ou en présence d'un solvant miscible à l'eau et non réactif avec les composés de la réaction tel que des éthers comme les dioxanes et le tetrahydrofurane qui est le solvant préféré en mélange avec l'eau.

Ainsi pour une mole de dérivé (II.1) à hydrolyser la réaction est pratiquée en dissolvant le produit dans 0,5 à 10 litres de THF puis à ajouter de l'eau en proportions variables selon le dérivé à hydrolyser, la composition relative du mélange THF-eau (v/v) pouvant varier dans des proportions comprises entre 5-95 et 95-5.

Le catalyseur acide est ajouté, par exemple l'acide chlorhydrique sous forme de solution aqueuse concentrée, à raison de 0,2 à 10,0 mol par mole de composé (II.1) et plus généralement de 0,5 à 5 mol.

Le milieu réactionnel est ensuite porté à une température comprise entre 50°C et la température de reflux des solvants et chauffé de 2 à 72 h pour obtenir une quantité satisfaisante de produit.

Habituellement, une durée de chauffage de 5 à 24 heures est nécessaire ; après quoi le solvant est éliminé par distillation, le résidu aqueux traité pour isoler l'amine primaire (I.1) qui est finalement purifiée par distillation, cristallisation ou chromatographie comme il est décrit à la partie expérimentale du texte.

Le procédé de préparation des benzylamines de l'invention (I.2) dans lesquelles R3 ou R4 est l'hydrogène l'autre radical pouvant avoir les significations énoncées à la formule générale (I) : alkyle, alkènyle inférieur, consiste :

i) pour obtenir directement un composé dans lequel R3 ou R4 est méthyle, à réduire selon la réaction b.1) du schéma 1 un isocyanate (II.1) précédemment décrit. Avantageusement, il est utilisé comme agent réducteur un hydrure métallique ou organométallique dans des conditions appropriées à la réduction spécifique de la fonction isocyanate sans agir sur la liaison éthylènique des composés.

A cet effet, on utilise favorablement l'hydrure de lithium aluminium ou l'hydrure d'aluminium qui est préféré. Les réactions sont effectuées dans des solvants inertes aux réactifs utilisés tels que dans des éthers comme par exemple l'éther diéthylique, le 1,2-diméthoxy éthane ou le tetrahydrofurane (THF) qui est préféré.

Egalement de façon préférée, l'hydrure d'aluminium est préparé "in situ" à partir d'halogènures d'aluminium et d'hydrures métalliques comme il est par exemple décrit dans "Réduction with complex métal hydrides" - N.G. Gaylord., 1956, Ed. Interscience - p. 6 à 8, 51 à 53.

Avantageusement, la réaction de réduction dans le THF d'une mole d'isocyanate (II.1) consiste dans un premier temps à préparer "in situ" l'hydrure d'aluminium par réaction sur 0.75 à 2 moles de chlorure d'aluminium

et de 2,25 à 6 moles d'hydrure de lithium aluminium, ces réactifs étant utilisés dans un rapport moléculaire voisin de 1 pour 3, puis à introduire à une température comprise entre - 10 et + 30°C l'isocyanate, à laisser la réaction de réduction se développer de 1 à 24 h à la même température, puis à décomposer le complexe réduit obtenu et à isoler la N-méthyl amine de formule (I.2) par les méthodes habituelles.

Le plus couramment, les réductions sont effectuées à une température comprise entre 10 à 20°C durant 2 à 6 h.

ii) pour obtenir une benzylamine dans laquelle R3 est alkyle ou alkényle inférieur, R4 étant l'hydrogène, soit :

- à alkyler, selon la réaction c.1) du schéma 1, un composé de l'invention (I.1) par un halogénure Z1-R3 dans lequel R3 est précédemment défini et Z1 est le chlore, le brome ou l'iode, dans des conditions adaptées à favoriser la mono-alkylation de l'amine, qui est réalisée dans des solvants inertes aux réactifs comme par exemple le toluène et l'acétonitrile, et en faisant réagir une mole de benzylamine (I.1) avec de 0,5 à 1,5 mol d'halogénure.

D'une manière préférée, on utilise de 0,80 à 1,20 mol de dérivé ou l'halogène est le brome ou l'iode et optionnellement on ajoute une base, organique ou minérale, pour favoriser la réaction qui consiste à chauffer le milieu réactionnel à une température comprise entre 20 et 110°C et ce durant de 2 à 5 h, les produits étant ensuite isolés et purifiés par les méthodes habituelles, notamment par chromatographie,

- à réduire, selon la réaction e.1) du schéma 1 la fonction carboxamide d'un composé intermédiaire de l'invention (II.2.1.) précédemment décrit pour obtenir une amine secondaire de l'invention de formule (I.2).

La réduction est effectuée par des hydrures métalliques ou organométalliques appropriés et dans des conditions adaptées pour réduire sélectivement la fonction carboxamide.

Dans ces conditions et de façon analogue du procédé de réduction des isocyanates selon la réaction b.1) le réducteur préféré est l'hydrure d'aluminium que l'on utilise dans des conditions opératoires identiques à celles précédemment décrites.

Le procédé de préparation des benzylamines de l'invention (I.3) dans lesquelles R3 et R4 ont les définitions énoncées pour (I) à l'exception de l'hydrogène et sont alkyle ou alkènyle inférieur, consiste :

i) pour obtenir des benzylamines (I.3) dans lesquelles R3 et R4 sont méthyle, à pratiquer une diméthylation des benzylamines (I.1) de l'invention selon la réaction 1.f) qui consiste à faire réagir sur une amine primaire (I.1) soit le formaldéhyde en solution aqueuse et l'acide formique selon la réaction de Eschweiler-Clarke, soit dans l'acétonitrile, le formaldéhyde en solution aqueuse et un agent réducteur tel qu'un hydrure de bore et plus avantageusement le cyanoborohydrure de sodium dans des conditions décrites par exemple dans J. of Med. Chem. 1982, 25, 4, p. 446-51.

La diméthylation selon la réaction de Eischweler-Clarke consiste, pour traiter 1 mole de benzylamine (I.1), à ajouter de 2,0 à 5,0 mole de formaldéhyde en solution aqueuse à 30-40 % en poids pour volume, et ce à une température comprise entre 10 et 50°C mais plus favorablement entre 10 et 30°C durant 30 minutes à 3 heures puis à additionner à l'émulsion formée de 2,0 à 10,0 mole d'acide formique, pur ou en solution aqueuse de titre supérieur à 50 % (p/v), puis à chauffer la solution obtenue à une température comprise entre 50 et 100°C jusqu'à fin de dégagement gazeux, ce qui nécessite, selon les cas, de 35 minutes à 5 heures.

Selon la méthode préférée, à une mole de benzylamine (I.1), il est ajouté en homogénéisant énergiquement une solution aqueuse de 2,1 mole de formaldéhyde. Lorsque le mélange est intimement réalisé, on ajoute à l'émulsion obtenue 3 moles d'acide formique pur. La solution est chauffée progressivement à 100°C et maintenue à cette température jusqu'à fin de dégagement gazeux ce qui nécessite une durée d'une heure.

La solution est ensuite traitée pour isoler la benzylamine (I.3) formée qui est ensuite purifiée par les procédés conventionnels notamment par chromatographie.

ii) et, pour obtenir une benzylamine (I.3) dans laquelle R3 et R4 sont différents et ne sont pas l'hydrogène, à réduire la fonction carboxamide d'un composé (II.2.2) de préparation précédemment décrite. La réaction de réduction h.1) est pratiquée avec les mêmes réactifs et le même mode opératoire que la réaction e.1) décrite pour la préparation des benzylamines de formule (I.2).

iii) et, pour obtenir les benzylamines (I.3) dans lesquelles R3 et R4 sont différents et ne sont pas l'hydrogène, à faire réagir sur un composé intermédiaire de l'invention (II.3) et selon la réaction i.1) montrée au schéma 1, un réactif organométallique comme un dérivé organo magnésien de Grignard de formule R2 Mg Z3 dans lequel R2 est alkyle inférieur et Z3 un halogène et plus particulièrement le brome ou le chlore, selon une réaction décrite comme par exemple par N.J. Léonard et coll., J. Am. Chem. Soc., 1956, 78, p. 1986 et 1957, 79, p. 5279. Cette substitution du radical nitrile du composé (II.3) par le radical R2 alkyle du dérivé organo magnésien est effectuée dans les éthers comme l'éther diethylique, le méthyl-t.butyl éther, les éthers di-isopropylique ou dibutylique ou encore le tetrahydrofurane qui est le solvant préféré, et consiste pour une mole de composé (II.3) à faire réagir 1,5 à 6 moles de dérivé organo magnésien à une température comprise entre 5 et 50°C et ce durant 30 minutes à 12 heures.

La méthode préférée consiste à ajouter à une température comprise entre 10 et 20°C 1 mol de composé (II.3), éventuellement en solution dans le THF à 4 à 5 moles du composé organomagnésien également en solution dans le THF. La réaction est poursuivie durant de 2 à 5 heures à la même température puis le complexe obtenu décomposé par addition de solution aqueuse de chlorure d'ammonium. Après traitements, la benzylamine (I.3) est isolée et purifiée par les méthodes déjà citées.

Les benzylamines de l'invention optiquement actives sont préparées par résolution des formes racémiques correspondantes.

Pour réaliser cette opération, les méthodes de résolution proposées sont diverses et répertoriées dans des ouvrages de la littérature scientifique comme "Optical résolution procédures for Chemical Compounds" Vol. 1 - Amines and related compounds - Ed. Paul Newman 1981.

Dans le cadre de l'invention les énantiomères sont préparés par cristallisation fractionnée de leurs diastéréoisomères formés par réaction de la forme racémique avec un acide optiquement actif.

A cet effet de nombreux énantiomères d'acides peuvent permettre de réaliser ces résolutions qui sont effectuées à partir des produits de l'invention de structure (I.1), (I.2) ou (I.3).

Avantageusement, le procédé de l'invention consiste à former dans l'eau des diastéréoisomères de l'acide tartrique lévogyre ou de l'acide tartrique dextrogyre avec les produits racémiques de formule (I.1).

Habituellement, dans les conditions pratiquées, le précipité obtenu est le sel constitué par l'énantiomère de la benzylamine (I.1) de rotation opposée à celle de l'acide tartrique utilisé.

La purification des produits est réalisée par cristallisations répétées jusqu'à l'obtention d'une valeur stable de pouvoir rotatoire.

Les modes opératoires qui suivent illustrent, sans pour autant la limiter, la préparation de dérivés intermédiaires essentiels et celle des benzylamines de l'invention.

Les produits sont, selon les réactions effectuées, obtenus tels quels dans un état de pureté satisfaisant, ou purifiés par des techniques appropriées indiquées dans les exemples et qui sont généralement la cristallisation, la distillation sous vide ou encore la chromatographie sur colonne. Dans ce dernier cas on utilise favorablement la technique dite de "chromatoflash" sur un support de silice (marque "Merck", produit Kieselgel 60, granulométrie 230 à 400 mesh).

Par ailleurs, la pureté, l'identité et les caractéristiques physico chimiques des produits préparés sont rapportées et déterminées par :

- leur point d'ébullition sous la valeur du vide lors de leur distillation,
- leur point de fusion, déterminé par la méthode du tube capillaire et dont la valeur indiquée n'est pas corrigée,
- la chromatographie sur couches minces (CCM) de silice (plaques prêtes à l'emploi : produit "Merck" réf. 60 F 254) selon une technique qui est brièvement rappelée: les produits à étudier sont déposés sur la plaque à raison de 100 mcg environ puis élués de façon ascendante par des solvants ou leurs mélanges qui sont énumérés ci-après, les proportions respectives étant indiquées en volumes pour volumes dans la liste qui suit :

| | | | | |
|---|---|---|---|---|
| réf. S.A | - hexanes | 100 / | acétate d'éthyle | 10 |
| S.B | - " | 60 / | " | 10 |
| S.C | - " | 40 / | " | 10 |
| S.D | - " | 20 / | " | 10 |
| S.E | - " | 10 / | " | 10 |
| S.F | - dichlorométhane | 20 / | hexanes | 80 |
| S.G | - dichlorométhane | | | |
| S.H | - " | 90 / | acétone | 10 |
| S.I | - " | 85 / | " | 15 |
| S.J | - " | 80 / | " | 20 |
| S.K | - " | 98 / | méthanol | 2 |
| S.L | - " | 95 / | " | 5 |
| S.M | - " | 90 / | " | 10 |
| S.N | - " | 85 / | " | 15 |

Après développement, les chromatogrammes sont observés sous lumière ultra violette de 254 nm de longueur d'onde et/ou après révélation colorée par pulvérisation du réactif de Dragendorff ou du réactif à la tolidine. Les Rf observés ainsi que les références des solvants d'élution utilisés sont indiqués dans les exemples.

- l'analyse centésimale élémentaire dont les résultats, conformes aux normes admises ne sont pas reportés de façon chiffrée mais sont signalés être effectués par la représentation de l'élément dosé,
- la résonance magnétique nucléaire du proton (RMN) est étudiée à 60 ou 90 MHz, les produits étant solubilisés dans le deutérochloroforme. L'aspect des signaux, leur déplacement chimique exprimé en p.p.m par rapport au tétraméthylsilane utilisé comme référence interne sont indiqués. Les protons dits "échangeables" après addition d'oxyde de deutérium sont également signalés.
- La mesure du pouvoir rotatoire est exprimé par la rotation optique spécifique de produits (α) dans des conditions (concentration, solvant) indiquées de façon conventionnelle.

Enfin, divers réactifs ou solvants peuvent être indiqués sous leur forme abrégée courante, comme entre autres exemples, THF pour le tétrahydrofurane.

PRODUITS UTILISES DANS LA SYNTHESE

- Produits de structure (X)

X.a / α-diméthylamino phénylacétonitrile

(R1 = C6H5 ; R3 = R4 = CH3)

Dans un réacteur, à une solution de 11,82 g (0,241 mol) de cyanure de sodium et de 19,61 g (0,240 mol) de chlorhydrate de diméthylamine dans 40 ml d'eau, il est ajouté en une heure, à une température comprise entre 30 et 40°C une solution de benzaldéhyde (0,200 mol) dans 20 ml de méthanol. Le mélange est agité 4 h à la température ambiante, plus précipité dans 150 ml d'eau glacée et extrait à l'éther.

Les phases éthérées sont lavées successivement à l'eau, par une solution à 25 % de bisulfite de sodium, puis à nouveau à l'eau. Après évaporation de l'éther, le résidu est purifié par distillation. Eb/0,7 = 74 - 79°C.

Poids = 30,4 g Rdt = 95%

Selon ce mode opératoire et à partir de benzaldéhydes et de chlorhydrates d'amines secondaires appropriés les α-amino-phénylacétonitriles intermédiaires X.b à X.g ont été préparés.

X.b / α-(N-allyl, N-méthyl) amino-phénylacétonitrile.

(R1 = C6H5 ; R3 = CH3 ; R4 = CH2 = CH - CH2)
Rdt = 78% Eb/0,1 = 85 - 90°C

X.c / α-diméthylamino-p.chlorophénylacétonitrile.

(R1 = p.Cl-C6H4 ; R3 = R4 = CH3)
Rdt = 84 % F environ 50°C (éth. pétr.)

X.d / α-diméthylamino-3,4-dichlorophénylacétonitrile.

(R1 = 3,4(Cl)2-C6H3 ; R3 = R4 = CH3)
Rdt = 58% Eb/0,1 = 95 - 100°C

X.e / α-diméthylamino-p.toluylacétonitrile.

(R1 = p.CH3-C6H4 ; R3 = R4 = CH3)
Rdt = 64% Eb/0,1 = 80 - 85°C

X.f/α-diméthylamino-p.trifluorométhylphénylacétonitrile.

(R1 = p.F3C-C6H4 ; R3 = R4 = CH3)
Rdt = 67% F environ 50°C (éth. pétr.)

X.g / α-diméthylamino-p.méthoxyphénylacétonitrile.

(R1 = p.CH3O-C6H4 ; R3 = R4 = CH3)
Rdt = 82% Eb/0,1 = 95 - 105°C

- Produits de structures (VI) et (VII).

VI.a / Acide 2-p.méthoxyphényl-butanoïque.

(R1 = p.CH3O-C6H4 ; R2 = C2H5)

Dans un réacteur protégé de l'humidité et sous atmosphère d'azote, on introduit 29,4 g (0,23 mol) de naphtalène dans 200 ml de THF. A cette solution il est ajouté 5,5 g (0,23 mol) de sodium en morceaux préalablement dégraissés par du toluène. On obtient une solution verdâtre qui est maintenue sous agitation durant une nuit.

Par ailleurs dans un autre réacteur, 16,6 g, (0,10 mol) d'acide p.méthoxyphénylacétique sont dissous dans 200 ml de THF. La solution de naphtalènate de sodium précédemment préparée est introduite sous agitation, le mélange est maintenu 4 heures à température ambiante puis on ajoute en une heure environ 23,4 g (0,15 mol) d'iodoéthane.

Après une nuit d'agitation la suspension est précipitée dans 150 ml de solution de carbonate de sodium à 10 % p/v. La phase aqueuse est extraite à l'éther, les phases organiques réunies lavées par une solution HCl N puis avec une solution saturée en NaCl.

L'éther est évaporé, le résidu cristallisé dans 150 ml d'éther de pétrole.

Poids = 16,1 g Rdt = 83% F = 64°C

Les intermédiaires acides VI b, c et d sont préparés selon le mode opératoire précédent à partir des acides phénylacétiques substitués et des halogènures d'alkyle appropriés.

VI.b / Acide 2 - p.toluyl-butanoïque.

(R1 = p.CH3-C6H4 ; R2 = C2H5)
Rdt = 89% F = 62°C

VI.c / Acide 3 - méthyl-2-phényl butanoïque.

(R1 = C6H5 ; R2 = (CH3)2-CH)
Rdt = 67% F = 70°C

VI.e / Acide 2 - (3,4-dichloro) phényl-butanoïque.

(R1 = 3,4-Cl2-C6H3 ; R2 = C2H5)

Dans un réacteur on introduit à 5°C environ et sous agitation violente 100,0 g (0,537 mol) de 3,4-dichlorophénylacétonitrile dans une solution de 59,2 g (1,48 mol) de soude en pastilles dans 60 ml d'eau, puis on ajoute 1,2 g (52 mmol) de chlorure de benzyltriéthylammonium. Le mélange est maintenu 10 minutes à 5°C puis après retour à la température ambiante, 50,3 g (0,462 mol) de bromure d'éthyle sont introduits en 40 minutes environ et à 20°C.

Le mélange de couleur rouge est agité 4 h puis abandonné une nuit à 4°C.

Après addition de 560 ml d'eau, on extrait au benzène. Les phases organiques réunies sont lavées par une solution saturée en chlorure de sodium. Le benzène est évaporé, l'intermédiaire nitrile VII.e obtenu est purifié par distillation :

Eb/0,2 = 110 - 120°C.

Poids = 100,0 g Rdt = 87%

Sous atmosphère anhydre, 100,0 g (0,47 mol) du nitrile VII.e et 21,5 ml d'éthanol absolu sont saturés à -15°C par un courant d'acide bromhydrique gazeux. Après une nuit à la température ambiante on ajoute 880 ml d'acétone puis chauffe et porte une heure au reflux. La solution est concentrée au bain marie et sous vide puis on ajoute au résidu huileux 800 ml de solution concentrée d'hydroxyde de sodium à 30 % p/v et agite au reflux et sous agitation pendant 30 heures.

Le mélange est refroidi, extrait au chloroforme, la phase aqueuse alcaline est acidifiée à froid jusqu'à pH 1 par une solution concentrée d'acide sulfurique diluée au demi.

Après extraction au chloroforme et traitements habituels des phases organiques, les solvants sont évaporés et le résidu obtenu cristallisé pour purification dans 300 ml d'éther de pétrole.

Poids = 80,0 g Rdt = 74% F = 82°C

Selon ce mode opératoire les acides VI.f et VI.g ont été préparés par l'intermédiaire de leurs nitriles correspondants VII.f et VII.g

VI.f / Acide 2 - p.chlorophényl-butanoïque.

(R1 = p.Cl-C6H4 ; R2 = C2H5)

nitrile VII.f : Rdt = 82% Eb/0,05 = 85 - 95°C
acide VI.f : Rdt = 65% F = 84°C

VI.g / Acide 2 - (m. trifluorométhyl) phényl-butanoïque.

(R1 = m.F3C-C6H4 ; R2 = C2H5)

nitrile VII.g : Rdt = 58% Eb/0,06 = 75 - 80°C
acide VI.g : Rdt = 77% F = 72°C

- Produits de structures (V).

V.a / 1 - (3-bromo-1-propényl)-3,4-dichlorobenzène.

(R5 = 3,4 Cl2-C6H3 ; Z7 = Br)

Dans un réacteur protégé de l'humidité et sous atmosphère d'azote 100,6 g (0,46 mol) d'acide 3,4-dichloro cinnamique sont mis en suspension dans 1700 ml de méthanol.

Après addition de 56,6 ml de complexe BF3 - éther (65,3 g, 0,46 mol) le mélange est porté au reflux sous agitation pendant 18 h. La solution est évaporée, le résidu repris par environ 1 l. de dichlorométhane, la solution obtenue lavée avec une solution saturée en bicarbonate de sodium puis à l'eau. Après distillation le résidu est purifié par cristallisation dans l'éthanol. On obtient 99,2 g de 3,4- dichlorocinnamate de méthyle sous forme de cristaux blancs.

Rdt = 93% F = 115°C

Sous atmosphère d'azote, il est ajouté à une solution de 5,0 g (0,24 mol) de l'ester précédent dans 540 ml de toluène, à -40°C et en une heure environ, 300 g d'une solution toluénique 1,5 M de DIBAL-H(R). Le mélange est maintenu 2 h 30 à -40°C puis après retour à une température de 10°C environ, on introduit avec précautions 1 litre de solution d'acide sulfurique environ 2 M. La phase toluénique est séparée, la phase acide extraite à l'éther. Les phases organiques réunies sont lavées par une solution saturée en bicarbonate de sodium puis séchées sur Na2SO4.

Les solvants sont éliminés par distillation et le résidu purifié par chromatographie sur colonne.

L'élution par le mélange dichlorométhane-acétone 80/20 puis une cristallisation dans l'hexane permet d'obtenir 47,9 g (Rdt = 97 %) d'alcool 3,4-dichlorocinnamique purifié F = 64°C.

Dans un réacteur protégé de l'humidité et sous atmosphère d'azote, on introduit une solution de 25,1 g (0,29 mol) de bromure de lithium dans 225 ml d'acétonitrile.

Puis, à 40°C sous agitation, on ajoute successivement, en 30 minutes, 39,3 g (0,36 mol) de chlorotriméthylsilane et ensuite en 20 minutes, 29,45 g (0,145 mol) d'alcool 3,4-dichlorocinnamique en solution dans 125 ml d'acétonitrile.

Le mélange, sous agitation, est maintenu au reflux 16 heures puis refroidi et précipité dans 400 ml d'éther et 250 ml d'eau glacée. La phase organique séparée est lavée par une solution saturée en bicarbonate de sodium puis avec une solution saturée en NaCl. Les solvants sont éliminés et le produit brut résiduel purifié par distillation.

Poids = 35,1 g  Rdt = 91 %  Eb/0,1 = 115-120°C.

V.b / 1-(3-chloro 1-propènyl)- 3,4,5 triméthoxybenzène.

(R5 = 3,4,5(CH3O)3-C6H2 ; Z7 = Cl)

A une température de -10°C et sous atmosphère d'azote, il est introduit lentement dans une suspension de 25,7 g (0,193 mol) de chlorure d'aluminium dans 300 ml d'éther, une suspension de 22,0 g (0,58 mol) d'hydrure de lithium-aluminium dans 300 ml de THF. A la même température, en solution dans 280 ml de THF, 73,0 g (0,290 mol) de 3-(3,4,5-triméthoxyphényl)-2-propènoate de méthyle sont introduits en 10 minutes. Le mélange est agité ensuite 15 minutes puis précipité dans une solution glacée d'acide sulfurique 2,5 M, extrait à l'éther. Les phases organiques réunies sont lavées, séchées. L'évaporation des solvants conduit à l'alcool 3,4,5-triméthoxycinnamique qui est utilisé tel quel.

Une solution de 62,6 g (0,279 mol) de l'alcool précédent dans 500 ml de dichlorométhane est refroidie dans un bain d'eau glacée. On y ajoute successivement et sous agitation 20,4 g de 4-diméthylaminopyridine, 63,9 g de p.toluène- sulfochlorure et 38,9 ml de triéthylamine. La solution est maintenue 1 h à température ambiante sous agitation puis diluée par addition d'un litre d'éther. La suspension est filtrée, la phase organique extraite avec une solution à 10% (p/v) de sulfate de cuivre, puis avec une solution saturée en bicarbonate de sodium et enfin avec une solution saturée en chlorure de sodium. Après sèchage et évaporation de l'éther on obtient 47,9 g (71%) de produit huileux jaune orangé qui est instable et est immédiatement utilisé tel quel pour la suite des synthèses.

Produits intermédiaires de structure (III).

III.a / Acide α-cinnamyl-α-éthyl-phénylacétique.

(R1 = R5 = C6H5 ; R2 = C2H5)

Une solution de 339 g de diisopropylamine (3,35 mol) dans 2,2 l de THF anhydre, dans un réacteur de 12 litres protégé de l'humidité et sous atmosphère d'azote, est refroidie à -10°C. On ajoute 330 ml de solution de butyl-lithium 10M dans l'hexane (3,30 mol), lentement et à une température inférieure à -10°C. La solution est maintenue 30 minutes à cette température puis on ajoute une solution de 250 g (1,52 mol) d'acide 2 - phényl butanoïque dans 300 ml de THF en laissant la température remonter progressivement jusqu'à 15°C. On chauffe ensuite à 55 - 60°C durant 3 h, refroidit à 5°C et introduit sans dépasser 15°C 300 g (1,52 mol) de bromure de cinnamyle en solution dans 250 ml de THF.

On maintient sous agitation à la température du laboratoire durant 18 h puis sans dépasser 30°C ajoute 2 l de solution HCl 3N puis 1 l d'eau ; on extrait par 2 fois 1,5 l d'acétate d'éthyle, ajoute 4 l d'hexanes aux phases d'extractions réunies, lave le mélange par 2 fois 1,2 l de solution NaOH N. Les phases aqueuses alcalines sont acidifiées par une solution d'HCl concentré puis extraites par 2 fois 1,5 l d'acétate d'éthyle. Les phases organiques réunies sont lavées par une solution saturée en chlorure de sodium puis concentrées par distillation.

Le produit brut résiduel obtenu (428 g) est purifié par cristallisation dans 2 l de mélange hexanes - acétate d'éthyle 3/1 v/v. Le produit est obtenu sous forme de fins cristaux blancs.

Poids = 320 g  Rdt = 75%

F = 134 - 136°C  CCM : 0,40 ; S.C

A partir de bromure de cinnamyle et des acides intermédiaires de structure VI précédemment décrits les dérivés III.b à III.f sont préparés selon le mode opératoire de l'exemple III.a.

III.b / Acide α-cinnamyl-α-éthyl-p.chlorophénylacétique

(R1 = p.Cl-C6H4 ; R2 = C2H5 ; R5 = C6H5)
Rdt = 86% F = 185°C (éther) CCM : 0,40 ; S.H

III.c / Acide α-cinnamyl-α-éthyl-(3,4-dichloro)phényl acétique.

(R1 = 3,4 Cl2-C6H3 ; R2 = C2H5 ; R5 = C6H5)
Rdt = 72% F = 125°C (éth. de pétr.) CCM: 0,55 ; S.J

III.d / Acide α-cinnamyl-α-éthyl-p.tolylacétique.

(R1 = p.CH3-C6H4 ; R2 = C2H5 ; R5 = C6H5)
Rdt = 72% F = 169°C (éther) CCM: 0,45 ; S.H

III.e / Acide α-cinnamyl-α-éthyl-(3-trifluorométhyl) phénylacétique.

(R1 = m.F3C-C6H4 ; R2 = C2H5 ; R5 = C6H5)
Rdt = 67% F = 135°C (éther) CCM: 0,40 ; S.H

III.f / Acide α-cinnamyl-α-éthyl-p.méthoxyphénylacétique

(R1 = p.CH3O-C6H4 ; R2 = C2H5 ; R5 = C6H5)
Rdt = 54% F = 147°C (éther) CCM: 0,40 ; S.H

Le procédé de l'exemple III.a appliqué au 1-(3-bromo-1-propènyl)-3,4-dichlorobenzène (intermédiaire V.a) avec les acides intermédiaires (VI) permet d'obtenir les composés III.g à III.m.

III.g / Acide α-(3',4'-dichloro) cinnamyl-α-méthylphénylacétique.

(R1 = C6H5 ; R2 = CH3 ; R5 = 3,4 Cl2-C6H3)
Rdt = 61% F = 116°C (hexanes) CCM : 0,50 ; S.I

III.h / Acide α-(3',4'-dichloro) cinnamyl-α-éthylphénylacétique.

(R1 = C6H5 ; R2 = C2H5 ; R5 = 3,4 Cl2-C6H3)
Rdt = 58% F = 121°C (hexanes) CCM: 0,50 ; S.C

III.i / Acide α-(3',4'-dichloro)cinnamyl-α-isopropyl phénylacétique.

(R1 = C6H5 ; R2 = (CH3)2-CH ; R5 = 3,4 Cl2-C6H3)
Rdt = 69% F = 115°C (éth. pétr.) CCM: 0,45 ; S.H.

III.j / Acide α-(3',4'-dichloro)cinnamyl-α-éthyl- p.tolyl acétique

(R1 = p.CH3-C6H4 ; R2 = C2H5 ; R5 = 3,4 Cl2-C6H3)
Rdt = 72 % F = 105°C (acét. éthyle) CCM : 0,60 ; S.E.

III.k / Acide α-(3',4'-dichloro)cinnamyl-α-éthyl-p.méthoxyphénylacétique.

(R1 = p.CH3O-C6H4 ; R2 = C2H5 ; R5 = 3,4 Cl2-C6H3)
Rdt = 70% F = 135°C (éth. pétr.) CCM: 0,60 ; S.J.

III.l / Acide α-(3',4'-dichloro)cinnamyl-α-éthyl p.chlorophénylacétique.

(R1 = p.Cl-C6H4 ; R2 = C2H5 ; R5 = 3,4 Cl2-C6H3)

Rdt = 70 % F = 160°C (éth. pétr.) CCM: 0,60 ; S.L

III.m / Acide α-(3′,4′-dichloro)cinnamyl-α-éthyl-(3-trifluorométhyl)phénylacétique.

(R1 = m.F3C-C6H4 ; R2 = C2H5, ; R5 = 3,4 Cl2-C6H3)
Rdt = 62 % F = 96°C (hexanes) CCM: 0,40 ; S.L

PRODUITS DE L'INVENTION : EXEMPLES.

Exemple 1 : benzylisocyanates de formule (II.1)

Les produits de l'invention des exemples 1.a à 1.m qui suivent sont préparés par réaction de Curtius en une étape à partir d'acides de formule (III) précédemment décrits. Les isocyanates (II.1), instables, sont purifiés par chromatographies. Ce sont des huiles visqueuses dont la pureté et l'identité sont vérifiées par les analyses déja décrites. En outre, et plus particulièrement, ces composés présentent en spectrographie infra rouge une bande caractéristique des fonctions isocyanates à 2200-2300cm-1.

Mode opératoire :

Dans un réacteur protégé de l'humidité, à 8 litres de dichlorométhane on ajoute 1,0 mol d'acide (III) à traiter, 162,5 g (2,5 mol) d'azide de sodium et 197,75 g (202 ml, 2,5 mol) de pyridine. Sous agitation et à température ambiante 263,7 g (187 ml, 1,25 mol) de dichlorophosphate de phényle sont introduits goutte à goutte en 15 minutes.

Le mélange est agité à la température du laboratoire durant 18 à 20 h puis extrait successivement à l'eau puis avec HCl 0,1 N. La phase organique est séchée sur sulfate de sodium, celui ci est filtré puis on ajoute au filtrat 5 litres de toluène ; la solution est distillée à la pression ordinaire pour éliminer le dichlorométhane.

Le résidu toluènique est chauffé très progressivement et porté au reflux durant 45 minutes à 2 h, jusqu'à fin de dégagement gazeux. Après refroidissement on ajoute de l'hexane, filtre le mélange puis élimine les solvants par distillation sous vide et sur bain marie. Le résidu coloré, huileux est purifié par chromatographie.

Exemple 1.a : α-cinnamyl-α-éthyl-benzylisocyanate.

(II.1 ; R1 = R5 = C6H5 ; R2 = C2H5)
Rdt = 94% CCM : 0,45 - 0,55 ; S.C
RMN : 0,80 (t, 3H) ; 2,00 (q,2H) ; 2,80 (d,2H) ; 6.00 (m, 1H) ; 6,50 (m,1H) ; 7,25 (m,5H) ; 7,35 (m,5H)

Exemple 1.b : α-cinnamyl-α-éthyl-p.chlorobenzylisocyanate.

(II.1 ; R1 = p.Cl-C6H4 ; R2 = C2H5 ; R5 = C6H5)
Rdt = 86% CCM : 0,75 - 0,86 ; S.F
RMN : 0,80 (t,3H) ; 1,95 (q,2H) ; 2,75 (d,2H) ; 5,70-6,60 (m,2H) ; 7,15-7,40 (m,9H)

Exemple 1.c : α-cinnamyl-α-éthyl-(3,4-dichloro)benzylisocyanate.

(II.1 ; R1 = 3,4 Cl2-C6H4 ; R2 = C2H5 ; R5 = C6H5)
Rdt = 77% CCM : 0,30 - 0,40 ; S.F
RMN : 0,80 (t,3H) ; 1,95 (q,2H) ; 2,75 (d,2H) ; 5,70-6,60 (m,2H) ; 7,00-7,50 (m,8H)

Exemple 1.d : α-cinnamyl-α-éthyl-p.méthylbenzylisocyanate.

(II.1 ; R1 = p.CH3-C6H4 ; R2 = C2H5 ; R5 = C6H5)
Rdt = 97% CCM : 0,80 - 0,90 ; S.G
RMN : 0,80 (t,3H) ; 2,00 (q,2H), 2,35 (s,3H) ; 2,85 (d,2H) 5,65-6,70 (m,2H) ; 7,00-7,40 (m,9H)

Exemple 1.e : α-cinnamyl-α-éthyl-m.(trifluorométhyl)benzylisocyanate.

(II.1 ; R1 = m.F3C-C6H4 ; R2 = C2H5 ; R5 = C6H5)
Rdt = 89% CCM : 0,75 - 0,85 ; S.G.

RMN : 0,80 (t,3H) ; 2,00 (q,2H) ; 2,80 (d,2H) ; 5,75-6,60 (m,2H) ; 7,10-7,65 (m,9H)

Exemple 1.f : α-cinnamyl-α-éthyl-p.méthoxybenzylisocyanate.

(II.1 ; R1 = p.CH3O-C6H4 ; R2 = C2H5 ; R5 = C6H5)
Rdt = 96% CCM : 0,75-0,85 ; S.G
RMN : 0,80 (t,3H) ; 2,00 (q,2H) ; 2,80 (d,2H) ; 3,80(s,3H) 5,80-6,70 (m,2H) ; 6,80-7,50 (m,9H)

Exemple 1.g : α-(3′,4′-dichloro) cinnamyl-α-méthyl-benzyl isocyanate

(II.1 ; R1 = C6H5 ; R2 = CH3 ; R5 = 3,4 Cl2-C6H3)
Rdt = 93% CCM : 0,55 - 0,65 ; S.F
RMN : 1,75 (t,3H) ; 2,70 (d,2H) ; 5,60 - 6,50 (m,2H) ; 6,90-7,50 (m,8H)

Exemple 1.h : α-(3′,4′-dichloro) cinnamyl-α-éthyl-benzyl isocyanate

(II.1, R1 = C6H5 ; R2 = C2H5 ; R5 = 3,4 Cl2-C6H3)
Rdt = 78% CCM : 0,55 - 0,65 ; S.F
RMN : 0,90 (t,3H) ; 2,25 (m,2H) ; 3,25 - 3,90 (m,2H) ; 6,30 (s,2H) ; 7,70 - 7,70 (m,8H)

Exemple 1.i : α-(3′,4′-dichloro) cinnamyl-α-isopropylbenzylisocyanate

(II.1, R1 = C6H5 ; R2 = (CH3)2CH ; R5 = 3,4 Cl2-C6H3)
Rdt = 76% CCM: 0,45 - 0,55 ; S.F
RMN : 0,30 - 1,25 (m,7H) ; 2,35 - 2,90 (m,2H) ; 5,70 - 6,65 (m,2H) ; 6,75 - 7,40 (m,8H)

Exemple 1.j : α-(3′,4′-dichloro) cinnamyl-α-éthyl-p.méthylbenzylisocyanate

(II.1, R1 = p.CH3-C6H4 ; R2 = C2H5 ; R5 = 3,4 Cl2-C6H3)
Rdt = 91 % CCM: 0,85 ; S.F
RMN : 0,75 (t,3H) ; 1,95 (q,2H) ; 2,25 (s,3H) ; 2,70 (d,2H) - 5,60-6,35 (m,2H) ; 6,90-7,30 (m,7H) -

Exemple 1.k : α-(3′,4′-dichloro) cinnamyl-α-éthyl-p.méthoxybenzylisocyanate

(II.1, R1 = p.CH30-C6H4 ; R2 = C2H5 ; R5 = 3,4 Cl2-C6H3)
Rdt = 89% CCM : 0,30 ; S.F
RMN : 0,80 (,3H) ; 2,00 (q,2H) ; 2,75 (d,2H) ; 3,80 (s,3H) 5,70-6,45 (m,2H) ; 6,80-7,35 (m,7H)

Exemple 1.l : α-(3′,4′-dichloro) cinnamyl-α-éthyl-p.chlorobenzylisocyanate

(II.1, R1 = p.Cl-C6H4 ; R2 - C2H5 ; R5 = 3,4 Cl2-C6H3)
Rdt = 87 % CCM : 0,50 ; S.F
RMN : 0,80 (t,3H) ; 1,95 (q,2H) ; 2,75 (d,2H) ; 5,70 - 6,45 (m,2H) ; 6,90-7,60 (m,7H)

Exemple 1.m : α-(3′,4′-dichloro) cinnamyl-α-éthyl-m.trifluorométhylbenzylisocyanate

(II.1, R1 = m.F3C-C6H4 ; R2 = C2H5 ; R5 = 3,4 Cl2-C6H3)
Rdt = 86 % CCM : 0,40 ; S.F
RMN : 0,85 (t,3H) ; 2,05 (q,2H) ; 2,80 (d,2H) ; 5,70 - 6,45 (m,2H) ; 6,95 - 7,70 (m,7H)

Exemple 2 : α-alkyl-α-amino phénylacétonitriles de formule (II.3)

Les composés sont préparés par alkylation des phényl acétonitriles (X) avec les dérivés halogènes (V), dont les intermédiaires précédémment décrits aux préparations V.a et V.b.

Après réaction, les composés obtenus, instables, sont soit purifiés par cristallisation soit engagés tels quels dans la suite des réactions.

La pureté et l'identité des produits est vérifiée par chromatographies sur couches minces et par RMN.

Mode opératoire :

Dans un réacteur protégé de l'humidité et sous atmosphère d'azote, 1,025 mole de n. butyllithium (solution 10M/ hexanes) est ajoutée goutte à goutte à - 20°C dans une solution de 1,025 mole de diisopropylamine dans 1 litre de tetrahydrofurane anhydre.

Le mélange est maintenu 15 minutes à - 20°C. A -72°C, il est introduit 1,0 mole de nitrile (X) en solution dans 200 ml de THF, l'agitation est maintenue 1 h 30 à cette température puis on ajoute 1,025 mole de dérivé halogéné (V) en solution dans 500 ml de THF. Après 20 minutes à -72°C le mélange est agité 1 h à température ambiante.

On ajoute ensuite 1,5 l de solution de NH4Cl à 10 % (p/v) et 750 ml de mélange hexanes-acétate d'éthyle 1-1 (v/v).

La phase organique est séparée, la phase aqueuse réextraite par le même mélange de solvants. Les phases organiques réunies sont lavées par extraction avec une solution saturée en chlorure de sodium, puis séchées sur MgSO4. Les solvants sont éliminés par distillation sous vide et sur bain-marie. Le résidu huileux est, selon les cas, cristallisé par addition d'hexanes, ou utilisé tel quel dans la réaction i.1 décrite au schéma 1.

Selon ce mode opératoire et par alkylation des composés (X) avec les dérivés halogénés (V) appropriés, les produits des exemples 2.a à 2.g et 2.m à 2.s sont préparés.

Exemple 2.a : α-cinnamyl-α-diméthylamino-phénylacétonitrile.

(II.3 ; R1 = R5 = C6H5 ; R3 = R4 = CH3)
Rdt = 68 % (brut)      CCM : 0,40-0,50 ; S.C.
RMN : 2,30 (s, 6H) ; 2,85 (m, 2H) ; 5,55-5,90 (m, 1H) ; 6,10-6,50 (m, 1H) ; 7,00-7,70 (m, 10H).

Exemple 2.b : α-cinnamyl-α-diméthylamino-p.chlorophénylacétonitrile.

(II.3 ; R1 = p.Cl-C6H4 ; R3 = R4 = CH3 ; R5 = C6H5)
Rdt = 70 % (brut)      CCM : 0,70 - 0,80 ; S.G
RMN : 2,35 (s, 6H) ; 2,85 (m, 2H) ; 5,45-5,85 (m, 1H) ; 6,20-6,40 (m, 1H) ; 7,10-7,60 (m, 9H).

Exemple 2.c : α-cinnamyl-α-diméthylamino-3,4-dichlorophényl-acétonitrile

(II.3 ; R1 = 3,4Cl2-C6H3 ; R3 = R4 = CH3 ; R5 = C6H5)
Rdt = 85 % F = 88°C (hexanes) CCM : 0,85 - 0,95 ; S.J.
RMN : 2,30 (s, 6H) ; 2,80 (m, 2H) ; 5,50-5,90 (m, 1H) ; 6,15-6,40 (m, 1H) ; 7,15-7,50 (m, 8H).

Exemple 2.d : α-cinnamyl-α-diméthylamino-p.méthylphényl acétonitrile.

(II.3 ; R1 = p.CH3-C6H4 ; R3 = R4 = CH3 ; R5 = C6H5)
Rdt = 86 %      CCM : 0,25-0,35 ; S.G
F = 85°C (hexanes)
RMN : 2,35 (s, 6H) ; 2,65-3,15 (m, 2H) ; 5,50-5,85 (m, 1H) ; 6,25-6,40 (m, 1H) ; 7,10-7,50 (m, 9H).

Exemple 2.e : α-cinnamyl-α-diméthylamino-p.trifluorométhyl-phényl-acétonitrile.

(II.3 ; R1 = p.F3C-C6H4 ; R3 = R4 = CH3 ; R5 = C6H5)
Rdt = 99 % (brut)      CCM : 0,70-0,80 ; S.G
RMN : 2,35 (s, 6H) ; 2,90 (m, 2H) ; 5,45-5,85 (m, 1H) ; 6,15-6,40 (m, 1H) ; 7,10-7,45 (m, 5H) ; 7,55-7.80 (m, 4H).

Exemple 2.f : α-cinnamyl-α-diméthylamino-p.méthoxyphényl acétonitrile.

(II.3 ; R1 = p.CH3O-C6H4 ; R3 = R4 = CH3 ; R5 = C6H5)
Rdt = 85 %      F = 77°C (hexanes)      CCM : 0,85-0,95 ; S.G
RMN : 2,25 (s, 6H) ; 2,90 (m, 2H) ; 3,85 (s, 3H) ; 5,50-5,85 (m, 1H) ; 6,20-6,45 (m, 1H) ; 6,80-7,55 (m, 9H).

Exemple 2.g : α-(3′,4′-dichloro)cinnamyl-α-diméthylaminophényl acétonitrile.

(II.3 ; R1 = C6H5 ; R3 = R4 = CH3 ; R5 = 3,4 Cl2-C6H3)
Rdt = 78 % (brut) CCM : 0,35-0,45 ; S.C.
RMN : 2,25 (s, 6H) ; 2,85 (d, 2H) ; 6,05-6,50 (m, 2H) ; 7,05-7,55 (m, 8H).

Exemple 2.m. : α-diméthylamino-α-(3′,4′,5′ triméthoxy)cinnamyl- phénylacétonitrile.

(II.3 ; R1 = C6H5 ; R3 = R4 = CH3 ; R5 = 3,4,5 (CH3O)3 -C6H2)
Rdt = 85 % (brut) CCM : 0,45-0,55 ; S.E.
RMN : 2,25 (S,6H) ; 2,75-3,05 (m, 2H) ; 3,90 (s, 9H) ; 6,10-6,45 (m, 2H) ; 6,60 (s, 2H) ; 7,20-7,65 (m, 5H)

Exemple 2.n : α-(N-allyl-N-méthyl) amino-α-cinnamyl-phényl acétonitrile.

(II.3 ; R1 = R5 = C6H5 ; R3 = CH3 ; R4 = CH2-CH=CH2)
Rdt = 99 % (brut) CCM : 0,55 - 0,65 ; S.C.
RMN : 2,35 (s, 3H) ; 2,85 (d, 2H) ; 3,10 (d, 2H) ; 4,95-6,60 (m, 5H) ; 7,10-7,60 (m, 10H)

Exemple 2.o : α-(N-allyl-N-méthyl)amino-α-(3′,4′dichloro) cinnamyl-phénylacétonitrile.

(II.3 ; R1 = C6H5 ; R3 = CH3 ; R4 = CH2-CH=CH2 ; R5 = 3,4Cl2 - C6H3)
Rdt = 88 % (brut) CCM : 0,80 - 0,90 ; S.C.
RMN : 2,30 (s, 3H) ; 2,85 (t, 2H) ; 3,05 (d, 2H) ; 4,90-6,30 (m, 5H) ; 7,00-7,60 (m, 8H).

Exemple 2.p : α-(3′,4′-dichloro)cinnamyl-α-diméthylamino-p.chlorophénylacétonitrile

(II.3 ; R1 =p.Cl-C6H4 ; R3 = R4 = CH3 ; R5 = 3,4Cl2C6H3)
Rdt = 89 % (brut) CCM : 0,50 - 0,55 ; S.C.
RMN : 2,30 (s, 6H) ; 2,55-3,15 (m, 2H) ; 5,45-6,35 (m,2H) 6,85-7,60(m,7H)

Exemples 2.q : α-(3′,4′-dichloro)cinnamyl-α-diméthylamino-p.méthylphénylacétonitrile

(II.3 ; R1 = p.CH3-C6H4 ; R3 = R4 = CH3 ; R5 = 3,4Cl2 - C6H3)
Rdt = 100 % (brut) CCM : 0,70 ; S.C.
RMN : 2,30 (s, 9H) ; 2,50-3,10 (m, 2H) ; 5,40-6,35 (m,2H) 6,75-7,50 (m,7H)

EXEMPLE 2.r : α-(3′,4′-dichloro) cinnamyl-α-diméthylamino-p.trifluorométhylphénylacétonitrile

(II.3 ; R1 = p.F3C-C6H4 ; R3 = R4 = CH3 ; R5 = 3,4Cl2 - C6H3)
Rdt = 100 % (brut) CCM : 0,80 - 0,90 ; S.G.
RMN : 2,30 (s, 6H) ; 2,60-3,15 (m, 2H) ; 5,40-6,30 (m,2H) 6,90-7,80 (m,7H)

Exemple 2.s : α-(3′,4′-dichloro)cinnamyl-α-diméthylamino-p.méthoxyphénylacétonitrile

(II.3 ; R1 = p.CH30-C6H4 ; R3 = R4 = CH3 ; R5 = 3,4Cl2 - C6H3)
Rdt = 82 % (brut) CCM : 0,65-0,70 ; S.G.
RMN : 2,30 (s, 6H) ; 2,40-3,15 (m, 2H) ; 3,80 (s,3H) ; 5,45-6,35 (m,2H) 6,80-7,55 (m,7H)

Exemple 3 : α-cinnamyl-α-éthyl-benzylamine.

(I.1 ; R1 = R5 = C6H5 ; R2 = C2H5 ; R3 = R4 = H)
58,0 g (0,209 mol) du composé de l'exemple 1-a : α-cinnamyl-α-éthyl-benzylisocyanate sont ajoutés à un mélange de 1,5 l de tetrahydrofurane, 0,2 l d'eau et 50 ml d'acide chlorhydrique concentré (d = 1,19). La solution sous agitation est chauffée au reflux durant 18 heures.

Après refroidissement, le THF est éliminé par distillation sous vide et sur bain-marie. Au résidu, on ajoute 200 ml d'eau, alcalinise le mélange à froid jusqu'à pH 10 par ajout de solution concentrée d'hydroxyde de sodium puis extrait à trois reprises par 150 ml d'éther. Les phases éthérées sont réunies, lavées à l'eau puis déshydratées sur sulfate de magnésium.

L'éther est éliminé par distillation, le résidu huileux de couleur orangée (54,8 g) est purifié par distillation sous vide. Le produit est obtenu sous forme d'huile incolore visqueuse. Eb/0,01 = 135-150°C.
Poids = 38,0 g    Rdt = 72 %    CCM : 0,15-0,20 ; S.C.
RMN : 0,70 (t,3H) ; 1,45 (s,2H éch. D20) ; 1,80 (m,2H) ; 2,60 (m,2H) ; 6,00(m,1H) ; 6,45 (m,1H) ; 7,10-7,55 (m,10H)

Chlorhydrate :

A une solution de 13,04 g (53,3 mmol) du produit précédemment obtenu dans 100 ml d'éther, on ajoute à une température inférieure à 20°C, 20 ml d'éther chlorhydrique 2,8 M.

La solution est évaporée et le résidu solide blanc cristallisé dans un mélange éther-hexanes.
Poids : 10,07 g    F = 85-110°C    Rdt = 67 %
Anal. (C18H21N.HCl) C,H,Cl,N

Les benzylamines des exemples 4 à 15 décrites ci-dessous sont obtenus selon le mode opératoire de l'exemple 3 précédent, à partir des dérivés isocyanates de formula (II-1), décrits dans les exemples 1.b à 1.m.

<u>Exemple 4 : α-cinnamyl-α-éthyl-p.chlorobenzylamine.</u>

(I.1 ; R1 = p.Cl-C6H4 ; R2 = C2H5 ; R3 = R4 = H ; R5 = C6H5)
A partir de 1.b :
Rdt = 88 % (brut)    CCM : 0,50-0,60 ; S.L.
RMN : 0,75 (t,3H) ; 1,60 (s,2H ech. D20) ; 1,40-2,00 (m,2H) ; 2,30-2,90 (m,2H) ; 5,70-6,60 (m,2H) ; 6,85-7,75 (m, 9H)

Chlorhydrate, monohydrate :

Rdt = 92 %    F = 100-110°C (éthanol-eau)
Anal. (C18H20ClN.HCl.H20) C, H, Cl, N

<u>Exemple 5 : α-cinnamyl-α-éthyl-3,4 dichlorobenzylamine.</u>

(I.1 ; R1 = 3,4Cl2-C6H3 ; R2 = C2H5 ; R3 = R4 = H ; R5 = C6H5)
A partir de 1.c :
Rdt = 78 % (brut)    CCM : 0,60-0,80 ; S.L.
RMN : 0,75 (t,3H) ; 1,45 (s,2H ech. D20) ; 1,60-2,00 (m,2H) ; 2,30-2,85 (m,2H) ; 5,70-6,55 (m,2H) ; 7,15-7,65 (m,8H)

Chlorhydrate :

Rdt = 85 %    F = 204-207°C (méthanol-éther)
Anal. (C18H19Cl2N.HCl) C, H, Cl, N

<u>Exemple 6 : α-cinnamyl-α-éthyl-p.méthylbenzylamine.</u>

(I.1 ; R1 = p.CH3-C6H4 ; R2 = C2H5 ; R3 = R4 = H ; R5 = C6H5)
A partir de 1.d :
Rdt = 22 %    Eb/0,1 = 145-150°C    CCM : 0,45-0,65 ; S.L.
RMN : 0,60 (t,3H) ; 1,30-1,85 (m,2H) 1,80 (s,2H ech. D20) ; 2,20 (m,3H) ; 2,35-2,65 (m,2H) ; 5,50-6,50 (m,2H) ; 6,85-7,35 (m,9H)
Anal. (C19H23N) C, H, N

<u>Exemple 7 : α-cinnamyl-α-éthyl-(m.trifluorométhyl) benzylamine.</u>

(I.1 ; R1 = m.F3C-C6H4 ; R2 = C2H5 ; R3 = R4 = H ; R5 = C6H5)
A partir de 1.e :
Rdt = 71 % (brut)    CCM : 0,35-0,50 ; S.K.
RMN : 0,70 (t,3H) ; 1,60 (s,2H ech. D20) ; 1,50-2,05 (m,2H) ; 2,60-2,85 (m,2H) ; 5,70-6,55 (m,2H) ; 7,10-7,85 (m,9H)

Chlorhydrate :

Rdt = 64 % F = 208°C (éther)
Anal. (C19H20F3N.HCl) C, H, Cl, F, N

Exemple 8 : α-cinnamyl-α-éthyl-p.méthoxybenzylamine.

(I.1 ; R1 = p.CH3O-C6H4 ; R2 = C2H5 ; R3 = R4 = H ; R5 = C6H5)
A partir de 1.f :
Rdt = 59 % Eb/0,1 = 160-165°C CCM : 0,70-0,90 ; S.L.
RMN : 0,75 (t,3H) ; 1,60 (s,2H ech. D20) ; 1,45-2,10 (m,2H) ; 2,50-2,75 (m,2H) ; 5,65-6,65 (m,2H) ; 6,80-7,55 (m,9H)

Chlorhydrate :

Rdt = 87 % F = 95-105°C (éther pétrole)
Anal. (C19H23NO.HCl) C, H, Cl, N, 0

Exemple 9 : α-(3′,4′-dichloro) cinnamyl-α-méthylbenzylamine.

(I.1 ; R1 = C6H5 ; R2 = CH3 ; R3 = R4 = H ; R5 = 3,4Cl2-C6H3)
A partir de 1.g :
Rdt = 96 % (brut) CCM : 0,30-0,50 ; S.M.
RMN : 1,50 (t,3H) ; 1,60 (s,2H ech. D20) ; 2,50-270 (m,2H) ; 5,65-6,20 (m,2H) ; 6,90-7,60 (m,8H)

Chlorhydrate :

Rdt = 65 % F = 209-210°C (méthanol-éther)
Anal. (C17H17Cl2N.HCl) C, H, Cl, N

Exemple 10 : α-(3′,4′-dichloro) cinnamyl-α-éthylbenzylamine.

(I.1 ; R1 = C6H5 ; R2 = C2H5 ; R3 = R4 = H ; R5 = 3,4Cl2-C6H3)
A partir de 1.h :
Rdt = 87 % (brut) CCM : 0,65-0,70 ; S.E.
RMN : 0,90 (t,3H) ; 1,90-2,40 (m, 2H) ; 2,90-3,25 (m,2H) ; 6,30 (s,2H) ; 7,10-7,70 (m,7H) ; 9,05-9,65 (m,3H)

Chlorhydrate :

Rdt = 85 % F = 165-170°C (méthanol-éther)
Anal. (C18H19Cl2N.HCl) C, H, Cl, N

Exemple 11 : α-(3′,4′-dichloro) cinnamyl-α-isopropylbenzylamine.

(I.1 ; R1 = C6H5 ; R2 = (CH3)2-CH ; R3 = R4 = H ; R5 = 3,4Cl2-C6H3)
A partir de 1.i :
Rdt = 44 % (chromatographie) CCM : 0,25-0,45 ; S.K.
RMN : 0,70 (d,3H) ; 1,00 (d,3H) ; 1,70 (s, 2H ech. D20) ; 1,85-2,90 (m,1H) ; 3,45-3,70 (q,2H) ; 5,60-6,40 (m,2H) ; 6,85-7,50 (m,8H)

Chlorhydrate :

Rdt = 87 % F = 129°C (éthanol-éther)
Anal. (C19H21Cl2N.HCl) C, H, Cl, N

Exemple 12 : α-(3′,4′-dichloro) cinnamyl-α-éthyl-p.méthylbenzylamine.

(I.1 ; R1 = p.CH3-C6H4 ; R2 = CH5 ; R3 = R4 = H ; R5 = 3,4Cl2-C6H3)

A partir de 1.j :
Rdt = 61 % (chromatographie) CCM : 0,70 ; S.M.
RMN : 0,70 (t,3H) ; 1,50 (s, 2H ech. D20) ; 1,55-2,05 (m,2H) ; 2,30 (s,3H) ; 2,35-2,85 (m,2H) ; 5,70-6,45 (m,2H) ; 6,95-7,40 (m,7H)

Chlorhydrate :

Rdt = 85 % F = 190°C (méthanol-éther)
Anal. (C19H21Cl2N.HCl) C, H, Cl, N

Exemple 13 : α-(3′,4′-dichloro) cinnamyl-α-isopropylméthoxybenzylamine.

(I.1 ; R1 = p.CH30-C6H4 ; R2 = CH5 ; R3 = R4 = H ; R5 = 3,4Cl2-C6H3)
A partir de 1.k :
Rdt = 92 % (brut) CCM : 0,40 ; S.L.
RMN : 0,75 (t,3H) ; 1,50 (s, 2H ech. D20) ; 1,60-2,00 (m,2H) ; 2,25-2,85 (m,2H) ; 3,80 (s,3H) ; 5,70-6,40 (m,2H) ; 6,75-7,40 (m,7H)

Chlorhydrate :

Rdt = 92 % F = 158°C (acét. éthyle)
Anal. (C19H21Cl2NO.HCl) C, H, Cl, N, 0

Exemple 14 : α-(3′,4′-dichloro) cinnamyl-α-éthyl-p.chlorobenzylamine.

(I.1 ; R1 = p.Cl-C6H4 ; R2 = C2H5 ; R3 = R4 = H ; R5 = 3,4Cl2-C6H3)
A partir de 1.l :
Rdt = 76 % (chromatographie) CCM : 0,55 ; S.L.
RMN : 0,70 (t,3H) ; 1,40-2,10 (m,4H dont 2 ech. D20) ; 2,40-2,85 (m,2H) ; 5,70-6,50 (m,2H) ; 6,90-7,65 (m,7H)

Chlorhydrate :

Rdt = 87 % F = 166°C (éther)
Anal. (C18H18Cl3N.HCl) C, H, Cl, N

Exemple 15 : α-(3′,4′-dichloro) cinnamyl-α-éthyl-m.trifluorométhylbenzylamine.

(I.1 ; R1 = mF3C-C6H4 ; R2 = C2H5 ; R3 = R4 = H ; R5 = 3,4Cl2-C6H3)
A partir de 1.m :
Rdt = 96 % (brut) CCM : 0,75 ; S.L.
RMN : 0,75 (t,3H) ; 1,30-2,10 (m,4H dont 2 ech. D20) ; 2,40-2,90 (m,2H) ; 5,70-6,40 (m,2H) ; 6,90-7,805 (m,7H)

Chlorhydrate :

Rdt = 83 % F = 255°C (chlor. de méthylène)
Anal. (C19H18Cl2F3N.HCl) C, H, Cl, F, N

Exemple 16 : A - (-) tartrate de (+) α-cinnamyl-α-éthylbenzylamine

(I.1: R1 = R5 = C6H5 ; R2 = C2H5 ; R3 = R4 = H)

Dans un réacteur de 6 litres, on introduit 4,0 litres d'eau déminéralisée que l'on chauffe au reflux sous agitation. On ajoute alors 213,0 g (0,487 mol) de (±) α-cinnamyl-α-éthyl- benzylamine (produit de l'exemple 3) puis 139,8 g (0,932 mol) d'acide L-(-) tartrique. Le mélange est maintenu au reflux durant 30 minutes puis filtré à chaud sur Buchner.

La solution légèrement trouble est abandonnée une nuit pour cristallisation.

Le précipité formé est filtré et séché sous vide à 60°C jusqu'à poids constant. On obtient 127,0 g de produit. Le filtrat est conservé pour traitements.

Le produit est recristallisé dans 1,3 l d'eau déminéralisée au reflux. Après une nuit de repos, le précipité

est filtré puis séché sous vide. On obtient 115,7 g de produit. Le second filtrat est également conservé.

On recristallise de la même façon que précédemment : il est obtenu 92,4 g de produit (0,230 mol). Le troisième filtrat est aussi conservé.

- Pureté optique des produits :

Des prélèvements de l'insoluble purifié et du second et troisième filtrat de cristallisation sont traités par une solution d'hydroxyde de sodium puis extraits à l'éther. Après évaporation de l'éther, le pouvoir rotatoire spécifique des résidus est déterminé par polarimétrie.

- Résultats.

Résidu du filtrat de seconde cristallisation
$\alpha_D^{25}$ = + 12,3° (c = 6,33 ; MeOH)
Résidu du filtrat de troisième cristallisation
$\alpha_D^{25}$ = + 36,9° (c = 6,40 ; MeOH)
Résidu du précipité de troisième cristallisation
$\alpha_D^{25}$ = + 40,0° (c = 6,26 ; MeOH)

Le précipité obtenu après la troisième cristallisation est considéré de pureté optique satisfaisante :
Poids = 92,4 g    F = 158°C    Rdt = 54,3 %
Anal. C18H21N, C4H6O4) C, H, N, O
$\alpha_D^{25}$ (base) = + 40,0° (c = 6,26 ; MeOH) RMN (base) : 0,70 (t, 3H) ; 1,45 (s, 2H éch. D20) ; 1,80 (m, 2H) ; 2,60 (m, 2H) ; 6,00 (m,, 1H) ; 6,45 (m, 1H) ; 7,10-7,55 (m, 10H)

B - (+) tartrate de (-)α-cinnamyl-α-éthyl-benzylamine.

(I.1: R1 = R5 = C6H5 ; R2 = C2H5 ; R3 = R4 = H)

Le filtrat obtenu à la première cristallisation du produit A précédent est alcalinisé par une solution concentrée d'hydroxyde de sodium puis extrait par 3 fois 500 ml d'éther. les phases éthérées réunies sont lavées par une solution saturée en chlorure de sodium puis déshydratées sur Na2SO4. L'éther est éliminé par distillation.
Poids de résidu : 135,0 g
$\alpha_D^{25}$ = - 20,6° (c = 5,7 ; MeOH)

Le produit est introduit au reflux dans 2,3 l d'eau et on ajoute 88,7 g (0,59 mol) d'acide D-(+) tartrique. Après dissolution et filtration pour éliminer les impuretés mécaniques, la solution est abandonnée une nuit.

Le précipité formé est filtré et séché à 60°C sous vide. Poids : 149,0 g. Le filtrat de première cristallisation est conservé.

L'insoluble est recristallisé dans 1,5 l d'eau au reflux ; après une nuit de repos, le précipité est filtré et séché. Poids : 127,0 g. Le filtrat est conservé.

- Pureté optique des produits.

On procède de la même façon que décrit en A avec un échantillon des filtrats de première et de seconde cristallisation ainsi qu'avec le produit obtenu après la seconde cristallisation.

- Résultats.

Filtrat de première cristallisation
$\alpha_D^{25}$ = + 18,0° (c = 6,30 ; MeOH)
Filtrat de seconde cristallisation
$\alpha_D^{25}$ = - 23,0° (c = 6,10 ; MeOH)
Précipité de seconde cristallisation
$\alpha_D^{25}$ = - 39,3° (c = 5,5 ; MeOH)

Ce dernier produit est considéré de pureté optique satisfaisante et comparable à celle du produit A obtenu précédemment.
Poids : 127,0 g    F = 158°C    Rdt = 74,7 %
Anal. (C18H21N, C4H6O4) C, H, N, O

$\alpha_D^{25}$ (base) = - 39,3° (c = 5,5 ; MeOH)

RMN (base) : 0,70 (t, 3H) ; 1,45 (s, 2H éch. D20) ; 1,80 (m, 2H) ; 2,60 (m, 2H) ; 6,00 (m, 1H) ; 6,45 (m, 1H) ; 7,10-7,55 (m, 10H)

Exemple 17 : A - (+) α-cinnamyl-α-éthyl-p.chlorobenzylamine.

(I.1 ; R1 = p.Cl-C6H4 ; R2 = C2H5 ; R3 = R4 = H ; R5 = C6H5)

Tel que décrit à l'exemple 16 précédent et à partir de l'α-cinnamyl-α-éthyl-p.chlorobenzylamine racémique de l'exemple 4 et de l'acide (-) tartrique on isole le (-) tartrate de l'énantiomère dextrogyre qui traité en milieu alcalin et extrait au dichlorométhane permet d'obtenir le produit sous forme de base.

Rdt = 60 %   CCM : 0,5 ; S.L.

$\alpha_D^{20}$ = + 66,4 (c = 6,0 ; MeOH)

RMN : 0,75 (t,3H) ; 1,60 (s, 2H ech. D20) ; 1,40-2,00 (m, 2H) ;2,30-2,90 (m, 2H) ; 5,70-6,60 (m,2H) ; 6,85-7,75 (m,9H)

Chlorhydrate :

Rdt = 83 %   F = 204-205°C (éther)

$\alpha_D^{20}$ = + 25,4°C   (c = 5,0 ; MeOH)

Anal. (C18H20ClN,HCl) C, H, Cl, N

B - (-)α-cinnamyl-α-éthyl-p.chlorobenzylamine.

(I.1 ; R1 = p.Cl-C6H4 ; R2 = C2H5 ; R3 = R4 = H ; R5 = C6H5)

Le composé est obtenu par traitement du filtrat de cristallisation de l'énantiomère (+) précédent puis, de la même manière qu'à l'exemple 16B, préparation de diastéréoisomères avec l'acide tartrique (+) et purification par cristallisation. Le composé est finalement obtenu sous sa forme de base libre

Rdt = 47 %   CCM : 0,5 ; S.L.

$\alpha_D^{20}$ = - 64,2°   (c = 6,2 ; MeOH)

RMN : 0,75 (t,3H) ; 1,60 (s, 2H ech. D20) ; 1,40-2,00 (m, 2H) ;2,30-2,90 (m, 2H) ; 5,70-6,60 (m,2H) ; 6,85-7,75 (m,9H)

Chlorhydrate :

Rdt = 77 %   F = 202-204°C (éther)

$\alpha_D^{20}$ = - 23,6°   (c = 5,2 ; MeOH)

Anal. (C18H20ClN,HCl) C, H, Cl, N

Exemple 18 : α-cinnamyl-α-éthyl-N-méthyl-benzylamine.

(I.2 ; R1 = R5 = C6H5 ; R2 = C2H5 ; R3 =CH3 ; R4 = H)

Dans un réacteur de 12 l refroidi dans un bain de glace, on introduit sous atmosphère d'azote, 145,0 g (1,09 mol) de chlorure d'aluminium. Lentement et avec précautions, on ajoute 900 ml de THF anhydre. A la solution rougeâtre obtenue, on ajoute 3,2 l de solution 1 M d'hydrure de lithium aluminium (3,2 mol) dans le THF.

Le mélange est agité 15 minutes puis on ajoute goutte à goutte et sans dépasser 5°C, 233,0 g (0,84 mol) d'α-cinnamyl-α-éthyl-benzylisocyanate (exemple 1.a) en solution dans 200 ml de THF.

Après introduction le mélange est maintenu sous agitation 4 h à la température du laboratoire puis refroidi à 5°C environ par un bain de glace.

On ajoute 1,8 l d'éther méthyl-t.butylique puis goutte à goutte, avec précautions, 195 ml de solution de NaOH à 10 % (p/v). On ajoute ensuite 240 ml d'eau et laisse agiter le mélange 16 h à la température ambiante.

La suspension est filtrée sur buchner et sous vide. L'insoluble est écarté et le filtrat concentré par distillation sous vide et sur bain marie. Le produit brut résiduel est une huile visqueuse jaune qui est purifiée par distillation. Produit purifié : Eb/0,02 = 135-150°C, qui cristallise dans l'hexane.

Poids = 183,2 g   F = 54-56°C   Rdt = 82 %

CCM : 0,45-0,55 ; S.C.

Anal. (C19H23N) C, H, N

RMN : 0,70 (t, 3H) ; 1,40 (s, 1H ech. D20) ; 1,80 (q,2H) ; 2,20 (s,3H) ; 2,70 (d,2H) ; 6,00-6,45 (m,2H) ; 7,10-7,60 (m, 10H)

Telle que pratiquée dans l'exemple précédent, la réduction des isocyanates des exemples 1-b, 1-c, 1-e, 1-f et 1-i à 1-m conduit aux composés de l'invention décrits dans les exemples 19 à 27.

Exemple 19 : α-cinnamyl-α-éthyl-N-méthyl-p.chlorobenzylamine

(I.2 ; R1 = p.Cl-C6H4 ; R2 = C2H5 ; R3 = CH3 ; R4 = H ; R5 = C6H5)
Rdt = 74 % F = 50°C (hexanes) CCM : 0,40-0,70 ; S.L.
RMN : 0,75 (t,3H) ; 1,40-1,90 (m, 3H dont 1 H ech.) ; 2,15 (s,3H) ; 2,60 (d,2H) ; 5,70-6,50 (m,2H) ; 7,10-7,45 (m,9H)

Chlorhydrate :

Rdt = 82 % F = 205-210°C (méthanol)
Anal. (C19H22ClN.HCl) C, H, Cl, N

Exemple 20 :α-cinnamyl-α-éthyl-N-méthyl-3,4-dichlorobenzylamine

(I.2 ; R1 = 3,4Cl2-C6H3 ; R2 = C2H5 ; R3 = CH3 ; R4 = H ; R5 = C6H5)
Rdt = 59 % F = 68°C (hexanes) CCM : 0,50-0,70 ; S.L.
RMN : 0,75 (t,3H) ; 1,35 (s,1H ech) ; 1,75 (q,2H) ; 2,15 (s,3H) ; 2,65 (d,2H) ; 5,80-6,55 (m,2H) ; 7,10-7,70 (m, 8H)

Chlorhydrate :

Rdt = 75 % F = 206-208°C (méthanol)
Anal. (C19H21Cl2N.HCl) C, H, Cl, N

Exemple 21 : α-cinnamyl-α-éthyl-N-méthyl-(m.trifluorométhyl)benzylamine.

(I.2 ; R1 = m.F3C-C6H4 ; R2 = C2H5 ; R3 = CH3 ; R4 = H ; R5 = C6H5)
Rdt = 93 % (brut) CCM : 0,80-0,90 ; S.L.
RMN : 0,70 (t,3H) ; 1,50 (s,1H ech.) ; 1,80 (q,2H) ; 2,20 (s,3H) ; 2,65 (d,2H) ; 5,70-6,55 5m, 2H) ; 7,10-7,80 (m,9H)

Chlorhydrate :

Rdt = 68 % F = 172°C (éthanol)
Anal. (C20H22F3N.HCl) C, H, Cl, F, N

Exemple 22 : α-cinnamyl-α-éthyl-N-méthyl-p.méthoxybenzylamine.

(I.2 ; R1 = p.CH3O-C6H4 ; R2 = C2H5 ; R3 = CH3 ; R4 = H ; R5 = C6H5)
Rdt = 52 % (chromatographie) F = 95° CCM : 0,40-0,60 ;S.M.
Anal. (C20H25N0) C, H, N, 0
RMN : 0,75 (t,3H) ; 1,55 (s,1H ech) ; 1.80 (q,2H) ; 2,20 (s,3H) ; 2,65 (d,2H) ; 3,85 (s,3H) ; 5,75-6,40 (m, 2H) ; 6,60-7,60 (m, 9H)

Exemple 23 : α-(3',4'-dichloro)cinnamyl-α-isopropyl-N-méthyl-benzylamine.

(I.2 ; R1 = C6H5 ; R2 = (CH3)2-CH ; R3 = CH3 ; R4 = H R5 = 3,4Cl2-C6H3)
Rdt = 54 % (chromatographie) CCM : 0,50-0,70 ; S.L.
RMN : 0,70 (d,3H) ; 0,80 (d, 3H) ; 1,40 (s,1H ech.) ; 1,90 (q,1H) ; 2,30 (s,3H) ; 2,80-3,00 (m, 2H) ; 6,00-6,60 (m,2H) ; 7,05-7,50 (m,8H)

Chlorhydrate :

Rdt = 79 % F = 156-160°C (éther)
Anal. ($C_{20}H_{23}Cl_2N.HCl$) C, H, Cl, N

Exemple 24 : α-(3',4'-dichloro)cinnamyl-α-éthyl-N-méthyl-p.méthylbenzylamine.

(I.2 ; R1 = $CH_3-C_6H_4$ ; R2 = $C_2H_5$ ; R3 = $CH_3$ ; R4 = H R5 = 3,4$Cl_2-C_6H_3$)
Rdt = 45 % (chromatographie) CCM : 0,60 ; S.K.
RMN : 0,70 (t,3H) ; 1,60-2,05 (m,2H) ; 2,20 (s,4H dont 1 ech. D20) ; 2,30 (s,3H) ; 2,70 (d, 2H) ; 5,70-6,45 (m,2H) ; 7,00-7,50 (m,7H)

Chlorhydrate :

Rdt = 87 % F = 196°C (méthanol-éther)
Anal. ($C_{20}H_{23}Cl_2N.HCl$) C, H, Cl, N

Exemple 25 : α-(3',4'-dichloro)cinnamyl-α-éthyl-N-méthyl-p.méthoxybenzylamine.

(I.2 ; R1 = p.$CH_3O-C_6H_4$ ; R2 = $C_2H_5$ ; R3 = $CH_3$ ; R4 = H R5 = 3,4$Cl_2-C_6H_3$)
Rdt = 40 % (chromatographie) CCM : 0,55 ; S.L.
RMN : 0,70 (t,3H) ; 1,40 (s,1H ech. D20) ; 1,60-1,90 (q,2H) ; 2,15 (s,3H) ; 2,60 (d, 2H) ; 3,80 (s,3H) ; 5,70-6,40 (m,2H) ; 6,70-7,40 (m,7H)

Chlorhydrate :

Rdt = 77 % F = 186°C (acét. éthyle)
Anal. ($C_{20}H_{23}Cl_2NO.HCl$) C, H, Cl, N, 0

Exemple 26 : α-(3',4'-dichloro)cinnamyl-α-éthyl-N-méthyl-p.chlorobenzylamine.

(I.2 ; R1 = p.$Cl-C_6H_4$ ; R2 = $C_2H_5$ ; R3 = $CH_3$ ; R4 = H R5 = 3,4$Cl_2-C_6H_3$)
Rdt = 86 % (brut) CCM : 0,50 ; S.L.
RMN : 0,70 (t,3H) ; 1,35 (s,1H ech. D20) ; 1,80 (q,2H) ; 2,20 (s,3H) ; 2,65 (d, 2H) ; 5,70-6,50 (m,2H) ; 6,90-7,50 (m,7H)

Chlorhydrate :

Rdt = 79 % F = 180°C (méthanol-éther)
Anal. ($C_{19}H_{20}Cl_3N.HCl$) C, H, Cl, N

Exemple 27 : α-(3',4'-dichloro)cinnamyl-α-éthyl-N-méthyl-m.trifluorométhylbenzylamine.

(I.2 ; R1 = m.$F_3C-C_6H_4$ ; R2 = $C_2H_5$ ; R3 = $CH_3$ ; R4 = H R5 = 3,4$Cl_2-C_6H_3$)
Rdt = 88 % (brut) CCM : 0,70 ; S.L.
RMN : 0,75 (t,3H) ; 1,40 (s,1H ech. D20) ; 1,85 (q,2H) ; 2,20 (s,3H) ; 2,70 (d, 2H) ; 5,70-6,45 (m,2H) ; 6,95-7,85 (m,7H)

Chlorhydrate :

Rdt = 93 % F = 165°C (éther)
Anal. ($C_{20}H_{20}Cl_2F_3N.HCl$) C, H, Cl, F, N

Exemple 28 : (-)α-cinnamyl-α-éthyl-N-méthyl benzylamine.

(I.2 ; R1 = R5 = $C_6H_5$ ; R2 = $C_2H_5$ ; R3 = $CH_3$ ; R4 = H)
Dans un réacteur protégé de l'humidité et sous atmosphère d'azote, on introduit 182 ml de THF anhydre, 7,7 g (0,167 mol) d'acide formique pur et 26,6 g (0,164 mol) de 1,1'-carbonyldiimidazole.

La solution est agitée une heure à température ambiante puis on ajoute 40,0 g (0,159 mol) de (+) α-cinnamyl-α-éthyl-benzylamine obtenue à l'exemple 16 A en solution dans 430 ml de THF anhydre.

L'introduction est réalisée en 15 minutes à température ambiante puis l'agitation est maintenue durant 4 heures.

Le THF est éliminé par distillation sous vide et sur bain-marie et le résidu est repris par 300 ml de solution HCl N. Le mélange est extrait à l'éther et la phase acide écartée.

La phase éthérée est extraite par une solution saturée en bicarbonate de sodium, puis à l'eau et enfin déshydratée sur Na2SO4. L'éther est concentré jusqu'à obtenir un volume résiduel de 200 ml. Cette solution résiduelle éthérée est maintenue 24 h à 4°C.

L'insoluble cristallin qui précipite est le (+) α-cinnamyl-α-éthyl-N-formyl-benzylamine (intermédiaire II.2 de formule II.2.1 ; R1 = R5 = C6H5 ; R2 = C2H5 ; R6 = H).
Il est filtré, séché sous vide à 60°C jusqu'à poids constant.
Poids : 41,0 g F = 101°C Rdt = 92,3 %
$\alpha_D^{25}$ = + 38,5° (c = 4,05 ; MeOH)

Dans un réacteur protégé de l'humidité et sous atmosphère d'azote, on introduit 25,5 g (0,192 mol) de chlorure d'aluminium. Le réacteur est refroidi par un bain de carboglace/acétone et on ajoute 309 ml d'éther anhydre en 15 minutes environ et sans dépasser 0°C. On obtient une solution qui est maintenue 30 minutes à 0°C.

Parallèlement, dans un réacteur également protégé de l'humidité et sous atmosphère d'azote, il est introduit 22,3 g (0,587 mol) d'hydrure de lithium aluminium et après refroidissement par un bain carboglace/acétone, 309 ml de THF anhydre en 10 minutes environ.

La suspension obtenue est maintenue sous agitation durant 15 minutes à 0°C puis transférée dans le premier réacteur contenant la solution éthérée de chlorure d'aluminium. Le transfert est effectuée en 10 minutes à une température inférieure à 0°C.

Après 30 minutes d'gitation, on y ajoute 41,0 g (0,147 mol) de l'intermédiaire précédent en solution dans 120 ml de THF anhydre, en 30 minutes et à une température voisine de 0°C.

Après retour vers 20°C, le mélange réactionnel est porté au reflux, sous agitation durant 2 heures. On refroidit à 0°C puis introduit goutte à goutte 47,8 ml d'une solution d'hydroxyde de sodium à 10 % (p/v) puis 42 ml d'eau.

Le mélange est abandonné une nuit, l'insoluble filtré sur buchner et lavé au THF. Les filtrats réunis sont évaporés sous vide et sur bain-marie. On obtient un résidu brut huileux.
Poids : 36,1 g
$\alpha_D^{25}$ = - 12,4° (c = 6,39 ; MeOH)

Le produit est purifié par chromatographie sur colonne de silice. L'élution par un mélange chlorure de méthylène-méthanol 98-2 (v/v) permet d'obtenir le produit purifié sous forme d'une huile incolore.
Poids : 32,6 g Rdt = 83,5 % CCM : 0,45-0,55 ; S.L.
$\alpha_D^{25}$ = - 16,4° (c = 6,1 ; MeOH)
RMN (base) : 0,70 (t, 3H) ; 1,40 (s, 1H éch. D20) ; 1,80 (q, 2H) ; 2,20 (s, 3H) ; 2,70 (d, 2H) ; 5,75-6,50 (m, 2H) ; 7,10-7,60 (m, 10H)

(+) Tartrate dihydraté :

Dans un réacteur équipé en position de reflux on dissout 32,6 g (0,123 mol) du produit précédent dans 250 ml d'éthanol à 95 %.

Parallèlement, 20,3 g (0,135 mol) d'acide D-(+) tartrique sont dissous au reflux dans 250 ml d'éthanol à 95 %.

Cette solution chaude est introduite dans la solution de produit. Le mélange est agité 15 minutes au reflux puis abandonné une nuit à température ambiante. L'insoluble est filtré puis séché à 60°C sous vide.
Poids : 51,0 g F = 127°C Rdt = 91,8 %
Anal. (C19H23N, C4H6O6, 2H20) C, H, N, O

Exemple 29 : (+) α-cinnamyl-α-éthyl-N-méthyl benzylamine.

(I.2 ; R1 = R5 = C6H5 ; R2 = C2H5 ; R3 = CH3 ; R4 = H)
En procédant de façon identique à celle décrite à l'exemple 28 et à partir de 40,0 g de (-)α-cinnamyl-α-éthyl-benzylamine préparée à l'exemple 16 B, on obtient :

(-)α-cinnamyl-α-éthyl-N-formyl benzylamine.

(Intermédiaire II.2.1 - R1 = R5 = C6H5 ; R2 = C2H5 ; R6 = H)
Poids : 39,3 g F = 101°C Rdt = 88,5 %
$\alpha_D^{25}$ = - 37,9° (c = 5,82 ; MeOH)

Puis après réduction de 39,0 g (0,140 mol) de ce composé et tel que décrit à l'exemple 28, on obtient à l'état brut 36,4 g de produit :
$\alpha_D^{25}$ = + 15,3° (c = 5,1 ; MeOH)
qui sont purifiés par chromatographie sur colonne de silice.
Poids : 31,3 g Rdt = 84,2 % CCM : 0,45-0,55 ; S.L.
$\alpha_D^{25}$ = + 16,3° (c = 5,58 ; MeOH)
RMN : 0,70 (t, 3H) ; 1,40 (s, 1H éch. D20) ; 1,80 (q, 2H) ; 2,20 (s, 3H) ; 2,70 (d, 2H) ; 5,75-6,50 (m, 2H) ; 7,10-7,60 (m, 10H)

(-) Tartrate dihydraté :

Préparé par salification de 15,5 g (0,058 mol) de produit avec 9,64 g (0,064 mol) d'acide 1-(-) tartrique on obtient le produit purifié
Poids : 25,2 g F = 128°C Rdt = 96,2 %
Anal. (C19H23N, C4H6O6, 2H2O) C, H, N, O

Exemple 30 : α-cinnamyl-N,N-diméthyl-α-éthyl-benzylamine.

(I.3 ; R1 = R5 = C6H5 ; R2 = C2H5 ; R3 = R4 = CH3)

Dans un réacteur protégé de l'humidité et sous atmosphère d'azote, on introduit 2,8 l de solution 2,0 M de bromure d'éthylmagnésium (5,6 mol) dans le THF.

En 15 minutes, sous agitation et à la température ambiante, on introduit 340,0 g (1,24 mol) d'α-diméthylamino-α-cinnamyl-phénylacétonitrile (exemple 2-a) en solution dans 2 l de THF.

Le mélange est agité à la température du laboratoire durant 3 h puis on ajoute sans dépasser 20°C et avec précautions 5,5 l de solution aqueuse saturée en chlorure d'ammonium.

La phase aqueuse est décantée et extraite par 2 fois 800 ml de mélange hexanes-acétate d'éthyle 1-3 (v/v).

Les phases organiques réunies sont extraites par 2 fois 730 ml de solution HCl N. Les phases aqueuses acides réunies sont alcalinisées par une solution concentrée d'hydroxyde de sodium puis le mélange est extrait par 3 fois 800 ml de mélange hexanes-acétate d'éthyle. Les phases organiques réunies sont lavées à l'eau, séchées sur MgSO4 puis évaporées sous vide. L'huile jaune résiduelle (282,0 g) est purifiée par cristallisation dans l'hexane.

Poids = 166,1 g Rdt = 48 % F = 60-62°C
CCM : 0,35-0,45 ; S.C.

RMN : 0,80 (t,3H) ; 1,90 (q,2H) ; 2,25 (s,6H) ; 2,90 (d,2H) ; 6,00-6,60 (m,2H) ; 7,10-7,55 (m,10H)

Chlorhydrate :

72,4 g (0,229 mol) du produit précédent sont dissous dans 400 ml d'éther.

A cette solution, on ajoute 140 ml de solution d'éther chlorhydrique 2N (0,280 mol). On ajoute du méthanol pour dissoudre le solide gommeux formé et abandonne la solution 3 jours à 4°C.
Le précipité blanc est filtré et séché sous vide.

Poids = 64,5 g Rdt = 79 % F = 182-184°C
Anal. (C20H25N.HCl) C, H, Cl,N

Les produits des exemples 31 à 46 qui suivent sont obtenus selon le mode opératoire précédent en engageant des α-diméthylamino phénylacétonitriles des exemples 2 à réagir avec les halogénures organomagnésiens appropriés.

Exemple 31 : α-cinnamyl-N,N-diméthyl-α-éthyl-p.chlorobenzylamine.

(I.3 ; R1 = p.Cl-C6H4 ; R2 = C2H5 ; R3 = R4 = CH3 ; R5 = C6H5)
A partir de 2.b et C2H5MgBr

Rdt = 49 % F = 45-50°C (éthanol)
CCM : 0,40-0,60 ; S.K.
RMN : 0,75 (t,3H) ; 1,85 (q,2H) ; 2,25 (s,6H) ; 2,80 (d,2H) ; 5,95-6,55 (m,2H) ; 7,10-7,45 (m,9H)

Chlorhydrate :

Rdt = 58 % F = 204°C (éthanol)
Anal. (C20H24ClN.HCl) C, H, Cl,N

Exemple 32 : α-cinnamyl-N,N-diméthyl-α-éthyl-(3,4-dichloro)benzylamine.

(I.3 ; R1 = 3,4Cl2-C6H3 ; R2 = C2H5 ; R3 = R4 = CH3 ; R5 = C6H5) A partir de 2.c et C2H5MgBr:
Rdt = 54 % F = 82°C (éther de pétrole)
CCM : 0,85-0,95 ; S.K.
RMN : 0,75 (t,3H) ; 1,85 (q,2H) ; 2,25 (s,6H) ; 2,80 (d,2H) ; 5,95-6,55 (m,2H) ; 7,10-7,60 (m,8H)

Chlorhydrate :

Rdt = 72 % F = 215°C (méthanol)
Anal. (C20H23Cl2N.HCl) C, H, Cl,N

Exemple 33 : α-cinnamyl-N,N-diméthyl-α-éthyl-p.méthylbenzylamine.

(I.3 ; R1 = p.CH3-C6H4 ; R2 = C2H5 ; R3 = R4 = CH3 ; R5 = C6H5)
A partir de 2.d et C2H5MgBr
Rdt = 51 % (chromatographie) CCM : 0,75-0,85 ; S.N.
RMN : 0,80 (t,3H) ; 1,95 (q,2H) ; 2,25 (s, 6H) ; 2,35 (s,3H) ; 2,90 (d,2H) ; 6,00-6,65 (m,2H) ; 7,05-7,45 (m,9H)

Chlorhydrate :

Rdt = 83 % F = 197°C (méthanol-éther)
Anal. (C21H27N.HCl) C, H, Cl,N

Exemple 34 : α-cinnamyl-N,N-diméthyl-α-éthyl-p.trifluorométhylbenzylamine.

(I.3 ; R1 = p.F3C-C6H4 ; R2 = C2H5 ; R3 = R4 = CH3 ; R5 = C6H5)
A partir de 2.e et C2H5MgBr
Rdt = 33 % F = 52°C (éthanol) CCM : 0,75-0,85 ; S.K.
RMN : 0,75 (t,3H) ; 1,90 (q,2H) ; 2,30 (s,6H) ; 2,85 (d,2H) ; 6,00-6,60 (m,2H) ; 7,15-7,40 (m,5H) ; 7,60 (s, 4H)

Chlorhydrate :

Rdt = 76 % F = 196-198°C (méthanol-éther)
Anal. (C21H24F3N.HCl) C, H, Cl, F, N

Exemple 35 : α-cinnamyl-N,N-diméthyl-α-éthyl-p.méthoxy benzylamine.

(I.3 ; R1 = p.CH3O-C6H4 ; R2 = C2H5 ; R3 = R4 = CH3 ; R5 = C6H5)
A partir de 2.f et C2H5MgBr
Rdt = 47 % F = 55°C (hexanes) CCM : 0,20-0,40 ; S.L.
RMN : 0,80 (t,3H) ; 1,90 (q,2H) ; 2,25 (s,6H) ; 2,85 (d,2H) ; 3,80 (s,3H) ; 6,00-6,60 (m,2H) ; 6,80-7,45 (m,9H)

Chlorhydrate :

Rdt = 77 % F = 167°C (méthanol)
Anal. (C21H27NO.HCl) C, H, Cl, N

Exemple 36 : α-(3',4'-dichloro) cinnamyl-N,N-diméthyl-α-éthyl-benzylamine.

(I.3 ; R1 = C6H5 ; R2 = C2H5 ; R3 = R4 = CH3 ; R5 = 3,4Cl2-C6H3)
A partir de 2.g et C2H5MgBr
Rdt = 58 % F = 76-80°C (hexanes) CCM : 0,30-0,40 ; S.B.
Anal. (C20H23Cl2N) C, H, Cl,N
RMN : 0,75 (t,3H) ; 1,80 (q,2H) ; 2,25 (s,6H) ; 2,85 (d,2H) ; 6,05-6,50 (m,2H) ; 7,05-7,55 (m,8H)

Exemple 37 : N,N-diméthyl-α-éthyl-α-(3',4',5'-triméthoxy) cinnamyl-benzylamine.

(I.3 ; R1 = C6H5 ; R2 = C2H5 ; R3 = R4 = CH3 ; R5 = 3,4,5 (CH3O)3-C6H2)
A partir de 2.m et C2H5MgBr
Rdt = 45 % F = 116-118°C (méthanol)
CCM : 0,35-0,45 ; S.C.
Anal. (C23H31NO3) C, H, N, O
RMN : 0,80 (t,3H) ; 1,95 (q,2H)) ; 2,25 (s,6H) ; 2,75-3,05 (m,2H) ; 3,90 (s,9H) ; 6,10-6,45 (m,2H) ; 6,60 (s, 2H) ; 7,20-7,70 (m, 5H)

Exemple 38 : N-allyl-α-cinnamyl-α-éthyl-N-méthylbenzylamine

(I.3 ; R1 = R5 = C6H5 ; R2 = C2H5 ; R3 = CH3 ; R4 = CH2-CH=CH2)
A partir de 2.n et C2H5MgBr
Rdt = 45 % Eb/0,025 = 150-165°C
CCM : 0,15-0,25 ; S.D.
Anal. (C22H27N) C, H, N
RMN : 0,75 (t,3H) ; 1,90 (q,2H) ; 2,35 (s,3H) ; 2,85 (d,2H) ; 3,10 (d,2H) ; 4,95-6,60 (m,5H) ; 7,10-7,60 (m, 10H)

Exemple 39 : N-allyl-α-(3',4'-dichloro)cinnamyl-α-éthyl-N-méthylbenzylamine.

(I.3 ; R1 = C6H5 ; R2 = C2H5 ; R3 = CH3 ; R4 = CH2-CH=CH2 R5 = 3,4Cl2-C6H3)
A partir de 2.o et C2H5MgBr
Rdt = 23 % Eb/0,5 = 190°C CCM : 0,45-0,55 ; S.A.
RMN : 0,75 (t,3H) ; 1,90 (q,2H)) ; 2,30 (s,3H) ; 2,85 (d,2H) ; 3,05 (d,2H) ; 4,90-6,30 (m, 5H) ; 7,00-7,60 (m,8H)

Chlorhydrate :

Rdt = 93 % F = 70-80°C (chlorure de méthylène)
Anal. (C22H25Cl2N.HCl) C, H, Cl,N

Exemple 40 : α-cinnamyl-N,N-diméthyl-α-méthyl-benzylamine.

(I.3 ; R1 = C6H5 ; R2 = CH3 ; R3 = R4 = CH3 ; R5 = C6H5)
A partir de 2.a et CH3MgBr
Rdt = 75 % (brut) CCM : 0,50-0,65 ; S.M.
RMN : 1,30 (s,3H)) ; 2,15 (s,6H) ; 2,30-2,85 (m,2H) ; 5,50-6,40 (m,2H) ; 7,00-7,50 (m,10H)

Exemple 41 : α-cinnamyl-N,N-diméthyl-α-isopropyl-benzylamine.

(I.3 ; R1 = C6H5 ; R2 = (CH3)2CH ; R3 = R4 = CH3 ; R5 = C6H5)
A partir de 2.a et (CH3)2CHMgBr
Rdt = 31 % F env. 50°C (éthanol) CCM : 0,60 ; S.A.
RMN : 0,75 (m,6H) ; 2,40 (s,7H) ; 2,85-3,25 (m,2H) ; 6,20-6,60 (m,2H) ; 7,00-7,60 (m,10H)

Chlorhydrate

Rdt = 92 % F = 134°C (acét. d'éthyle)
Anal. (C21H27N.HCl) C, H, Cl, N

Exemple 42 : α-cinnamyl-N,N-diméthyl-α-pentyl-benzylamine.

(I.3 ; R1 = C6H5 ; R2 = CH3-(CH2)4 ; R3 = R4 = CH3 ; R5 = C6H5)
A partir de 2.a et CH3(CH2)4MgBr
Rdt = 25 % (chromatographie) CCM : 0,80 ; S.M.
RMN : 0,70-1,00 (m,3H) ; 1,00-1,50 (m,6H) ; 1,70-2,00 (m,2H) ; 2,25 (s,6H) ; 2,85 (d,2H) ; 6,00-6,60 (m,2H) ; 7,00-7,50 (m,10H)

Exemple 43 : α-(3′,4′ dichloro) cinnamyl-N,N-diméthyl-α-éthyl-p.chlorobenzylamine.

(I.3 ; R1 = p.Cl-C6H4 ; R2 = C2H5 ; R3 = R4 = CH3 ; R5 = 3,4Cl2-C6H3)
A partir de 2.p et C2H5MgBr
Rdt = 50 % (purifié) F = 66°C (éthanol) CCM : 0,80 ; S.A.
RMN : 0,60-0,90 (t,3H) ; 1,65-2,10 (q,2H) ; 2,25 (s,6H) ; 2,80 (d,2H) ; 6,00-6,50 (m,2H) ; 7,00-7,50 (m,7H)

Chlorhydrate :

Rdt : 96 % F = 134°C (méthanol)
Anal. C20H22Cl3N.HCl) C, H, Cl, N

Exemple 44 : α-(3′,4′-dichloro) cinnamyl-N,N-diméthyl-α-éthyl-p.méthylbenzylamine.

(I.3 ; R1 = p.CH3-C6H4 ; R2 = C2H5 ; R3 = R4 = CH3 ; R5 = 3,4Cl2-C6H3)
A partir de 2.q et C2H5MgBr
Rdt = 23 % (purifié) F = 70°C (éthanol) CCM : 0,85 ; S.M.
RMN : 0,80 (t,3H) ; 1,60-2,10 (m,2H) ; 2,20 (s,6H) ; 2,35 (s,3H) ; 2,65-3,05 (m,2H) ; 6,00-6,50 (m,2H) ; 7,00-7,50 (m,7H)

Chlorhydrate :

Rdt : 87 % F = 138°C (méthanol)
Anal. C21H25Cl2N.HCl) C, H, Cl, N

Exemple 45 : α-(3′,4′-dichloro) cinnamyl-N,N-diméthyl-α-éthyl-p.trifluorométhylbenzylamine.

(I.3 ; R1 - F3C-C6H4 ; R2 = C2H5 ; R3 = R4 = CH3 ; R5 = 3,4Cl2-C6H3)
A partir de 2.r et C2H5MgBr
Rdt = 24 % (purifié) F = 66°C (éthanol) CCM : 0,20 ; S.G.
RMN : 0,75 (t,3H) ; 1,80-2,25 (m,2H) ; 2,35 (s,6H) ; 2,65-3,20 (m,2H) ; 5,90-6,50 (m,2H) ; 7,00-7,90 (m,7H)

Chlorhydrate :

Rdt : 85 % F = 150°C (acét. d’éthyle)
Anal. C21H22Cl2F3N.HCl) C, H, Cl, F, N

Exemple 46 : α-(3′,4′-dichloro) cinnamyl-N,N-diméthyl-α-éthyl-p.méthoxybenzylamine.

(I.3 ; R1 = p.CH30-C6H4 ; R2 = C2H5 ; R3 = R4 = CH3 ; R5 = 3,4Cl2-C6H3)
A partir de 2.s et C2H5MgBr
Rdt = 31 % (purifié) F = 90°C (éthanol) CCM : 0,55 ; S.M.
RMN : 0,80 (t,3H) ; 1,90 (q,2H) ; 2,20 (s,6H) ; 2,80 (d,2H) ; 3,80 (s,3H) ; 6,10-6,50 (m, 2H) ; 6,80-7,50 (m,7H)

Chlorhydrate :

Rdt : 90 % F = 210°C (méthanol)
Anal. C21H25Cl2NO.HCl) C, H, Cl, N, 0

Exemple 47 : α-cinnamyl-α-éthyl-N-méthyl-N-propyl-benzylamine.

(I.3 ; R1 = R5 = C6H5 ; R2 = C2H5 ; R3 = CH3 ; R4 = C3H7)

a) sous atmosphère anhydre 15,0 g (57 mmol) d'α-cinnamyl-α-éthyl-N-méthyl-benzylamine (composé de l'exemple 18) sont dissous dans 225 ml de dichlorométhane. Après addition de 6,9 g (68 mmol) de triéthylamine, la solution est refroidie et on ajoute sans dépasser 5°C 6,3 g (68 mmol) de chlorure de propionyle.

La suspension est agitée 2 h 30 à la température ambiante puis extraite successivement par 400 ml de solution d'ammoniaque à 5 %, 400 ml de solution d'HCl à 5 % puis à l'eau jusqu'à neutralité.

Le chlorure de méthylène est évaporé et le résidu huileux (18,8 g) est purifié par chromatographie (composé II.2 de formule II.2.2 R1 = R5 = C6H5 ; R2 = C2H5 ; R3 = CH3 ; R7 = C2H5)

Poids : 16,2 g Rdt : 89 % CCM : 0,40 ; S.G.

RMN : 0,75 (t,3H) ; 1,05 (t,3H) ; 1,75-2,50 (m,4H) ; 2,80-3,60 (m,5H) ; 5,75-6,50 (m,2H) ; 7,10-7,50 (m,10H)

b) Dans un réacteur protégé de l'humidité, on introduit 6,7 g (50 mmol) de chlorure d'aluminium à - 7°C puis rapidement 85 ml d'éther diéthylique. La solution obtenue est maintenue sous agitation. Parallèlement, dans un autre réacteur protégé de l'humidité on introduit 5,7 g (150 mmol) d'hydrure de lithium-aliminium puis à - 70°C, 85 ml de THF anhydre. La suspension est maintenue 20 minutes sous agitation à 8°C puis introduite dans la solution éthérée de chlorure d'aluminium.

Une solution de 16,0 g (50 mmole) de carboxamide préparé en a) dans 40 ml de THF est ajoutée à une température inférieure à 25°C. Le mélange est maintenu une heure à température ambiante puis refroidi à 0°C. Les complexes sont décomposés par addition d'une solution NaOH 10 % puis addition d'eau.

L'insoluble est filtré et le filtrat évaporé sous vide et sur bain-marie. Le résidu huileux jaune pale (13 g) est purifié par chromatographie.

Poids = 12,0 g Rdt = 78 % CCM : 0,80 ; S.H.

RMN : 0,60-0,95 (q,6H) ; 1,20-1,60 (m, 2H) ; 1,70-2,05 (q,2H) ; 2,15-2,60 (m,5H) ; 2,85 (d, 2H) ; 6,00-6,55 (m,10H)

Exemple 48 : α-(3,4 dichloro) cinnamyl-N,N-diméthyl-α-méthyl-benzylamine.

(I.3 ; R1 = C6H5 ; R2 = R3 = R4 = CH3 ; R5 = 3,4Cl2-C6H3)

Dans un ballon on mélange sous agitation et à la température ambiante 8,9 g (29 mmoles) d'α-(3,4 dichloro) cinnamyl-α-méthyl-benzylamine (exemple 9) avec 5,1 ml (68 mmol) de solution aqueuse de formaldéhyde à 37 % (p/v - d = 1,083).

Le mélange hétérogène est agité 30 minutes à la température du laboratoire puis on ajoute goutte à goutte en 5 minutes environ, 3,3 ml (87,5 mmol) d'acide formique pur (d = 1,22). On obtient une solution qui est chauffée sous agitation au bain marie bouillant pendant 1 heure.

La solution est refroidie, on ajoute 50 ml de solution HCl 2N puis extrait par 3 fois 60 ml d'éther. La phase aqueuse acide est alcalinisée à froid jusqu'à pH 12 puis extraite par 3 fois 100 ml d'éther.

Les phases éthérées lavées à l'eau puis séchées sur MgSO4 sont distillées sur bain marie. Le résidu est purifié par chromatographie.

Poids : 4,3 g Rdt = 44 % CCM : 0,45-0,55 ; S.M.

RMN : 1,35 (s,3H) ; 2,20 (s,6H)) ; 2,60 (m,2H) ; 5,70-6,30 (m, 2H) ; 6,80-7,70 (m, 8H).

Chlorhydrate :

Rdt = 87 % F = 174°C (méthanol-éther)

Anal. (C19H21Cl2N.HCl) C, H, Cl,N

Exemple 49 : (-)α-cinnamyl-N,N,-diméthyl-α-éthyl-benzylamine

(I.3 ; R1 = R5 = C6H5 ; R2 = C2H5 ; R3 = R4 = CH3)

Dans un réacteur sous atmosphère d'azote on introduit dans 365 ml d'acétonitrile 18,3 g (0,073 mol) de (+) α-cinnamyl-α éthyl-benzylamine obtenue à l'exemple 16A et 91,0 ml de solution de formaldéhyde à 37 % (p/v) (1,12 mol).

A 0°C et sous agitation on ajoute 13,65 g (0,217 mol) de cyanoborohydrure de sodium. Il se forme deux phases incolores, auxquelles il est ajouté ensuite en 10 minutes environ et à 10°C, 13,53 ml (0,230 mol) d'acide acétique pur. Le mélange est agité 1 heure à 30°C environ, puis à 20°C on ajoute 18 ml de solution de formaldéhyde à 37 % (p/v) (0,220 mol) puis 4,50 g de cyanoborohydrure de sodium (0,072 mol) et 4,55 ml d'acide acétique pur.

Après 1 heure d'agitation à température ambiante on ajoute à nouveau les mêmes réactifs en quantités identiques, puis agite 2 h 30 à température ambiante puis ajoute 700 ml d'éther et décante la phase aqueuse qui est écartée.

La phase organique est lavée par extractions successives avec 200 ml de solution d'hydroxyde de sodium N, puis à l'eau et enfin avec une solution saturée en chlorure de sodium. Après déshydratation et évaporation de l'éther, on obtient un résidu de 21,0 g que l'on purifie par cristallisation dans 60 ml d'éthanol à 95 %.

L'insoluble est filtré et séché sous vide à température ambiante.

Poids : 16,9 g F = 48°C Rdt = 81,4 %

CCM : 0,40-0,50 : S.M.

$\alpha_D^{25}$ = - 21,6° (c = 6,0 % ; MeOH)

RMN : 0,80 (t,3H) ; 1,90 (q,2H)) ; 2,25 (s,6H) ; 2,90 (d,2H) ; 6,00-6,60 (m, 2H) ; 7,10-7,55 (m, 10H).

Chlorhydrate :

Préparé de la façon habituelle, à partir de 16,6 g (0,059 mol) du produit précédent et de 44,0 ml de solution d'éther chlorhydrique environ 4N le produit brut obtenu après évaporation des solvants est purifié par recristallisation dans 90 ml d'acétate d'éthyle. Le précipité est filtré et séché sous vide.

Poids : 14,4 g F = 167°C Rdt = 77,2 %

Anal. (C20H25N,HCl) C, H, Cl, N

$\alpha_D^{25}$ = + 26,7° (c = 2,10 ; H20)

Exemple 50 : (+) α-cinnamyl-N,N-diméthyl-α-éthyl-benzylamine.

(I.3 ; R1 = R5 = C6H5 ; R2 = C2H5 ; R3 = R4 = CH3)

En procédant comme il est indiqué à l'exemple 49 précédent et à partir de 18,3 g (0,073 mol) de (-)α-cinnamyl-α-éthyl-benzylamine préparée à l'exemple 16 B le produit est obtenu et purifié par cristallisation dans l'éthanol à 95 %.

Poids : 12,8 g F = 48°C Rdt = 77,2 %

CCM : 0,40-0,50 ; S.L.

$\alpha_D^{25}$ = + 20,8° (c = 5,8 % ; MeOH)

RMN : 0,80 (t,3H) ; 1,90 (q,2H) ; 2,25 (s,6H) ; 2,90 (d,2H) ; 6,00-6,60 (m,2H) ; 7,10-7,55 (m,10H) ;

Chlorhydrate :

Obtenu comme décrit à l'exemple 49 précédent et à partir de 12,8 g (0,046 mol) de produit et de 34 ml d'éther chlorhydrique environ 4 N, le produit est purifié par recristallisation dans 60 ml d'acétate d'éthyle.

Poids : 11,4 g F = 167°C Rdt = 78,5 %

Anal. (C20H25N,HCl) C, H, Cl, N

$\alpha_D^{25}$ = - 25,8° (c = 2,15 % ; H20)

Exemple 51 : (-) α-cinnamyl-N,N-diméthyl-α-éthyl-p.chlorobenzylamine.

(I.3 ; R1 = p.Cl-C6H4 ; R2 = C2H5 ; R3 = R4 = CH3 ; R5 = C6H5

Le composé est préparé à partir de la (+) α-cinnamyl-α-éthyl-p.chlorobenzylamine de l'exemple 17 A en utilisant le mode opératoire décrit à l'exemple 49.

Rdt = 68 % F = 62°C (éthanol) CCM : 0,70 ; S.L.

$\alpha_D^{20}$ = - 23,7° (c = 2,60 % ; MeOH)

RMN : 0,75 (t,3H) ; 1,85 (q,2H)) ; 2,25 (s,6H) ; 2,80 (d,2H) ; 5,95-6,55 (m, 2H) ; 7,10-7,45 (m, 9H).

Exemple 52 : (+) α-cinnamyl-N,N-diméthyl-α-éthyl-p.chlorobenzylamine.

(I.3 ; R1 = p.Cl-C6H4 ; R2 = C2H5 ; R3 = R4 = CH3 ; R5 = C6H5

A partir de (-) α-cinnamyl-α-éthyl-p.chlorobenzylamine de l'exemple 17 B et selon le mode opératoire de l'exemple 49.

Rdt = 59 % F = 62°C (éthanol) CCM : 0,70 ; S.L.

$\alpha_D^{20}$ = + 24,2° (c = 2,55 %; MeOH)

RMN : 0,75 (t,3H) ; 1,85 (q,2H)) ; 2,25 (s,6H) ; 2,80 (d,2H) ; 5,95-6,55 (m, 2H) ; 7,10-7,45 (m, 9H).

Les essais toxicologiques et pharmacologiques réalisés avec les benzylamines de l'invention de formule (I) décrites dans les exemples 3 à 52 qui précèdent mettent en évidence leur faible toxicité de même que d'intéressantes propriétés psychotropes qui rendent ces composés utiles pour le traitement des affections neuropsychiques.

Par ailleurs, certains essais mettent en évidence le groupe de composés de formule (I.A) et définis par des significations particulières précédemment citées de R1, R3/R4 et R5, une activité de liaison "in vitro" aux récepteurs opiacés et plus particulièrement aux récepteurs mu. Cette affinité se traduisant "in vivo" par une activité sur le tractus urinaire qui consiste en une action sur les paramètres vésicaux du rat.

L'étude de la toxicité des produits de l'invention est recherchée chez la souris par voie orale par la détermination approchée de leur DL 50, qui est la dose léthale provoquant 50% de morts chez les animaux dans les conditions de l'expérience. Elle est réalisée sur des lots de quatre souris "Swiss" mâles d'un poids de 20g environ mises à jeun la veille de l'éssai.

Chaque détermination est effectuée avec quatre doses de produits correspondant respectivement à une administration de 100, 300, 600 et 1000 mg de produit, exprimé sous forme de base, par kg d'animal.

Il est ainsi constaté que les produits de l'invention ont en général une toxicité aigüe de DL50 supérieure ou égale à 1.000 mg/kg. Exceptionnellement, quelques composés présentent une DL50 d'environ 600 mg/kg.

Les propriétés psychotropes des composés ont été determinées par la protection des convulsions induites par la picrotoxine chez la souris, qui a été réalisée selon une méthode dérivée de cell de Krall et coll., "Epilepsia", 1978, 19, p.409-428.

L'administration de picrotoxine provoque chez l'animal une crise convulsive caractérisée par un syndrome d'extension myoclonique suivi de l'extension des membres conduisant à la mort de l'animal. Certaines substances, notamment celles actives sur le complexe GABA/benzodiazépines/Cl-ionophore permettent de protéger les animaux de cette crise convulsive.

Pratiquement l'étude est réalisée sur des lots de 10 souris "Swiss" mâles, d'un poids de 20 g environ auxquelles on administre le produit à étudier en solution aqueuse soit par voie intrapéritonéale (i.p.) à raison de 50 mg/kg et sous un volume de 0,2 ml de solution par animal, soit par voie orale (p.o) sous un volume de 2,0 ml de solution par animal.

On pratique ensuite une injection par voie intrapéritonéale d'une solution de picrotoxine à raison de 24 mg/kg sous un volume de 0,2ml par animal, soit 30 minutes après l'administration du produit par voie intrapéritonéale, soit 60 minutes après l'administration du produit par voie orale. La dose de produit injectée provoquant une crise clonique qui conduit à la mort des animaux non traités. Dans les conditions du test, on observe chez les animaux traités la suppression de la phase tonique d'extension. Les résultats sont exprimés :

- soit en pourcentages d'animaux protégés de cette phase sous l'action de 50 mg/kg du composé à l'étude administrée par voie i.p. ou 100 mg/kg par voie p.o.,
- soit en DE50 pour chacune de ces voies, qui est la dose efficace de composé à l'essai, exprimée en mg/kg protégeant 50 % des animaux de cette phase d'extension. La valeur significative des résultats étant généralement indiqué de la façon suivante :

| | | | |
|---|---|---|---|
| * | Résultat significatif | | à p. < 0,05 |
| ** | " | " | à p. < 0,01 |
| *** | " | hautement significatif | à p. < 0,001 |

Les résultats de l'étude sont reportés pour les produits de l'invention de formules (I.1), (I.2) et (I.3) dans les tableaux 1, 2 et 3 qui suivent :

**Tableau 1 : Résultats - Composés I.1.**

| Exemple | i.p. : % prot. ou DE50 | p.o. : % prot. ou DE50 |
|---|---|---|
| 3 | 80 % *** | 100 % *** |
| 4 | 60 % * | N.T. |
| 5 | 50 % * | N.T. |
| 6 | 70 % ** | 70 % *** |
| 7 | 100 % *** | N.T. |
| 8 | 80 % *** | N.T. |
| 9 | 100 % *** | N.T. |
| 10 | 90 % *** | N.T. |
| 11 | 70 % ** | 100 % *** |
| 12 | DE50 = 37,3 | DE50 = 50,0 |
| 13 | DE50 = 23,2 | 50 % *** |
| 15 | DE50 > 25,0 | N.T. |
| 16A | 90 % *** | 100 % *** |
| 16B | 100 % *** | 100 % *** |
| 17A | 80 % *** | 50 % * |
| 17B | 80 % *** | 50 % * |

N.T. Non testé

Tableau 2 : Résultats - Composés I.2.

| Exemple | i.p. : % prot. ou DE50 | p.o. : % prot. ou DE50 |
|---|---|---|
| 18 | 80 % *** | 100 % *** |
| 19 | 70 % ** | N.T. |
| 20 | N.T | 80 % *** |
| 21 | 90 % *** | 70 % ** |
| 22 | 80 % *** | 100 % *** |
| 23 | 60 % * | 100 % *** |
| 24 | N.T. | DE50 = 73,5 % |
| 25 | N.T. | 50 % * |
| 28 | 100 % *** | 100 % *** |
| 29 | 100 % *** | 100 % *** |

N.T. non testé

Tableau 3 : Résultats - Composés I.3.

| Exemple | i.p. : % prot. ou DE50 | p.o. : % prot. ou DE50 |
|---|---|---|
| 30 | 100 % *** | 100 % *** |
| 31 | 100 % *** | 50 % * |
| 32 | 50 % * | 60 % * |
| 34 | 80 % *** | 60 % * |
| 35 | 90 % *** | DE50 = 40,3 |
| 36 | N.T. | 100 % *** |
| 38 | 80 % *** | N.T. |
| 40 | DE50 = 16,6 | DE50 = 37,7 |
| 41 | N.T. | DE50 = 78,4 |
| 42 | N.T. | DE50 = 65,0 |
| 44 | DE50 = 21,4 | DE50 = 23,9 |
| 46 | DE50 = 29,8 | DE50 = 35,4 |
| 47 | DE50 = 58,2 | DE50 = 65,0 |
| 48 | 90 % *** | N.T. |
| 49 | 100 % *** | 100 % *** |
| 50 | 100 % ** | 100 % *** |

N.T. Non testé

Ces résultats mettent en évidence, pour les produits de l'invention étudiés, et indépendamment de la voie d'administration utilisée une activité dans le test de protection aux convulsions induites par la picrotoxine chez la souris.

En outre, et tel qu'il a été précédemment annoncé, le groupe de composés (IA) qui sont actifs "in vitro" sur les récepteurs opiacés.

Leur étude est réalisée selon la technique décrite par F. Roman et coll. dans J. Pharm. Pharmacol. 1987, 39, p. 404-407 qui consiste à étudier sur des récepteurs membranaires de cobaye le déplacement de ligands radioactifs spécifiques des sous récepteurs opiacés mu, delta et kappa par l'action des produits à l'essai.

Les résultats de l'étude sont présentés au tableau 4 et sont exprimés en CI50 qui sont les concentrations nanomolaires des produits en solution capables d'inhiber 50 % des liaisons du ligand radioactif spécifique lié au récepteur considéré. La morphine a été engagée dans cet essai à titre de produit de référence.

**Tableau 4 : Affinité de liaison des composés (IA) de l'invention aux récepteurs mu, delta et kappa**

| Exemple | Affinité CI50 | | |
| --- | --- | --- | --- |
| | rec. mu | rec. delta | rec. kappa |
| 17B | 11,4 | N.T. | N.T. |
| 19 | 200 | 3490 | 264 |
| 23 | 80 | 1380 | 340 |
| 24 | 21 | 1405 | 410 |
| 25 | 56 | 1591 | 875 |
| 26 | 28 | 1072 | 458 |
| 31 | 80 | 1340 | 210 |
| 33 | 50 | 1230 | 120 |
| 35 | 60 | 1280 | 290 |
| 36 | 90 | 680 | 220 |
| 37 | 100 | 150 | 230 |
| 43 | 16 | 434 | 197 |
| 44 | 4 | 223 | 110 |
| 46 | 12 | 260 | 235 |
| 52 | 7 | N.T. | 99 |
| Morphine | 7 | 152 | 127 |

Bien que globalement inférieures à celles montrées par la morphine, les affinités des composés aux récepteurs sont cependant significatives. Pour les composés des exemples 43, 44 et 46, elles atteignent le même ordre d'intensité que le produit de référence.

Egalement d'une manière générale les composés présentent une afinité préférentielle mais non spécifique pour les récepteurs mu. A un degré moindre ils sont aussi actifs sur les récepteurs kappa et beaucoup moins sur les récepteurs delta.

L'activité des composés opiacés "in vivo" sur le tractus urinaire a été décrite par divers auteurs. C. Soulard et coll. dans "Advanced in the Biosciences" Vol. 75,1989, montrent que sur la vessie du rat les composés d'affinité mu et les composés d'affinité kappa ont des activités différentes.

Ainsi les composés mu inhibent la motilité vésicale, les composés kappa l'activent, et il est aussi montré que les composés d'affinité mixte mu-kappa ont une activité inhibitrice de cette motilité.

Etudiés selon un mode opératoire semblable, les composés de l'invention (IA) qui montrent une affinité mu et kappa ont également chez le rat une activité sur certains paramètres fonctionnels de la vessie ce qui justifie leur intérêt pour le traitement de dysfonctionnements du tractus urinaire.

L'étude réalisée consiste en une cystomanométrie transvésicale chez le rat éveillé. Sous perfusion continue intravésicale de sérum physiologique stérile, on mesure, durant les phases de stockage et d'émission d'urine :

- la "pression seuil" qui est la pression intravésicale qui précède immédiatement la phase de contraction du détrusor
- le volume de chaque émission urinaire.

La technique employée consiste, chez le rat femelle, à préparer les animaux 48 h avant l'expérience proprement dite, en réalisant l'implantation sous anesthésie de deux catéthers dans l'apex de la vessie et d'un cathéter dans la veine jugulaire gauche, l'extrêmité extérieure de ces cathéters étant protégée et maintenue par un corset de sparadrap.

Au moment de l'essai les animaux sont placés dans des cages appropriées qui sont équipées pour pourvoir recueillir et mesurer les urines. Pour chaque animal, un cathéter vésical est relié à un capteur de pression, l'autre à un perfuseur qui délivre en continu 0,2 ml/min de sérum physiologique stérile.

La pression intravésicale est mesurée en continu durant la perfusion et permet d'observer :

- une phase de remplissage de la vessie durant laquelle la pression augmente progressivement,
- une phase d'élévation brusque de cette pression durant la contraction du détrusor : la pression de "seuil" qui est la pression observée juste avant la pression de la phase de contraction. Cette pression de seuil est relevée de même que le volume urinaire à chaque miction.

L'étude consiste à observer la modification de ces paramètres sous l'action des produits (IA) à l'essai qui sont administrés en solution par voie intraveineuse au moyen du cathéter placé dans la jugulaire gauche.

A titre de produits de comparaison la Dicyclomine et le Baclofen qui ont été envisagés pour le traitement des vessies hyperactives (Drugs of Today, Vol. 24, n° 5, 1988, p. 337-348) sont aussi engagés dans cet essai.

Tableau 5 : Activité urologique

| Exemple | Dose i.v. mg/kg | % variation pression seuil | % variation volume |
|---|---|---|---|
| 19 | 5,0 | + 354 | + 54 |
| 23 | 5,0 | + 196 | + 35 |
| 25 | 5,0 | + 85 | + 67 |
| 26 | 5,0 | + 229 | + 93 |
| 31 | 0,5 | + 73 | + 44 |
| 33 | 0,5 | + 199 | + 27 |
| 35 | 1,0 | + 200 | + 39 |
| 36 | 1,0 | + 125 | + 36 |
| 37 | 5,0 | + 188 | + 53 |
| 43 | 1,0 | + 109 | + 91 |
| 44 | 0,5 | + 183 | + 56 |
| 46 | 1,0 | + 111 | + 44 |
| 52 | 0,5 | + 72 | + 30 |
| Dicyclomine | 2,5 | + 28 | + 20 |
| Baclofen | 2,5 | + 113 | + 32 |

Ces résultats sont probants de l'activité de ces composés de l'invention sur le tractus urinaire de l'animal. On remarque plus particulièrement l'activité intéressante des composés des exemples 31, 33, 35, 36, 43, 44,

46 et 52 de l'invention comparée à celle des composés de référence.

Ainsi, par rapport au Baclofen, qui est le produit de comparaison le plus actif dans ces essais, et en tenant compte des doses de composés effectivement administrés, les composés des exemples 33 et 44 ont une activité de 5 à 10 fois supérieure sur la pression seuil alors que pour la variation de volume les produits des exmples 43 et 44 apparaissent de 5 à 10 fois plus actifs.

En conséquence des études pharmacologiques qui viennent d'être décrites l'ensemble des composés (I) sont utiles pour le traitement des affections neuro-psychiques, et, sous forme de médicaments, peuvent permettre le traitement des sujets qui présentent des troubles de l'humeur et/ou du comportement.

Plus particulièrement les composés (IA) précédemment définis possèdent "in vitro" une affinité de liaison aux récepteurs opiacés et notamment aux récepteurs mu et kappa qui apparaît correspondre à une activité remarquable sur le tractus urinaire au niveau de la vessie. Ces produits sont appropriés à la thérapie du dysfonctionement des vessies instables et sous forme de médicaments ils trouvent leur application au traitement des troubles de la miction et de l'incontinence.

Présentés sous les formes pharmaceutiques, de façon habituelle les doses unitaires utiles sont comprises entre 1 et 500 mg et plus particulièrement entre 5 et 200 mg de produit selon la nature et la gravité de l'affection a traiter. Les doses thérapeutiques journalières peuvent être réparties en plusieurs prises et sont comprises entre 5 et 2000 mg de produit par jour. De façon générale une posologie journalière de 50 à 500 mg de produit par jour répartis en deux à quatre prises est satisfaisante.

L'administration des produits de l'invention aux patients à traiter est réalisée sous la forme de médicaments de nature adaptée à l'affection à soigner.

Selon les cas les préparations médicamenteuses seront, comme exemples non limitatifs, des comprimés, dragées, capsules, poudres, solutions, suspensions, gels ou suppositoires. Ces formes pharmaceutiques sont préparées à partir des produits sous forme de base ou de leurs sels et selon des méthodes couramment pratiquées dans cette industrie.

Généralement dans les formes médicamenteuses de nature solide, le principe actif représente de 5 à 90 % en poids du total de la forme terminée alors que les excipients représentent de 95 à 10 %. Pour les formes liquides, ou pouvant être considérées comme telles, la quantité de principe actif est comprise entre 0,1 et 10 % en poids de la forme terminée alors que les excipients peuvent représenter de 99,9 à 90 % en poids de cette forme.

Comme exemples, il est présenté la formulation et la préparation des solutés isotoniques injectables, celle de comprimés et celle de gels administrables par voie orale.

Soluté isotonique injectable

- Formule :

| | |
|---|---|
| Substance active de l'exemple 9 (chlorhydrate) | 10 mg |
| Chlorure de sodium | 9 mg |
| Eau distillée en quantité suffisante pour | 1,0 ml |

- Préparation :

Le soluté isotonique est réparti en ampoules de volume approprié qui sont, après scellement, stérilisée par des moyens thermiques connus en soi, ou bien le soluté est stérilisé par filtration, réparti en ampoules qui sont ensuite scellées, l'ensemble des opérations étant effectué sous atmosphère stérile.

Dans ce dernier cas, on préfère ajouter à la formule décrite, 1% d'alcool benzylique à titre d'agent bactériostatique, soit 10 mg de cet alcool par ml de soluté.

Comprimés

- Formule :

| | |
|---|---|
| Substance active de l'exemple 31 | 10,0 à 50,0 mg |
| Polyvinylpyrrolidone | 20,0 mg |
| Carboxyméthylamidon | 8,0 mg |
| Stéarate de magnésium | 2,0 mg |
| Silice colloidale | 0,4 mg |
| Lactose en quantité suffisante pour | 200,0 mg |

- Préparation

Le principe actif est mélangé au lactose puis granulé avec la polyvinylpyrrolidone en solution. Les grains sont séchés et tamisés sur une grille d'ouverture de 1 mm. Le carboxyméthylamidon est mélangé à la silice colloidale puis ajouté aux granulés. On mélange ensuite intimement avec le stéarate de magnésium puis comprime à raison de 200,0 mg par comprimé.

Gel

- Formule :

| | |
|---|---|
| Substance active de l'exemple 30 (chlorhydrate) | 0,20 à 0,60 g |
| Hydroxypropylcellulose | 2,00 g |
| Saccharinate de sodium | 0,01 g |
| Sirop de sorbitol à 70% (p/v) | 25,00 g |
| Arome naturel de fraise | 0,50 g |
| Conservateur | 0,10 g |
| Eau purifiée en quantité suffisante pour | 100,00 g |

- Préparation :

Les conservateurs et le saccharinate de sodium sont dissous dans l'eau, puis, sous agitation, on ajoute en la dispersant l'hydroxypropylcellulose. L'agitation est maintenue jusqu'à obtention d'un gel auquel, toujours sous agitation, on ajoute le sirop de sorbitol puis finalement l'arome.

**Revendications**

**Revendications pour les Etats contractants suivants : AT, BE, CH, DE, FR, GB, IT, LI, LU, NL, SE**

1. Benzylamines disubstituées de formule

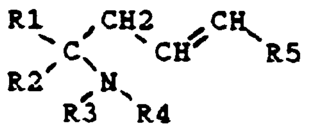

(I)

dans laquelle:

- R1 est phényle éventuellement mono-, ou di-substitué par des atomes d'halogène, par des radicaux alkyle inférieurs, haloalkyle inférieurs ou alkoxy inférieurs,
- R2 est alkyle inférieur,
- R3 et R4, identiques ou différents, sont l'hydrogène, des radicaux alkyle inférieur ou alkènyle inférieur,
- R5 est phényle éventuellement mono-, di- ou tri-substitué par des atomes d'halogène ou par des radicaux alkoxy inférieurs, leurs sels d'addition avec acides et leurs formes optiquement actives.

2. Benzylamines suivant la revendication 1, caractérisées en ce que R1 est phényle.

3. Benzylamines suivant les revendications 1 ou 2, caractérisées en ce que R5 est phényle.

4. Benzylamines suivant l'une des revendications 1 à 3, caractérisées en ce que R2 est éthyle.

5. Benzylamines suivant l'une des revendications 1 à 4, caractérisées en ce que R3 est l'hydrogène ou méthyle et R4 est l'hydrogène ou méthyle

6. Benzylamine suivant la revendication 1, caractérisé en ce que l'un, au moins, de R1 et de R5 est un phényle substitué, et R3 et R4 identiques ou différents sont l'hydrogène ou alkyle inférieur, R3 et R4 n'étant pas tous deux l'hydrogène.

7. Procédé de préparation des benzylamines de formule (I), caractérisé en ce qu'il consiste :
- pour obtenir une benzylamine de formule (I.1), répondant à la formule (I), dans laquelle R3 et R4 sont l'hydrogène, à hydrolyser un isocyanate de formule :

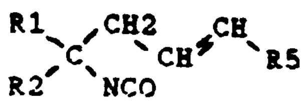

(II.1)

- pour obtenir une benzylamine de formule (I.2), répondant à la formule (I) dans laquelle R3 est méthyle et R4 est l'hydrogène,
  i) à réduire par un hydrure métallique un isocyanate de formule (II.1) ou
  ii) à acyler une benzylamine (I.1) par l'acide formique en présence de carbonyldiimidazole en le composé intermédiaire

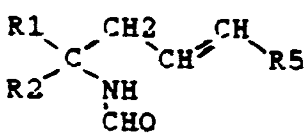

  puis à réduire ce composé intermédiaire par un hydrure métallique,
- pour obtenir une benzylamine de formule (I.2), répondant à la formule (I) dans laquelle R3 est autre que l'hydrogène et R4 est l'hydrogène.
  i) à alkyler une benzylamine de formule (I.1) par un halogénure de formule Z1R3 dans laquelle R3 est autre que l'hydrogène et Z1 est le chlore, le brome ou l'iode, ou
  ii) dans une première étape, à acyler une benzylamine (I.1) par un agent d'acylation de formule (R6-CO)n Z2 dans laquelle R6 est l'homologue immédiatement inférieur de R3 (R3=CH2R6), et Z2 est soit hydroxyle, soit le brome ou le chlore lorsque n est 1 et est l'oxygène quand n est 2 pour obtenir un carboxamide intermédiaire de formule

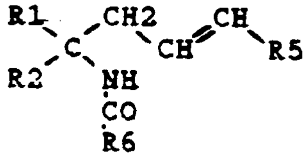

(II.2.1)

que l'on réduit par un hydrure métallique dans une seconde étape

- pour obtenir une benzylamine de formule (I.3) dans laquelle à la fois R3 et R4 sont méthyle, à diméthyler une benzylamine (I.1) en la faisant réagir sur du formaldéhyde et de l'acide formique selon la réaction de Eschweiler-Clarke ou sur du formaldéhyde en présence d'un agent réducteur,
- pour obtenir une benzylamine de formule (I.3), répondant à la formule (I) dans laquelle R3 n'est pas l'hydrogène et R4 n'est ni hydrogène ni méthyle,

i) dans une première étape, à acyler une benzylamine de formule (I.2) dans laquelle R3 est autre que l'hydrogène par un agent d'acylation de formule R7CO Z5 dans laquelle R7 est l'homologue immédiatement inférieur de R4 (R4 = -CH2-R7) et Z5 est le brome ou le chlore pour obtenir un carboxamide disubstitué sur l'azote de formule

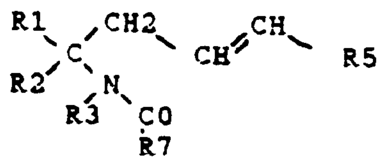

(II.2)

que l'on réduit, dans une seconde étape, par un hydrure métallique,

ii) ou à faire réagir un réactif organomagnésien de formule R2MgZ3.

Z3 étant un halogène, sur un nitrile de formule

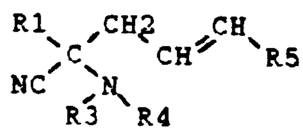

(II.3)

- pour obtenir une benzylamine de formule (I.3) répondant à la formule (I) dans laquelle R3 n'est pas l'hydrogène et R4 est méthyle, à N-méthyler une benzylamine de formule (I.2) dans laquelle R3 est alkyle ou alkényle inférieur par de l'aldéhyde formique en présence d'un réducteur tel qu'un hydrure métallique ou organo métallique.

**8.** Médicament, notamment psychotrope, caractérisé en ce qu'il comprend une benzylamine suivant l'une des revendications 1 à 5.

**9.** Médicament psychotrope et actif sur le tractus urinaire caractérisé en ce qu'il comprend une benzylamine suivant la revendication 6.

**10.** Composés intermédiaires de formule :

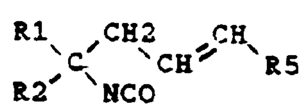

(II.1)

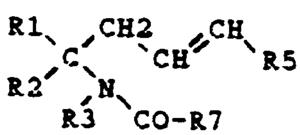

(II.2)

ou

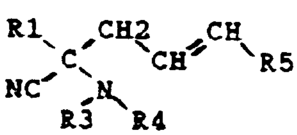

(II.3)

dans lesquelles R1 à R5 ont les significations indiquées à la revendication 1, R3 et R4 n'étant pas l'hydrogéne dans le cas des nitriles (II.3), R7 étant un homologue immédiatement inférieur à R4.

**11.** Procédé de préparation des composés intermédiaires de la revendication 9 caractérisé en ce qu'il consiste

- pour préparer les composés (II.1), à faire réagir un composé de départ de formule (VIII) R1-CH2-W dans laquelle R1 est tel que défini pour les benzylamines (I) et W est un radical nitrile (-CN) ou carboxy (-COOH) sur un halogènure d'alkyle de formule R2Z6, R2 étant tel que défini pour les composés (I) et Z6 étant un halogène, pour obtenir, quand W est carboxy un acide de formule (VI)

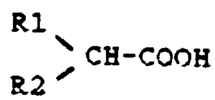

et, quand W est nitrile, un nitrile de formule (VII)

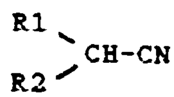

que l'on hydrolyse en l'acide de formule (VI), puis à alkyler l'acide (VI) par un halogènure d'alkènyle (V) de formule Z7-CH2-CH=CH-R5, Z7 étant un halogène et R5 étant tel que défini pour les benzylamines (I) pour obtenir un acide à chaîne insaturée (III) que l'on transpose en un isocyanate (II.1) par une réaction de Curtius.

- pour préparer les composés (II.2),

i) à acyler une benzylamine de formule

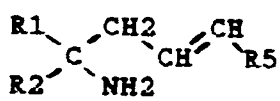 (I.1)

par un réactif de formule (R6C0)nZ2 dans lequel R6 est l'homologue carboné directement inférieur à R3 (R3 = CH2-R6) et dans lequel Z2 est halogène ou hydroxyle quand n = 1 et Z2 est l'oxygène quand n = 2, pour obtenir un carboxamide intermédiaire (II.2.1)

ii) à acyler une benzylamine de formule

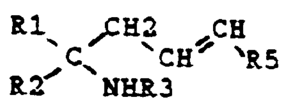

par un agent d'acylation de formule R7CO Z5, dans laquelle R7 est alkyle inférieur ou alkényle inférieur et Z5 est le brome ou le chlore pour obtenir un carboxamide intermédiaire (II.2.2)

- pour préparer les composés (II.3) à faire réagir un aldéhyde de formule (XI) R1-CHO avec une amine de formule R3-NH-R4 et un cyanure de métal alcalin pour obtenir un aminonitrile de formule

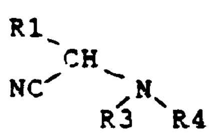 (X)

R4 et R3 étant tels que définis pour les benzylamines (I), mais n'étant pas l'hydrogène, puis, à alkyler l'aminonitrile (X) par un halogénure d'alkényle (V)

**Revendications pour l'Etat contractant suivant : ES**

**1.** Procédé de préparation des benzylamines disubstituées de formule

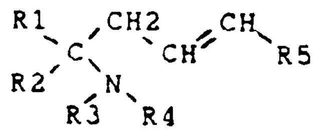

(I)

dans laquelle :

- R1 est phényle éventuellement mono-, ou disubstitué par des atomes d'halogène, par des radicaux alkyle inférieurs, haloalkyle inférieurs ou alkoxy inférieurs,
- R2 est alkyle inférieur,
- R3 et R4, identiques ou différents, sont l'hydrogène, des radicaux alkyle inférieur ou alkènyle inférieur,
- R5 est phényle éventuellement mono-, di- ou tri-substitué par des atomes d'halogène ou par des radicaux alkoxy inférieurs, de leurs sels d'addition avec des acides et de leurs formes optiquement actives, caractérisé en ce qu'il consiste :
  - pour obtenir une benzylamine de formule (I.1), répondant à la formule (I), dans laquelle R3 et R4 sont l'hydrogène, à hydrolyser un isocyanate de formule :

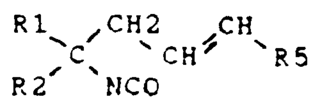

(II.1)

  - pour obtenir une benzylamine de formule (I.2), répondant à la formule (I) ans laquelle R3 est méthyle et R4 est l'hydrogène,
    i) à réduire par un hydrure métallique un isocyanate de formule (II.1) ou
    ii) à acyler une benzylamine (I.1) par l'acide formique en présence de carbonyldiimidazole en le composé intermédiaire

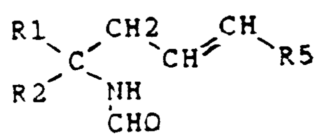

puis à réduire ce composé intermédiaire par un hydrure métallique,

  - pour obtenir une benzylamine de formule (I.2) répondant à la formule (I) dans laquelle R3 est autre que l'hydrogène et R4 est l'hydrogène.
    i) à alkyler une benzylamine de formule (I.1) par un halogénure de formule Z1R3 dans laquelle R3 est autre que l'hydrogène et Z1 est le chlore, le brome ou l'iode, ou
    ii) dans une première étape, à acyler une benzylamine (I.1) par un agent d'acylation de formule (R6-CO)n Z2 dans laquelle R6 est l'homologue immédiatement inférieur de R3 (R3=-CH2R6), et Z2 est soit hydroxyle, soit le brome ou le chlore lorsque n est 1 et est l'oxygène quand n est 2 pour obtenir un carboxamide intermédiaire de formule

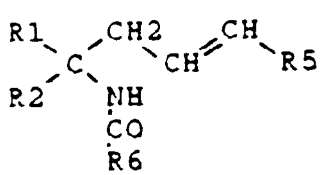

(II.2.1)

que l'on réduit par un hydrure métallique dans une seconde étape

  - pour obtenir une benzylamine de formule (I.3) dans laquelle à la fois R3 et R4 sont méthyle, à diméthyler une benzylamine (I.1) en la faisant réagir sur du formaldéhyde et de l'acide formqiue selon

la réaction de Eschweiler-Clarke ou sur du formaldéhyde en présence d'un agent réducteur,
- pour obtenir une benzylamine de formule (I.3), répondant à la formule (I) dans laquelle R3 n'est pas l'hydrogène et R4 n'est ni hydrogène ni méthyle,

i) dans une première étape, à acyler une benzylamine de formule (I.2) dans laquelle R3 est autre que l'hydrogène par un agent d'acylation de formule R7CO Z5 dans laquelle R7 est l'homologue immédiatement inférieur de R4 (R4 = -CH2-R7) et Z5 est le brome ou le chlore pour obtenir un carboxamide disubstitué sur l'azote de formule

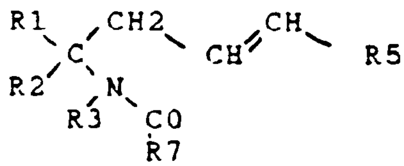

(II.2)

que l'on réduit, dans une seconde étape, par un hydrure métallique,

ii) ou à faire réagir un réactif organomagnésien de formule R2Mg Z3,

Z3 étant un halogène, sur un nitrile de formule

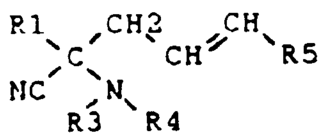

(II.3)

- pour obtenir une benzylamine de formule (I.3) répondant à la formule (I) dans laquelle R3 n'est pas l'hydrogène et R4 est méthyle, à N-méthyler une benzylamine de formule (I.2) dans laquelle R3 est alkyle ou alkènyle inférieur par de l'aldéhyde formique en présence d'un réducteur tel qu'un hydrure métallique ou organo métallique.
- pour obtenir un sel d'addition avec un acide à salifier la benzylamine par un acide et pour obtenir une forme optiquement active à dédoubler un racémique.

2. Procédé suivant la revendication 1, caractérisé en ce que R1 est phényle.

3. Procédé suivant les revendications 1 ou 2, caractérisé en ce que R5 est phényle.

4. Procédé suivant l'une des revendications 1 à 3, caractérisé en ce que R2 est éthyle.

5. Procédé suivant l'une des revendications 1 à 4, caractérisé en ce que R3 est l'hydrogène ou méthyle et R4 est l'hydrogène ou méthyle.

6. Procédé suivant la revendication 1, caractérisé en ce que l'un, au moins, de R1 et de R5 est un phényle substitué, et R3 et R4 identiques ou différents sont l'hydrogène ou alkyle inférieur, R3 et R4 n'étant pas tous deux h'hydrogène.

7. Procédé de préparation d'un médicament, notamment psychotrope, caractérisé en ce qu'il consiste à mélanger une benzylamine ou un de ses sels, telle que définie aux revendications 1 à 5 à un excipient pharmaceutique.

8. Procédé de préparation d'un médicament psychotrope et actif sur le tractus urinaire, caractérisé en ce qu'il consiste à mélanger une benzylamine ou un de ses sels, telle que définie à la revendication 6 à un excipient pharmaceutique.

9. Procédé de préparation des composés intermédiaires de formule

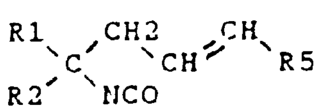

(II.1)

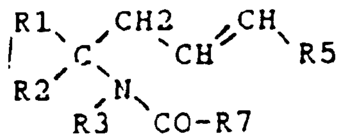

(II.2)

ou

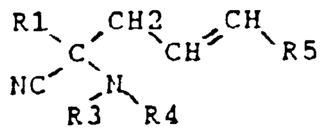

(II.3)

dans lesquelles R1 à R5 ont les significations indiquées à la revendication 1, R3 et R4 n'étant pas l'hydrogène dans le cas des nitriles (II.3), R7 étant un homologue immédiatement inférieur à R4, caractérisé en ce qu'il consiste

- pour préparer les composés (II.1), à faire réagir un composé de départ de formule (VIII) R1-CH2-W dans laquelle R1 est tel que défini pour les benzylamines (I) et W est un radical nitrile (-CH) ou carboxy (-COOH) sur un halogènure d'alkyle de formule R2Z6, R2 étant tel que défini pour les composés (I) et Z6 étant un halogène, pour obtenir, quand W est carboxy un acide de formule (VI)

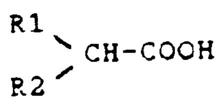

et, quand W est nitrile, un nitrile de formule (VII)

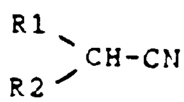

que l'on hydrolyse en l'acide de formule (VI), puis à alkyler l'acide (VI) par un halogènure d'alkènyle (V) de formule Z7-CH2-CH=CH-R5, Z7 étant un halogène et R5 étant tel que défini pour les benzylamines (I) pour obtenir un acide à chaîne insaturée (III) que l'on transpose en un isocyanate (II.1) par une réaction de Curtius.

- pour préparer les composés (II.2),

i) à acyler une benzylamine de formule

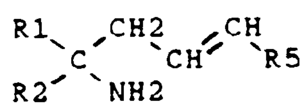

(I.1)

par un réactif de formule (R6CO)nZ2 dans lequel R6 est l'homologue carboné directement inférieur à R3 (R3 = CH2=R6) et dans lequel Z2 est halogène ou hydroxyle quand n = 1 et Z2 est l'oxygène quand n = 2, pour obtenir un carboxamide intermédiaire (II.2.1)

ii) à acyler une benzylamine de formule

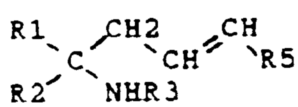

par un agent d'acylation de formule R7CO Z5, dans laquelle R7 est alkyle inférieur ou alkényle inférieur et Z5 est le brome ou le chlore pour obtenir un carboxamide intermédiaire (II.2).

- pour préparer les composés (II.3) à faire réagir un aldéhyde de formule (XI) R1-CHO avec une amine de formule R3-NH-R4 et un cyanure de métal alcalin pour obtenir un aminonitrile de formule

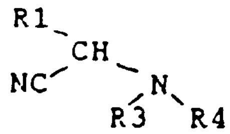

(X)

R4 et R3 étant tels que définis pour les benzylamines (I), mais n'étant pas l'hydrogène, puis, à alkyler l'aminonitrile (X) par un halogènure d'alkényle (V).

**10.** L'utilisation d'une benzylamine telle que définie aux revendications 1 à 5 ou de l'un de ses sels acceptables thérapeutiquement pour l'obtention d'un médicament psychotrope.

**11.** L'utilisation d'une benzylamine telle que définei à la revendication 6 ou de l'un de ses sels acceptables thérapeutiquement pour l'obtention d'un médicament psychotrope et actif sur le tractus urinaire.

**12.** L'utilisation d'un composé intermédiaire tel que préparé à la revendication 9 comme intermédiaire de synthèse de benzylamines.

**Revendications pour l'Etat contractant suivant : GR**

**1.** Benzylamines disubstituées de formule

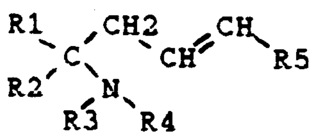

(I)

dans laquelle:

- R1 est phényle éventuellement mono-, ou di-substitué par des atomes d'halogène, par des radicaux alkyle inférieurs, haloalkyle inférieurs ou alkoxy inférieurs,
- R2 est alkyle inférieur,
- R3 et R4, identiques ou différents, sont l'hydrogène, des radicaux alkyle inférieur ou alkènyle inférieur,
- R5 est phényle éventuellement mono-, di- ou tri-substitué par des atomes d'halogène ou par des radicaux alkoxy inférieurs, leurs sels d'addition avec acides et leurs formes optiquement actives.

**2.** Benzylamines suivant la revendication 1, caractérisées en ce que R1 est phényle.

**3.** Benzylamines suivant les revendications 1 ou 2, caractérisées en ce que R5 est phényle.

**4.** Benzylamines suivant l'une des revendications 1 à 3, caractérisées en ce que R2 est éthyle.

**5.** Benzylamines suivant l'une des revendications 1 à 4, caractérisées en ce que R3 est l'hydrogène ou méthyle et R4 est l'hydrogène ou méthyle

**6.** Benzylamine suivant la revendication 1, caractérisé en ce que l'un, au moins, de R1 et de R5 est un phényle substitué, et R3 et R4 identiques ou différents sont l'hydrogène ou alkyle inférieur, R3 et R4 n'étant pas tous deux l'hydrogène.

**7.** Procédé de préparation des benzylamines de formule (I), caractérisé en ce qu'il consiste :

- pour obtenir une benzylamine de formule (I.1), répondant à la formule (I), dans laquelle R3 et R4 sont l'hydrogène, à hydrolyser un isocyanate de formule :

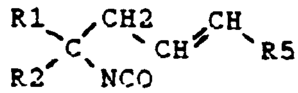

(II.1)

- pour obtenir une benzylamine de formule (I.2), répondant à la formule (I) dans laquelle R3 est méthyle et R4 est l'hydrogène,

i) à réduire par un hydrure métallique un isocyanate de formule (II.1) ou

ii) à acyler une benzylamine (I.1) par l'acide formique en présence de carbonyldiimidazole en le composé intermédiaire

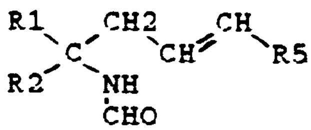

puis à réduire ce composé intermédiaire par un hydrure métallique,

- pour obtenir une benzylamine de formule (I.2), répondant à la formule (I) dans laquelle R3 est autre que l'hydrogène et R4 est l'hydrogène.

i) à alkyler une benzylamine de formule (I.1) par un halogénure de formule Z1R3 dans laquelle R3 est autre que l'hydrogène et Z1 est le chlore, le brome ou l'iode, ou

ii) dans une première étape, à acyler une benzylamine (I.1) par un agent d'acylation de formule (R6-CO)n Z2 dans laquelle R6 est l'homologue immédiatement inférieur de R3 (R3=-CH2R6), et Z2 est soit hydroxyle, soit le brome ou le chlore lorsque n est 1 et est l'oxygène quand n est 2 pour obtenir un carboxamide intermédiaire de formule

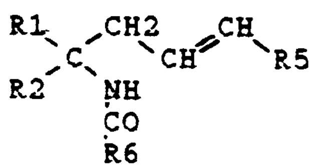

(II.2.1)

que l'on réduit par un hydrure métallique dans une seconde étape

- pour obtenir une benzylamine de formule (I.3) dans laquelle à la fois R3 et R4 sont méthyle, à diméthyler une benzylamine (I.1) en la faisant réagir sur du formaldéhyde et de l'acide formique selon la réaction de Eschweiler-Clarke ou sur du formaldéhyde en présence d'un agent réducteur,

- pour obtenir une benzylamine de formule (I.3), répondant à la formule (I) dans laquelle R3 n'est pas l'hydrogène et R4 n'est ni hydrogène ni méthyle,

i) dans une première étape, à acyler une benzylamine de formula (I.2) dans laquelle R3 est autre que l'hydrogène par un agent d'acylation de formula R7CO Z5 dans laquelle R7 est l'homologue immédiatement inférieur de R4 (R4 = -CH2=R7) et Z5 est le brome ou le chlore pour obtenir un carboxamide disubstitué sur l'azote de formule

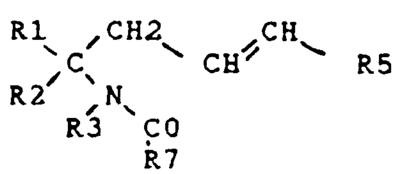

(II.2)

que l'on réduit, dans une seconde étape, par un hydrure métallique.

ii) ou a faire réagir un réactif organomagnésien de formule R2MgZ3.

Z3 étant un halogène, sur un nitrile de formule

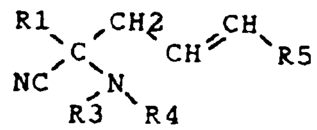

(II.3)

- pour obtenir une benzylamine de formule (I.3) répondant à la formule (I) dans laquelle R3 n'est pas l'hydrogène et R4 est méthyle, à N-méthyler une benzylamine de formule (I.2) dans laquelle R3 est alkyle ou alkényle inférieur par de l'aldéhyde formique en présence d'un réducteur tel qu'un hydrure métallique ou organo métallique;
- pour obtenir un sel d'addition avec un acide à salifier la benzylamine par un acide et pour obtenir une forme optiquement active à dédoubler un racémique.

**8.** Procédé de préparation d'un médicament, notamment psychotrope, caractérisé en ce qu'il consiste à mélanger une benzylamine ou l'un de ses sels suivant l'une des revendications 1 à 5 à un excipient pharmaceutique.

**9.** Procédé de préparation d'un médicament psychotrope et actif sur le tractus urinaire, caractérisé en ce qu'il consiste à mélanger une benzylamine ou un de ses sels suivant la revendication 6 à un excipient pharmaceutique.

**10.** L'utilisation d'une benzylamine telle que définie aux revendications 1 à 5 ou de l'un de ses sels acceptables thérapeutiquement pour l'obtention d'un médicament psychotrope.

**11.** L'utilisation d'une benzylamine telle que définie à la revendication 6 ou de l'un de ses sels acceptable thérapeutiquement pour l'obtention d'un médicament psychotrope et actif sur le tractus urinaire.

**12.** Composés intermédiaires de formule :

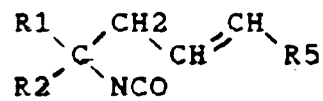

(II.1)

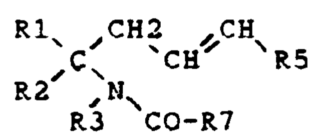

(II.2)

ou

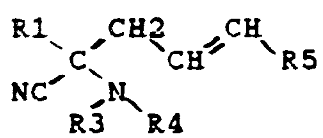

(II.3)

dans lesquelles R1 à R5 ont les significations indiquées à la revendication 1, R3 et R4 n'étant pas l'hydrogène dans le cas des nitriles (II.3), R7 étant un homologue immédiatement inférieur à R4.

**13.** Procédé de préparation des composés intermédiaires de la revendication 12, caractérisé en ce qu'il consiste

- pour préparer les composés (II.1), à faire réagir un composé de départ de formule (VIII) R1-CH2-W dans laquelle R1 est tel que défini pour les benzylamines (I) et W est un radical nitrile (-CN) ou carboxy (-COOH) sur un halogènure d'alkyle de formule R2Z6, R2 étant tel que défini pour les composés (I) et Z6 étant un halogène, pour obtenir, quand W est carboxy un acide de formule (VI)

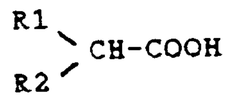

et, quand W est nitrile, un nitrile de formule (VII)

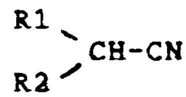

que l'on hydrolyse en l'acide de formule (VI), puis à alkyler l'acide (VI) par un halogènure d'alkènyle (V) de formule Z7-CH2-CH=CH-R5, Z7 étant un halogène et R5 étant tel que défini pour les benzylamines (I) pour obtenir un acide à chaîne insaturée (III) que l'on transpose en un isocyanate (II.1) par une réaction de Curtius.

- pour préparer les composés (II.2),
  i) à acyler une benzylamine de formule

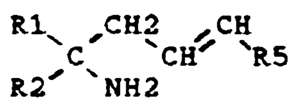 (I.1)

  par un réactif de formule (R6CO)nZ2 dans lequel R6 est l'homologue carboné directement inférieur à R3 (R3 = CH2-R6) et dans lequel Z2 est halogène ou hydroxyle quand n = 1 et Z2 est l'olygène quand n = 2, pour obtenir un carboxamide intermédiaire (II.2.1)
  ii) à acyler une benzylamine de formule

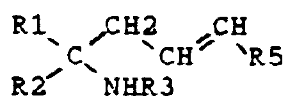

  par un agent d'acylation de formule R7CO Z5, dans laquelle R7 est alkyle inférieur ou alkényle inférieur et Z5 est le brome ou le chlore pour obtenir un carboxamide intermédiaire (II.2.2).
- pour préparer les composés (II.3) à faire réagir un aldéhyde de formule (XI) R1-CHO avec une amine de formule R3-NH-R4 et un cyanure de métal alcalin pour obtenir un aminonitrile de formule

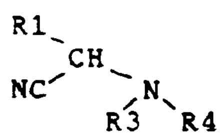 (X)

R4 et R3 étant tels que définis pour les benzylamines (I), mais n'étant pas l'hydrogène, puis, à alkyler l'aminonitrile (X) par un halogénure d'alkényle (V).

## Patentansprüche

### Patentansprüche für folgende Vertragsstaaten : AT, BE, CH, DE, FR, GB, IT, LI, LU, NL, SE

1. Disubstituierte Benzylamine der nachstehenden Formel (I)

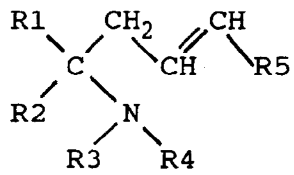

(I)

wobei

. R1 steht für einen Phenylrest, der gegebenenfalls mono- oder disubstituiert ist mit Halogenatomen, mit niederen Alkylresten, mit niederen halogenierten Alkylresten oder mit niederen Alkoxyresten;

. R2 steht für einen niederen Alkylrest;

. R3 und R4, die gleich oder verschieden sein können, stehen für Wasserstoff, für einen niederen Alkylrest oder für einen niederen Alkenylrest;

. R5 steht für einen Phenylrest, der gegebenenfalls mono-, di- oder trisubstituiert ist mit Halogenatomen oder mit niederen Alkoxyresten, sowie für deren Additionssalze mit Säuren, sowie für deren optisch aktive Formen.

**2.** Benzylamine nach Anspruch 1,
dadurch gekennzeichnet, daß
der Rest R1 ein Phenylrest ist.

**3.** Benzylamine nach Anspruch 1 oder 2,
dadurch gekennzeichnet, daß
der Rest R5 ein Phenylrest ist.

**4.** Benzylamine nach einem der Ansprüche 1 bis 3,
dadurch gekennzeichnet, daß
der Rest R2 ein Ethylrest ist.

**5.** Benzylamine nach einem der Ansprüche 1 bis 4,
dadurch gekennzeichnet, daß
der Rest R3 für Wasserstoff oder Methyl steht, und der Rest R4 für Wasserstoff oder Methyl steht.

**6.** Benzylamine nach Anspruch 1,
dadurch gekennzeichnet, daß
mindestens einer der Reste R1 und R5 für einen substituierten Phenylrest steht,
die Reste R3 und R4 gleich oder verschieden sind und für Wasserstoff oder für niedere Alkylreste stehen,
wobei die beiden Reste R3 und R4 nicht gleichzeitig Wasserstoff sind.

**7.** Verfahren zur Herstellung von Benzylaminen entsprechend nachstehender Formel (I)

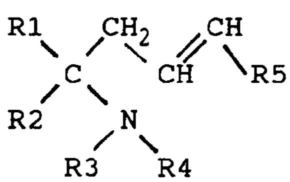

(I)

gekennzeichnet durch nachstehende alternative Verfahrensschritte:

. zur Erzeugung eines Benzylamin der Formel (I.1), welche der Formel (I) entspricht, wobei die Reste R3 und R4 je für Wasserstoff stehen, wird ein Isocyanat der nachstehenden Formel (II.1) hydrolisiert;

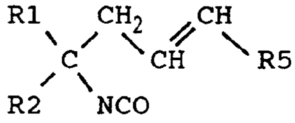

(II.1)

. zur Erzeugung eines Benzylamin der Formel (I.2), welche der Formel (I) entspricht,

wobei R3 für Methyl und R4 für Wasserstoff steht,

i) wird ein Isocyanat der Formel (II.1) mit einem Metallhydrid reduziert, oder

ii) wird ein Benzylamin der Formel (I.1) mit Ameisensäure in Gegenwart von Carbonyldiimidazol acyliert und nachstehendes Zwischenprodukt gebildet;

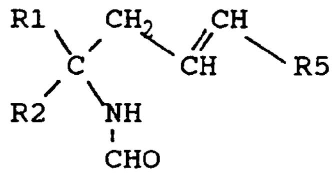

daraufhin wird dieses Zwischenprodukt mit einem Metallhydrid reduziert;

. zur Erzeugung eines Benzylamin der Formel (I.2), welche der Formel (I) entspricht, wobei R3 nicht Wasserstoff ist und R4 für Wasserstoff steht;

i) wird ein Benzylamin der Formel (I.1) mit einem Halogenid der Formel Z1R3 alkyliert,, wobei R3 nicht Wasserstoff ist und Z1 für Chlor, Brom oder Jod steht, oder

ii) wird in einer ersten Stufe ein Benzylamin der Formel (I.1) mit einem Acylierungsmittel der Formel $(R6\text{-}CO)_nZ2$ acyliert,

wobei R6 für das unmittelbar kleinere Homologe zu R3 steht (dh: R3 = $-CH_2R6$) und Z2 für Hydroxyl, für Brom oder für Chlor steht, wenn n den Wert "1" hat, oder Z2 für Sauerstoff steht, wenn n den Wert "2" hat,

um eine Carboxamid-Zwischenstufe der nachstehenden Formel (II.2.1) zu erhalten:

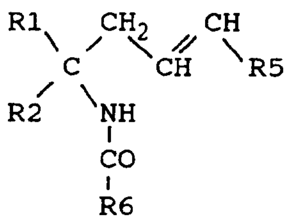

(II.2.1)

und in einer zweiten Stufe diese Carboxamid-Zwischenstufe (II.1.2) reduziert wird;

. zur Erzeugung eines Benzylamin der Formel (I.3), welche der Formel (I) entspricht, wobei beide Reste R3 und R4 für Methyl stehen,

wird ein Benzylamin der Formel (I.1) dimethyliert, nämlich mit Formaldehyd und Ameisensäure umgesetzt entsprechend der Reaktion von Eschweiler-Clarke, oder mit Formaldehyd in Gegenwart eines Reduktionsmittels umgesetzt;

. zur Erzeugung eines Benzylamin der Formel (I.3), welche der Formel (I) entspricht, wobei R3 nicht Wasserstoff ist, und wobei R4 weder Wasserstoff noch Methyl ist,

i) wird in einer ersten Stufe ein Benzylamin der Formel (I.2), wobei R3 nicht Wasserstoff ist, mit einem Acylierungsmittel der Formel R7COZ5 acyliert,

wobei R7 für das unmittelbar kleinere Homologe zu R4 steht (d.h.: R4 = $-CH_2R7$) und Z5 für Brom oder Chlor steht,

um ein am Stickstoff disubstituiertes Carboxamid nachstehender Formel (II.2) zu erhalten

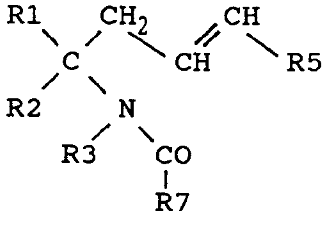

(II.2)

und in einer zweiten Stufe wird dieses Carboxamid (II.2) mit einem Metallhydrid reduziert;

ii) wird ein Nitril der nachstehenden Formel (II.3)

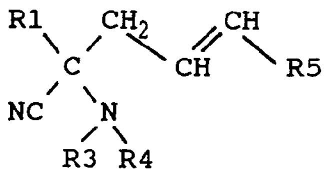

(II.3)

mit einer reaktionsfähigen organischen Magnesiumverbindung der Formel R2MgZ3 umgesetzt, wobei Z3 für ein Halogen steht;

. zur Erzeugung eines Benzylamin der Formel (I.3), welche der Formel (I) entspricht, wobei R3 nicht Wasserstoff ist und R4 für Methyl steht,
wird ein Benzylamin der Formel (I.2), wobei R3 für einen niederen Alkylrest oder einen niederen Alkenylrest steht, mit Formaldehyd in Gegenwart eines Reduktionsmittels, etwa Metallhydrid oder eine metallorganische Verbindung, umgesetzt, um eine Methylierung am Stickstoff zu erreichen.

**8.** Arzneimittel, insbesondere mit psychotroper Wirkung
dadurch gekennzeichnet, daß
das Arzneimittel als Wirkstoff ein Benzylamin nach einem der Ansprüche 1 bis 5 enthält.

**9.** Arzneimittel mit psychotroper Wirkung und mit einer Wirkung auf den Harntrakt,
dadurch gekennzeichnet, daß
das Arzneimittel als Wirkstoff ein Benzylamin nach Anspruch 6 enthält.

**10.** Zwischenprodukte entsprechend nachstehender Formel:

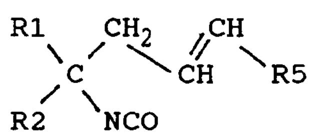

(II.1)

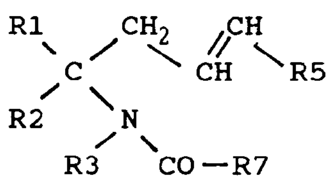

(II.2)

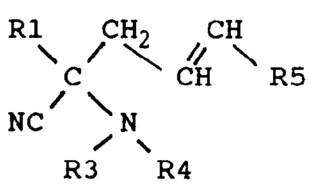

(II.3)

wobei die Reste R1 bis R5 die in Anspruch 1 angegebene Bedeutung haben, im Falle der Nitrile (II.3) die Reste R3 und R4 nicht Wasserstoff sind, und der Rest R7 ein unmittelbar kleineres Homologes zum Rest R4 ist.

**11.** Verfahren zur Herstellung der Zwischenprodukte nach Anspruch 9,
gekennzeichnet durch nachstehende alternative Verfahrensschritte:

. zur Erzeugung der Zwischenprodukte (II.1) wird eine Verbindung der Formel (VIII) R1-$CH_2$-W, wobei R1 die im Benzylamin (I) entsprechende Bedeutung hat und W für einen Nitrilrest (-CN) oder für einen Carboxyrest (-COOH) steht,
mit einem Alkylhalogenid der Formel R2Z6 umgesetzt, wobei R2 die im Benzylamin (I) entsprechende Bedeutung hat und Z6 für ein Halogen steht,

wobei im Falle, daß W für den Carboxyrest steht, eine Säure der nachstehenden Formel (VI)

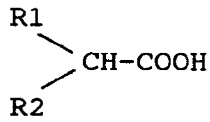

(VI)

erhalten wird, und
wobei im Falle, daß W für den Nitrilrest steht, ein Nitril der nachstehenden Formel (VII)

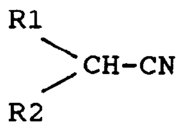

(VII)

erhalten wird,
das zur Säure (VI) hydrolysiert wird,
daraufhin die Säure (VI) mit einem Alkenylhalogenid der Formel (V) Z7-$CH_2$-CH=CH-R5 alkyliert wird, wobei Z7 für ein Halogen steht und R5 die im Benzylamin (I) entsprechende Bedeutung hat, um eine Säure mit einer ungesättigten Kette (III) zu erhalten, die daraufhin mit Hilfe der Curtius-Reaktion in das Isocyanat (II.1) übergeführt wird;
. zur Erzeugung der Zwischenprodukte (II.2)
i) wird ein Benzylamin der nachstehenden Formel (I.1)

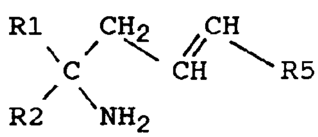

(I.1)

mit einem Reagens der Formel $(R6CO)_nZ2$ acyliert,
wobei der Rest R6 das unmittelbar kleinere C-Homologe zum Rest R3 ist (d.h.: R3 = -$CH_2$-R6) und Z2 für Halogen oder Hydroxyl steht, sofern n den Wert "1" hat, und Z2 für Sauerstoff steht, wenn n den Wert "2" hat,
um eine Carboxamid-Zwischenstufe (II.2.1) zu erhalten,
ii) wird ein Benzylamin der nachstehenden Formel

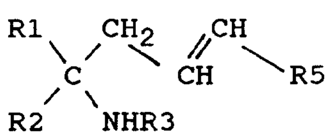

mit einem Acylierungsmittel der Formel R7COZ5 acyliert,
wobei R7 für einen niederen Alkylrest oder für einen niederen Alkenylrest steht und Z5 Brom oder Chlor ist, um eine Carboxamid-Zwischenstufe (II.2.2) zu erhalten;
. zur Erzeugung der Zwischenprodukte (II.3) wird ein Aldehyd der Formel (XI) R1-CHO mit einem Amin der Formel R3-NH-R4 und mit einem Alkalimetallcyanid umgesetzt, um ein Aminonitril der nachstehenden Formel (X)

(X)

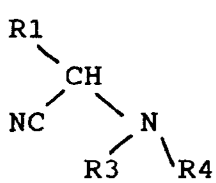

zu erhalten,
wobei R3 und R4 die im Benzylamin (I) entsprechende Bedeutung haben, jedoch nicht Wasserstoff sind,
und daraufhin wird das Aminonitril (X) mit einem Alkenylhalogenid (V) alkyliert.

**Patentansprüche für folgenden Vertragsstaat : ES**

1. Verfahren zur Herstellung von disubstituierten Benzylaminen der nachstehenden Formel (I)

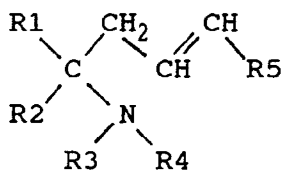

(I)

wobei

. R1 steht für einen Phenylrest, der gegebenenfalls mono- oder disubstituiert ist mit Halogenatomen, mit niederen Alkylresten, mit niederen halogenierten Alkylresten oder mit niederen Alkoxyresten;
. R2 steht für einen niederen Alkylrest;
. R3 und R4, die gleich oder verschieden sein können, stehen für Wasserstoff, für einen niederen Alkylrest oder für einen niederen Alkenylrest;
. R5 steht für einen Phenylrest, der gegebenenfalls mono-, di- oder trisubstituiert ist mit Halogenatomen oder mit niederen Alkoxyresten, sowie für deren Additionssalze mit Säuren, sowie für deren optisch aktive Formen,

gekennzeichnet durch nachstehende alternative Verfahrensschritte:

. zur Erzeugung eines Benzylamin der Formel (I.1), welche der Formel (I) entspricht, wobei die Reste R3 und R4 je für Wasserstoff stehen, wird ein Isocyanat der nachstehenden Formel (II.1) hydrolisiert;

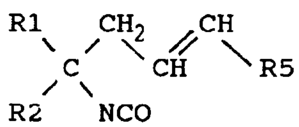

(II.1)

. zur Erzeugung eines Benzylamin der Formel (I.2), welche der Formel (I) entspricht,
wobei R3 für Methyl und R4 für Wasserstoff steht,

i) wird ein Isocyanat der Formel (II.1) mit einem Metallhydrid reduziert, oder
ii) wird ein Benzylamin der Formel (I.1) mit Ameisensäure in Gegenwart von Carbonyldiimidazol acyliert und nachstehendes Zwischenprodukt gebildet;

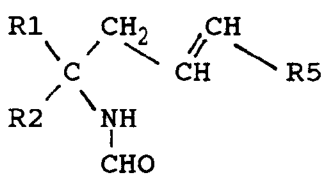

daraufhin wird dieses Zwischenprodukt mit einem Metallhydrid reduziert;

. zur Erzeugung eines Benzylamin der Formel (I.2), welche der Formel (I) entspricht, wobei R3 nicht Wasserstoff ist und R4 für Wasserstoff steht;

i) wird ein Benzylamin der Formel (I.1) mit einem Halogenid der Formel Z1R3 alkyliert,
wobei R3 nicht Wasserstoff ist und Z1 für Chlor, Brom oder Jod steht, oder
ii) wird in einer ersten Stufe ein Benzylamin der Formel (I.1) mit einem Acylierungsmittel der Formel $(R6\text{-}CO)_nZ2$ acyliert,
wobei R6 für das unmittelbar kleinere Homologe zu R3 steht (dh: $R3 = -CH_2R6$) und Z2 für Hydroxyl, für Brom oder für Chlor steht, wenn n den Wert "1" hat, oder Z2 für Sauerstoff steht, wenn n den Wert "2" hat,

um eine Carboxamid-Zwischenstufe der nachstehenden Formel (II.2.1) zu erhalten:

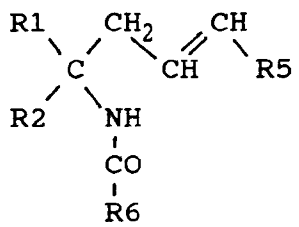

(II.2.1)

und in einer zweiten Stufe diese Carboxamid-Zwischenstufe (II.1.2) mit einem Metallhydrid reduziert wird;

. zur Erzeugung eines Benzylamin der Formel (I.3), welche der Formel (I) entspricht, wobei beide Reste R3 und R4 für Methyl stehen,
wird ein Benzylamin der Formel (I.1) dimethyliert, nämlich mit Formaldehyd und Ameisensäure umgesetzt entsprechend der Reaktion von Eschweiler-Clarke, oder mit Formaldehyd in Gegenwart eines Reduktionsmittels umgesetzt;

. zur Erzeugung eines Benzylamin der Formel (I.3), welche der Formel (I) entspricht, wobei R3 nicht Wasserstoff ist, und wobei R4 weder Wasserstoff noch Methyl ist,

i) wird in einer ersten Stufe ein Benzylamin der Formel (I.2), wobei R3 nicht Wasserstoff ist, mit einem Acylierungsmittel der Formel R7COZ5 acyliert,
wobei R7 für das unmittelbar kleinere Homologe zu R4 steht (d.h.: R4 = $-CH_2R7$) und Z5 für Brom oder Chlor steht,
um ein am Stickstoff disubstituiertes Carboxamid nachstehender Formel (II.2) zu erhalten

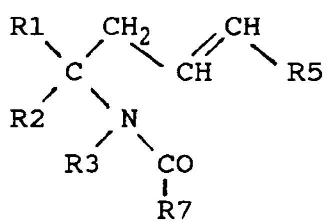

(II.2)

und in einer zweiten Stufe wird dieses Carboxamid (II.2) mit einem Metallhydrid reduziert;

ii) wird ein Nitril der nachstehenden Formel (II.3)

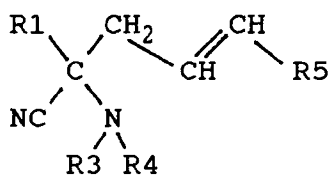

(II.3)

mit einer reaktionsfähigen organischen Magnesiumverbindung der Formel R2MgZ3 umgesetzt, wobei Z3 für ein Halogen steht;

. zur Erzeugung eines Benzylamin der Formel (I.3), welche der Formel (I) entspricht, wobei R3 nicht Wasserstoff ist und R4 für Methyl steht,
wird ein Benzylamin der Formel (I.2), wobei R3 für einen niederen Alkylrest oder einen niederen Alkenylrest steht, mit Formaldehyd in Gegenwart eines Reduktionsmittels, etwa Metallhydrid oder eine metallorganische Verbindung, umgesetzt, um eine Methylierung am Stickstoff zu erreichen;

. zur Erzeugung eines Additionssalzes mit einer Säure wird ein Benzylaminsalz mit dieser Säure gebildet,und eine optisch aktive Form durch die Spaltung eines Racemates erhalten.

**2.** Verfahren nach Anspruch 1,
dadurch gekennzeichnet, daß
der Rest R1 ein Phenylrest ist.

3. Verfahren nach Anspruch 1 oder 2, dadurch gekennzeichnet, daß der Rest R5 ein Phenylrest ist.

4. Verfahren nach einem Ansprüche 1 bis 3, dadurch gekennzeichnet, daß der Rest R2 ein Ethylrest ist.

5. Verfahren nach einem der Ansprüche 1 bis 4, dadurch gekennzeichnet, daß der Rest R3 für Wasserstoff oder Methyl steht, und der Rest R4 für Wasserstoff oder Methyl steht.

6. Verfahren nach Anspruch 1, dadurch gekennzeichnet, daß mindestens einer der Reste R1 und R5 für einen substituierten Phenylrest steht, die Reste R3 und R4 gleich oder verschieden sind und für Wasserstoff oder für niedere Alkylreste stehen, wobei die beiden Reste R3 und R4 nicht gleichzeitig Wasserstoff sind.

7. Verfahren zur Herstellung eines Arzneimittels mit psychotroper Wirkung dadurch gekennzeichnet, daß ein Benzylamin, das nach einem der Ansprüche 1 bis 5 erhältlich ist, oder eines seiner Salze, mit einem pharmazeutischen Träger vermischt wird.

8. Verfahren zur Herstellung eines Arzneimittels mit psychotroper Wirkung und mit einer Wirkung auf den Harntrakt, dadurch gekennzeichnet, daß ein Benzylamin, das nach Anspruch 6 erhältlich ist, oder eines seiner Salze, mit einem pharmazeutischen Träger vermischt wird.

9. Verfahren zur Herstellung von Zwischenprodukten nachstehender Formel:

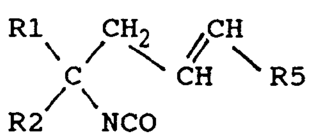

(II.1)

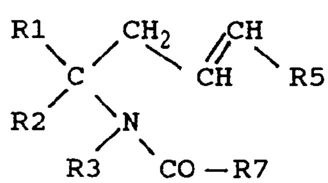

(II.2)

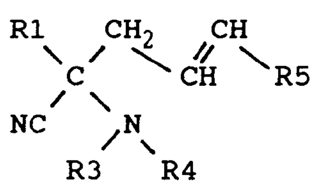

(II.3)

wobei die Reste R1 bis R5 die in Anspruch 1 angegebene Bedeutung haben, im Falle der Nitrile (II.3) die Reste R3 und R4 nicht Wasserstoff sind,

gekennzeichnet durch nachstehende alternative Verfahrensschritte:

. zur Erzeugung der Zwischenprodukte (II.1) wird eine Verbindung der Formel (VIII) R1-$CH_2$-W, wobei R1 die im Benzylamin (I) entsprechende Bedeutung hat und W für einen Nitrilrest (-CN) oder für einen Carboxyrest (-COOH) steht, mit einem Alkylhalogenid der Formel R2Z6 umgesetzt, wobei R2 die im Benzylamin (I) entsprechende Bedeutung hat und Z6 für ein Halogen steht, wobei im Falle, daß W für den Carboxyrest steht, eine Säure der nachstehenden Formel (VI)

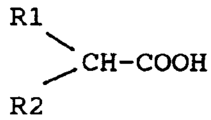

(VI)

erhalten wird, und
wobei im Falle, daß W für den Nitrilrest steht, ein Nitril der nachstehenden Formel (VII)

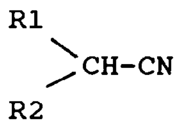

(VII)

erhalten wird,
das zur Säure (VI) hydrolysiert wird,
daraufhin die Säure (VI) mit einem Alkenylhalogenid der Formel (V) Z7-$CH_2$-CH=CH-R5 alkyliert wird, wobei Z7 für ein Halogen steht und R5 die im Benzylamin (I) entsprechende Bedeutung hat, um eine Säure mit einer ungesättigten Kette (III) zu erhalten, die daraufhin mit Hilfe der Curtius-Reaktion in das Isocyanat (II.1) übergeführt wird;

. zur Erzeugung der Zwischenprodukte (II.2)

i) wird ein Benzylamin der nachstehenden Formel (I.1)

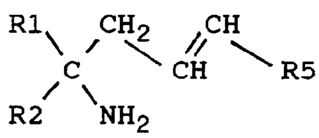

(I.1)

mit einem Reagens der Formel $(R6CO)_nZ2$ acyliert,
wobei der Rest R6 das unmittelbar kleinere C-Homologe zum Rest R3 ist (d.h.: R3 = -$CH_2$-R6) und Z2 für Halogen oder Hydroxyl steht, sofern n den Wert "1" hat, und Z2 für Sauerstoff steht, wenn n den Wert "2" hat,
um eine Carboxamid-Zwischenstufe (II.2.1) zu erhalten;

ii) wird ein Benzylamin der nachstehenden Formel

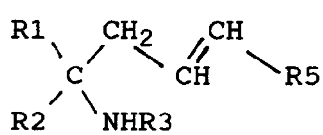

mit einem Acylierungsmittel der Formel R7COZ5 acyliert,
wobei R7 für einen niederen Alkylrest oder für einen niederen Alkenylrest steht, und Z5 Brom oder Chlor ist, um eine Carboxamid-Zwischenstufe (II.2) zu erhalten;

. zur Erzeugung der Zwischenprodukte (II.3) wird ein Aldehyd der Formel (XI) R1-CHO, mit einem Amin der Formel R3-NH-R4 und mit einem Alkalimetallcyanid umgesetzt, um ein Aminonitril der nachstehenden Formel (X)

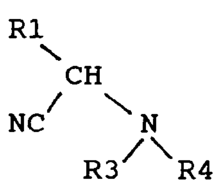

(X)

zu erhalten,

wobei R3 und R4 die im Benzylamin (I) entsprechende Bedeutung haben, jedoch nicht Wasserstoff sind,
und daraufhin wird das Aminonitril (X) mit einem Alkenylhalogenid (V) alkyliert.

**10.** Verwendung eines Benzylamin, das nach einem der Ansprüche 1 bis 5 erhältlich ist, oder eines seiner therapeutisch verträglichen Salze zur Erzeugung eines Arzneimittels mit psychotroper Wirkung.

**11.** Verwendung eines Benzylamin, das nach Anspruch 6 erhältlich ist, oder eines seiner therapeutisch verträglichen Salze zur Erzeugung eines Arzneimittels mit psychotroper Wirkung und mit einer Wirkung auf den Harntrakt.

**12.** Verwendung eines Zwischenproduktes, das nach Anspruch 9 erhältlich ist, als Zwischenprodukt zur Benzylaminsynthese.

**Patentansprüche für folgenden Vertragsstaat : GR**

**1.** Disubstituierte Benzylamine der nachstehenden Formel (I)

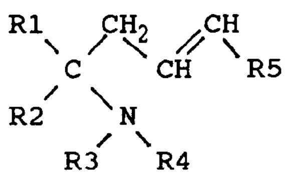

(I)

wobei
. R1 steht für einen Phenylrest, der gegebenenfalls mono- oder disubstituiert ist mit Halogenatomen, mit niederen Alkylresten, mit niederen halogenierten Alkylresten oder mit niederen Alkoxyresten;
. R2 steht für einen niederen Alkylrest;
. R3 und R4, die gleich oder verschieden sein können, stehen für Wasserstoff, für einen niederen Alkylrest oder für einen niederen Alkenylrest;
. R5 steht für einen Phenylrest, der gegebenenfalls mono-, di- oder trisubstituiert ist mit Halogenatomen oder mit niederen Alkoxyresten, sowie für deren Additionssalze mit Säuren, sowie für deren optisch aktive Formen.

**2.** Benzylamine nach Anspruch 1,
dadurch gekennzeichnet, daß
der Rest R1 ein Phenylrest ist.

**3.** Benzylamine nach Anspruch 1 oder 2,
dadurch gekennzeichnet, daß
der Rest R5 ein Phenylrest ist.

**4.** Benzylamine nach einem der Ansprüche 1 bis 3,
dadurch gekennzeichnet, daß
der Rest R2 ein Ethylrest ist.

**5.** Benzylamine nach einem der Ansprüche 1 bis 4,
dadurch gekennzeichnet, daß
der Rest R3 für Wasserstoff oder Methyl steht, und der Rest R4 für Wasserstoff oder Methyl steht.

**6.** Benzylamine nach Anspruch 1,
dadurch gekennzeichnet, daß
mindestens einer der Reste R1 und R5 für einen substituierten Phenylrest steht,
die Reste R3 und R4 gleich oder verschieden sind und für Wasserstoff oder für niedere Alkylreste stehen,
wobei die beiden Reste R3 und R4 nicht gleichzeitig Wasserstoff sind.

**7.** Verfahren zur Herstellung von Benzylaminen entsprechend nachstehender Formel (I)

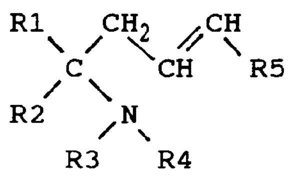

(I)

<u>gekennzeichnet durch</u> nachstehende alternative Verfahrensschritte:

. zur Erzeugung eines Benzylamin der Formel (I.1), welche der Formel (I) entspricht, wobei die Reste R3 und R4 je für Wasserstoff stehen, wird ein Isocyanat der nachstehenden Formel (II.1) hydrolisiert;

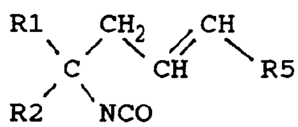

(II.1)

. zur Erzeugung eines Benzylamin der Formel (I.2), welche der Formel (I) entspricht, wobei R3 für Methyl und R4 für Wasserstoff steht,

i) wird ein Isocyanat der Formel (II.1) mit einem Metallhydrid reduziert, oder

ii) wird ein Benzylamin der Formel (I.1) mit Ameisensäure in Gegenwart von Carbonyldiimidazol acyliert und nachstehendes Zwischenprodukt gebildet;

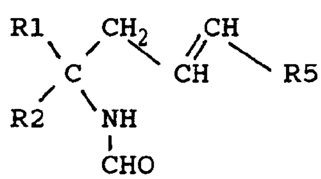

daraufhin wird dieses Zwischenprodukt mit einem Metallhydrid reduziert;

. zur Erzeugung eines Benzylamin der Formel (I.2), welche der Formel (I) entspricht, wobei R3 nicht Wasserstoff ist und R4 für Wasserstoff steht;

i) wird ein Benzylamin der Formel (I.1) mit einem Halogenid der Formel Z1R3 alkyliert, wobei R3 nicht Wasserstoff ist und Z1 für Chlor, Brom oder Jod steht, oder

ii) wird in einer ersten Stufe ein Benzylamin der Formel (I.1) mit einem Acylierungsmittel der Formel $(R6\text{-}CO)_nZ2$ acyliert,

wobei R6 für das unmittelbar kleinere Homologe zu R3 steht (dh: R3 = $-CH_2R6$) und Z2 für Hydroxyl, für Brom oder für Chlor steht, wenn n den Wert "1" hat, oder Z2 für Sauerstoff steht, wenn n den Wert "2" hat,

um eine Carboxamid-Zwischenstufe der nachstehenden Formel (II.2.1) zu erhalten:

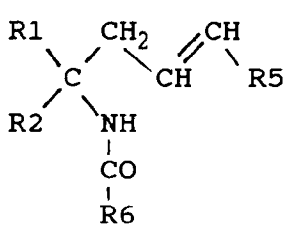

(II.2.1)

und in einer zweiten Stufe diese Carboxamid-Zwischenstufe (II.1.2) mit einem Methallhydrid reduziert wird;

. zur Erzeugung eines Benzylamin der Formel (I.3), welche der Formel (I) entspricht, wobei beide Reste R3 und R4 für Methyl stehen,

wird ein Benzylamin der Formel (I.1) dimethyliert, nämlich mit Formaldehyd und Ameisensäure umgesetzt entsprechend der Reaktion von Eschweiler-Clarke, oder mit Formaldehyd in Gegenwart eines Reduktionsmittels umgesetzt;

. zur Erzeugung eines Benzylamin der Formel (I.3), welche der Formel (I) entspricht, wobei R3 nicht Wasserstoff ist, und wobei R4 weder Wasserstoff noch Methyl ist,

i) wird in einer ersten Stufe ein Benzylamin der Formel (I.2), wobei R3 nicht Wasserstoff ist, mit einem Acylierungsmittel der Formel R7COZ5 acyliert,

wobei R7 für das unmittelbar kleinere Homologe zu R4 steht (d.h.: R4 = $-CH_2R7$) und Z5 für Brom oder Chlor steht,

um ein am Stickstoff disubstituiertes Carboxamid nachstehender Formel (II.2) zu erhalten

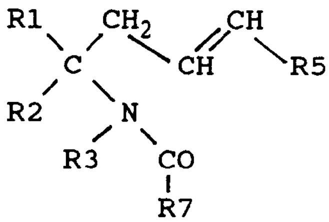

(II.2)

und in einer zweiten Stufe wird dieses Carboxamid (II.2) mit einem Metallhydrid reduziert;

ii) wird ein Nitril der nachstehenden Formel (II.3)

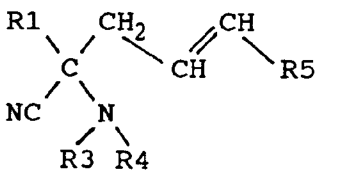

(II.3)

mit einer reaktionsfähigen organischen Magnesiumverbindung der Formel R2MgZ3 umgesetzt, wobei Z3 für ein Halogen steht;

. zur Erzeugung eines Benzylamin der Formel (I.3), welche der Formel (I) entspricht, wobei R3 nicht Wasserstoff ist und R4 für Methyl steht,

wird ein Benzylamin der Formel (I.2), wobei R3 für einen niederen Alkylrest oder einen niederen Alkenylrest steht, mit Formaldehyd in Gegenwart eines Reduktionsmittels, etwa Metallhydrid oder eine metallorganische Verbindung, umgesetzt, um eine Methylierung am Stickstoff zu erreichen;

. zur Erzeugung eines Additionssalzes mit einer Säure wird ein Benzylaminsalz mit dieser Säure gebildet, und eine optisch aktive Form durch die Spaltung eines Racemates erhalten.

**8.** Verfahren zur Herstellung eines Arzneimittels mit psychotroper Wirkung
dadurch gekennzeichnet, daß
ein Benzylamin, das nach einem der Ansprüche 1 bis 5 erhältlich ist, oder eines seiner Salze, mit einem pharmazeutischen Träger vermischt wird.

**9.** Verfahren zur Herstellung eines Arzneimittels mit psychotroper Wirkung und mit einer Wirkung auf den Harntrakt,
dadurch gekennzeichnet, daß
ein Benzylamin, das nach Anspruch 6 erhältlich ist, oder eines seiner Salze, mit einem pharmazeutischen Träger vermischt wird.

**10.** Verwendung eines Benzylamin, das nach einem der Ansprüche 1 bis 5 erhältlich ist, oder eines seiner therapeutisch verträglichen Salze zur Erzeugung eines Arzneimittels mit psychotroper Wirkung.

**11.** Verwendung eines Benzylamin, das nach Anspruch 6 erhältlich ist, oder eines seiner therapeutisch verträglichen Salze zur Erzeugung eines Arzneimittels mit psychotroper Wirkung und mit einer Wirkung auf den Harntrakt.

**12.** Zwischenprodukte entsprechend nachstehender Formel:

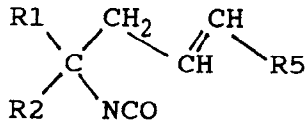

(II.1)

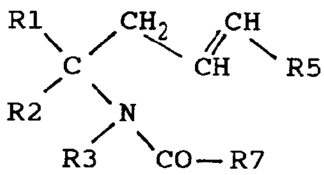

(II.2)

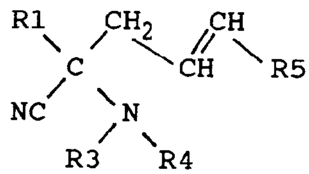

(II.3)

wobei die Reste R1 bis R5 die in Anspruch 1 angegebene Bedeutung haben, im Falle der Nitrile (II.3) die Reste R3 und R4 nicht Wasserstoff sind, und der Rest R7 ein unmittelbar kleineres Homologes zum Rest R4 ist.

**13.** Verfahren zur Herstellung der Zwischenprodukte nach Anspruch 9,
gekennzeichnet durch nachstehende alternative Verfahrensschritte:

. zur Erzeugung der Zwischenprodukte (II.1) wird eine Verbindung der Formel (VIII) R1-$CH_2$-W, wobei R1 die im Benzylamin (I) entsprechende Bedeutung hat und W für einen Nitrilrest (-CN) oder für einen Carboxyrest (-COOH) steht,
mit einem Alkylhalogenid der Formel R2Z6 umgesetzt,
wobei R2 die im Benzylamin (I) entsprechende Bedeutung hat und Z6 für ein Halogen steht,
wobei im Falle, daß W für den Carboxyrest steht, eine Säure der nachstehenden Formel (VI)

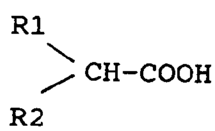

(VI)

erhalten wird, und
wobei im Falle, daß W für den Nitrilrest steht, ein Nitril der nachstehenden Formel (VII)

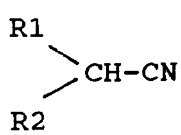

(VII)

erhalten wird,
das zur Säure (VI) hydrolysiert wird,
daraufhin die Säure (VI) mit einem Alkenylhalogenid der Formel (V) Z7-CH2-CH=CH-R5 alkyliert wird, wobei Z7 für ein Halogen steht und R5 die im Benzylamin (I) entsprechende Bedeutung hat, um eine Säure mit einer ungesättigten Kette (III) zu erhalten, die daraufhin mit Hilfe der Curtius-Reaktion in das Isocyanat (II.1) übergeführt wird;

. zur Erzeugung der Zwischenprodukte (II.2)

i) wird ein Benzylamin der nachstehenden Formel (I.1)

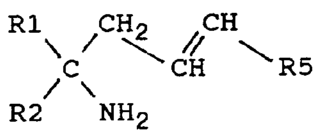

(I.1)

mit einem Reagens der Formel $(R6CO)_nZ2$ acyliert, wobei der Rest R6 das unmittelbar kleinere C-Homologe zum Rest R3 ist (d.h.: R3 = $-CH_2-R6$) und Z2 für Halogen oder Hydroxyl steht, sofern n den Wert "1" hat, und Z2 für Sauerstoff steht, wenn n den Wert "2" hat, um eine Carboxamid-Zwischenstufe (II.2.1) zu erhalten,

ii) wird ein Benzylamin der nachstehenden Formel

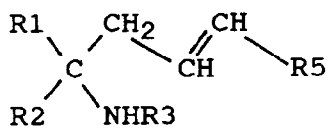

mit einem Acylierungsmittel der Formel R7COZ5 acyliert, wobei R7 für einen niederen Alkylrest oder für einen niederen Alkenylrest steht, und Z5 Brom oder Chlor ist, um eine Carboxamid-Zwischenstufe (II.2.2) zu erhalten;

. zur Erzeugung der Zwischenprodukte (II.3) wird ein Aldehyd der Formel (XI) R1-CHO, mit einem Amin der Formel R3-NH-R4 und mit einem Alkalimetallcyanid umgesetzt, um ein Aminonitril der nachstehenden Formel (X)

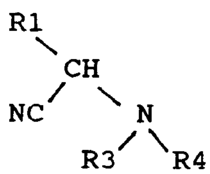

(X)

zu erhalten, wobei R3 und R4 die im Benzylamin (I) entsprechende Bedeutung haben, jedoch nicht Wasserstoff sind, und daraufhin wird das Aminonitril (X) mit einem Alkenylhalogenid (V) alkyliert.

## Claims

### Claims for the following Contracting States : AT, BE, CH, DE, FR, GB, IT, LI, LU, NL, SE

1. Disubstituted benzylamines corresponding to the formula

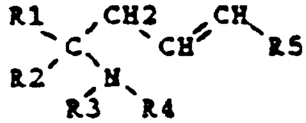

(I)

in which :
- R1 is a phenyl group, possibly mono- or disubstituted by halogen atoms or by lower alkyl, lower haloalkyl or lower alkoxy radicals,
- R2 is a lower alkyl radical,
- R3 and R4, which may be identical or different, are hydrogen, or lower alkyl or lower alkenyl radicals,
- R5 is a phenyl group, possibly mono-, di- or trisubstituted by halogen atoms or by lower alkoxy radicals,

their addition salts with acids and their optically active forms.

2. Benzylamines conforming to Claim 1 characterized in that R1 is a phenyl group.

3. Benzylamines conforming to Claims 1 or 2, characterized in that R5 is a phenyl group.

4. Benzylamines conforming to any of Claims 1 to 3, characterized in that R2 is an ethyl radical.

5. Benzylamines conforming to any of Claims 1 to 4, characterized in that R3 is hydrogen or a methyl radical and R4 is hydrogen or a methyl radical.

6. Benzylamine conforming to Claim 1, characterized in that at least one of R1 and R5 is a substituted phenyl group, and R3 and R4, whether identical or different, are hydrogen or a lower alkyl radical, but R3 and R4 are not both hydrogen.

7. Process for the preparation of benzylamines conforming to formula (I), characterized in that it consists of the following:
- to obtain a benzylamine of formula (I.1), corresponding to formula (I), in which R3 and R4 are hydrogen, hydrolysis of an isocyanate with formula:

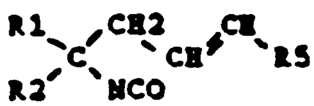

(II.1)

- to obtain a benzylamine of formula (I.2), corresponding to formula (I) in which R3 is a methyl radical and R4 is hydrogen,
i) reduction of an isocyanate of formula (II.1) using a metal hydride, or
ii) acylation of a benzylamine (I.1) with formic acid in the presence of carbonyl-diimidazole, to give the intermediate compound

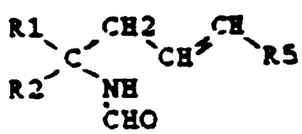

followed by reduction of this intermediate compound with a metal hydride,
- to obtain a benzylamine of formula (I.2), corresponding to formula (I) in which R3 is other than hydrogen and R4 is hydrogen
i) alkylation of a benzylamine of formula (I.1) using a halide of formula Z1R3 in which R3 is other than hydrogen and Z1 is chlorine, bromine or iodine, or
ii) in a first stage, acylation of a benzylamine (I.1) using an acylating agent of formula (R6-CO)nZ2, in which R6 is the immediately lower homologue of R3 (R3 = -CH2R6), and Z2 is either hydroxyl, or bromine or chlorine when n = 1, and is oxygen when n = 2, to obtain an intermediate carboxamide of formula

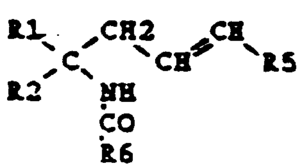

(II.2.1)

which is reduced by a metal hydride in a second stage,
- to obtain a benzylamine of formula (I.3) in which both R3 and R4 are methyl radicals, demethylation of a benzylamine (I.1) by reaction with formaldehyde and formic acid in accordance with the Eschweiler-Clarke reaction, or with formaldehyde in the presence of a reducing agent,
- to obtain a benzylamine of formula (I.3), corresponding to formula (I), in which R3 is not hydrogen and R4 is neither hydrogen nor a methyl radical,

i) in a first stage, acylation of a benzylamine of formula (I.2) in which R3 is other than hydrogen, using an acylating agent of formula R7COZ5 in which R7 is the immediately lower homologue of R4 (R4 = -CH2R7) and Z5 is bromine or chlorine, to obtain a carboxamide disubstituted on the nitrogen atom, of formula

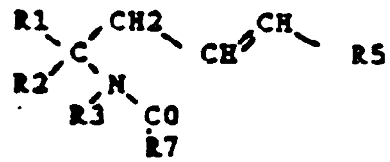

(II.2)

which is reduced in a second stage using a metal hydride,
ii) or reaction of an organomagnesium reagent of formula R2MgZ3, where Z5 is a halogen, with a nitrile of formula

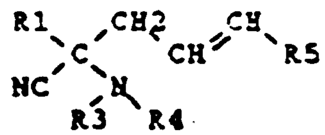

(II.3)

- to obtain a benzylamine of formula (I.3) corresponding to formula (I) in which R3 is not hydrogen and R4 is a methyl radical, N-methylation of a benzylamine of formula (I.2), in which R3 is a lower alkyl or alkenyl radical, using formic aldehyde in the presence of a reducing agent such as a metal or organometallic hydride.

**8.** Medicament, notably a psychotropic product, characterized in that it contains a benzylamine conforming to any of Claims 1 to 5.

**9.** Medicament that is psychotropic and active upon the urinary tract, characterized in that it contains a benzylamine conforming to Claim 6.

**10.** Intermediate compounds of the following formulae:

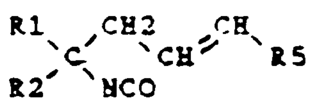

(II.1)

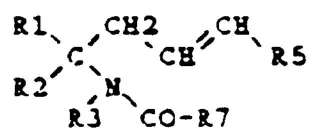

(II.2)

or

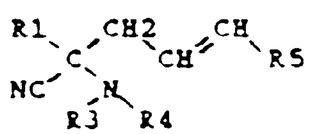

(II.3)

in which R1 to R5 are as indicated in Claim 1, R3 and R4 are other than hydrogen in the case of the nitriles (II.3), and R7 is the immediately lower homologue of R4.

**11.** Process for preparing the intermediate compounds of Claim 10, characterized in that it consists of the

following:

- to prepare the compounds (II.1), reaction of a starting compound of formula (VIII) R1-CH2-W in which R1 is as defined for the benzylamines (I) and W is a nitrile (-CN) or carboxy (-COOH) radical, with an alkyl halide of formula R2Z6, R2 being as defined for the compounds (I) and Z6 being a halogen, to obtain an acid of formula (VI) when W is a carboxy radical

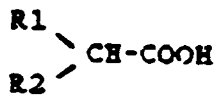

and, when W is a nitrile, a nitrile of formula (VII)

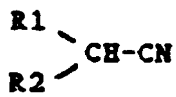

that is hydrolysed to the acid of formula VI, followed by alkylation of the acid (VI) by an alkenyl halide (V) of formula Z7-CH2-CH=CH-R5 in which Z7 is a halogen and R5 is as defined for the benzylamines (I), to obtain an unsaturated chain acid (III) that is transformed to an isocyanate (II.1) by a Curtius reaction,

- to prepare the compounds (II.2),

  i) acylation of a benzylamine with formula

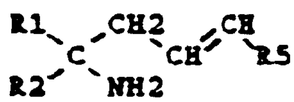

(I.1)

by means of a reagent with formula (R6CO)nZ2, in which R6 is the carbonated homologue directly below R3 (R3 = -CH2-R6) and in which Z2 is a halogen or hydroxyl when n = 1 and Z2 is oxygen when n = 2, to obtain an intermediate carboxamide (II.2.1),

  ii) acylation of a benzylamine with formula

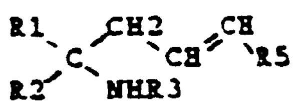

by an acylation agent with formula R7CO Z5, in which R7 is a lower alkyl or alkenyl radical and Z5 is bromine or chlorine, to obtain an intermediate carboxamide (II.2.2),

- to prepare the compounds (II.3), reaction of an aldehyde of formula (XI) R1-CHO with an amine of formula R3-NH-R4 and an alkali metal cyanide to give an aminonitrile with formula

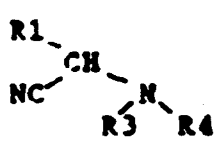

(X)

in which R4 and R3 are as defined for the benzylamines (I) but not hydrogen, and then alkylation of the aminonitrile (X) by an alkenyl halide (V).

**Claims for the following Contracting State : ES**

1. Process for the preparation of disubstituted benzylamines corresponding to the formula

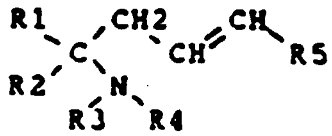

(I)

in which :

- R1 is a phenyl group, possibly mono- or disubstituted by halogen atoms or by lower alkyl, lower haloalkyl or lower alkoxy radicals,
- R2 is a lower alkyl radical,
- R3 and R4, which may be identical or different, are hydrogen, or lower alkyl or lower alkenyl radicals,
- R5 is a phenyl group, possibly mono-, di- or trisubstituted by halogen atoms or by lower alkoxy radicals,

their addition salts with acids and their optically active forms, characterized in that it consists of the following:

- to obtain a benzylamine of formula (I.1), corresponding to formula (I), in which R3 and R4 are hydrogen, hydrolysis of an isocyanate with formula:

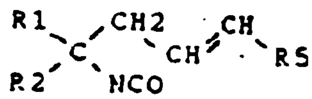

(II.1)

- to obtain a benzylamine of formula (I.2), corresponding to formula (I) in which R3 is a methyl radical and R4 is hydrogen,
  i) reduction of an isocyanate of formula (II.1) using a metal hydride, or
  ii) acylation of a benzylamine (I.1) with formic acid in the presence of carbonyl-diimidazole, to give the intermediate compound

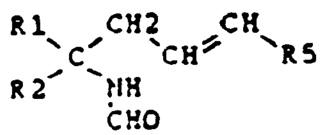

followed by reduction of this intermediate compound with a metal hydride,

- to obtain a benzylamine of formula (I.2), corresponding to formula (I) in which R3 is other than hydrogen and R4 is hydrogen
  i) alkylation of a benzylamine of formula (I.1) using a halide of formula Z1R3 in which R3 is other than hydrogen and Z1 is chlorine, bromine or iodine, or
  ii) in a first stage, acylation of a benzylamine (I.1) using an acylating agent of formula (R6-CO)nZ2, in which R6 is the immediately lower homologue of R3 (R3 = -CH2R6), and Z2 is either hydroxyl, or bromine or chlorine when n = 1, and is oxygen when n = 2, to obtain an intermediate carboxamide of formula

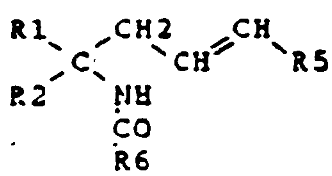

(II.2.1)

  which is reduced by a metal hydride in a second stage,

- to obtain a benzylamine of formula (I.3) in which both R3 and R4 are methyl radicals, demethylation of a benzylamine (I.1) by reaction with formaldehyde and formic acid in accordance with the Eschweiler-Clarke reaction, or with formaldehyde in the presence of a reducing agent,
- to obtain a benzylamine of formula (I.3), corresponding to formula (I), in which R3 is not hydrogen and R4 is neither hydrogen nor a methyl radical,
  i) in a first stage, acylation of a benzylamine of formula (I.2) in which R3 is other than hydrogen, using an acylating agent of formula R7COZ5 in which R7 is the immediately lower homologue of

R4 (R4 = -CH2R7) and Z5 is bromine or chlorine, to obtain a carboxamide disubstituted on the nitrogen atom, of formula

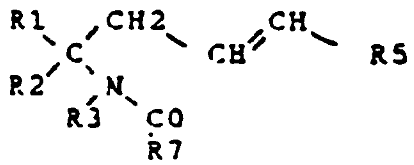

(II.2)

which is reduced in a second stage using a metal hydride,
ii) or reaction of an organomagnesium reagent of formula R2MgZ3, where Z5 is a halogen, with a nitrile of formula

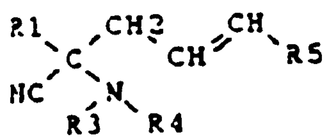

(II.3)

- to obtain a benzylamine of formula (I.3) corresponding to formula (I) in which R3 is not hydrogen and R4 is a methyl radical, N-methylation of a benzylamine of formula (I.2), in which R3 is a lower alkyl or alkenyl radical, using formic aldehyde in the presence of a reducing agent such as a metal or organometallic hydride.
- to obtain an addition salt with an acid, salification of the benzylamine with an acid, and to obtain an optically active form, resolution of a racemic.

2. Process conforming to Claims 1 or 2, characterized in that R1 is a phenyl group.

3. Process conforming to Claims 1 or 2, characterized in that R5 is a phenyl group.

4. Process conforming to any of Claims 1 to 3, characterized in that R2 is an ethyl radical.

5. Process conforming to any of Claims 1 to 4,, characterized in that R3 is hydrogen or a methyl radical and R4 is hydrogen or a methyl radical.

6. Process conforming to Claim 1, characterize4d in that at least one of R1 and R5 is a substituted phenyl group, and R3 and R4, whether identical or different, are hydrogen or a lower alkyl radical, but R3 and R4 are not both hydrogen.

7. Process for the preparation of a medicament, notably a psychotropic product, characterized in that it consists in mixing a benzylamine or one of its salts, as defined in Claims 1 to 5, together with a pharmaceutical excipient.

8. Process for the prepatration of a medicament that is psychotropic and active upon the urinary tract, characterized in that it consists in mixing a benzylamine or one of its salts, as defined in Claim 6, together with a pharmaceutical excipient.

9. Process for the preparation of intermediate compounds with the following formulae:

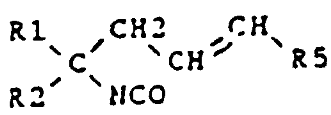

(II.1)

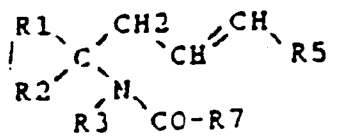 (II.2)

or

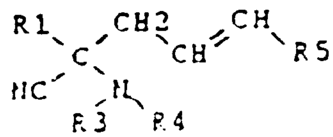 (II.3)

in which R1 to R5 are as indicated in Claim 1, R3 and R4 are other than hydrogen in the case of the nitriles (II.3), and R7 is the immediately lower homologue of R4, characterized in that it consists of the following:

- to prepare the compounds (II.1), reaction of a starting compound of formula (VIII) R1-CH2-W in which R1 is as defined for the benzylamines (I) and W is a nitrile (-CN) or carboxy (-COOH) radical, with an alkyl halide of formula R2Z6, R2 being as defined for the compounds (I) and Z6 being a halogen, to obtain an acid of formula (VI) when W is a carboxy radical

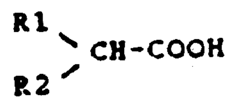

and, when W is a nitrile, a nitrile of formula (VII)

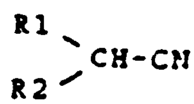

that is hydrolysed to the acid of formula VI, followed by alkylation of the acid (VI) by an alkenyl halide (V) of formula Z7-CH2-CH=CH-R5 in which Z7 is a halogen and R5 is as defined for the benzylamines (I), to obtain an unsaturated chain acid (III) that is transformed to an isocyanate (II.1) by a Curtius reaction,
- to prepare the compounds (II.2),

i) acylation of a benzylamine with formula

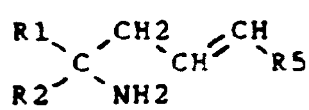 (I.1)

by means of a reagent with formula (R6CO)nZ2, in which R6 is the carbonated homologue directly below R3 (R3 = -CH2-R6) and in which Z2 is a halogen or hydroxyl when n = 1 and Z2 is oxygen when n = 2, to obtain an intermediate carboxamide (II.2.1),

ii) acylation of a benzylamine with formula

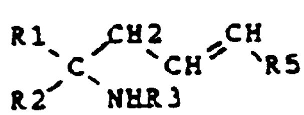

by an acylation agent with formula R7CO Z5, in which R7 is a lower alkyl or alkenyl radical and Z5 is bromine or chlorine, to obtain an intermediate carboxamide (II.2 ),
- to prepare the compounds (II.3), reaction of an aldehyde of formula (XI) R1-CHO with an amine of

formula R3-NH-R4 and an alkali metal cyanide to give an aminonitrile with formula

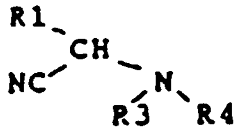

(X)

in which R4 and R3 are as defined for the benzylamines (I) but not hydrogen, and then alkylation of the aminonitrile (X) by an alkenyl halide (V).

**10.** Use of a benzylamine as defined in Claims 1 to 5, or one of its therapeutically acceptable salts, to obtain a medicament that is psychotropic.

**11.** Use of a benzylamine as defined in Claim 6, or one of its therapeutically acceptable salts, to obtain a medicament that is psychotropic and active upon the urinary tract.

**12.** Use of an intermediate compound as prepared in Claim 9, as an intermediate in the synthesis of benzylamines.

**Claims for the following Contracting State : GR**

**1.** Disubstituted benzylamines corresponding to the formula

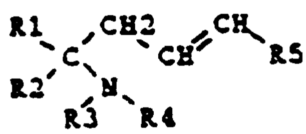

(I)

in which :
- R1 is a phenyl group, possibly mono- or disubstituted by halogen atoms or by lower alkyl, lower haloalkyl or lower alkoxy radicals,
- R2 is a lower alkyl radical,
- R3 and R4, which may be identical or different, are hydrogen, or lower alkyl or lower alkenyl radicals,
- R5 is a phenyl group, possibly mono-, di- or trisubstituted by halogen atoms or by lower alkoxy radicals,

their addition salts with acids and their optically active forms.

**2.** Benzylamines conforming to Claim 1 characterized in that R1 is a phenyl group.

**3.** Benzylamines conforming to Claims 1 or 2, characterized in that R5 is a phenyl group.

**4.** Benzylamines conforming to any of Claims 1 to 3, characterized in that R2 is an ethyl radical.

**5.** Benzylamines conforming to any of Claims 1 to 4, characterized in that R3 is hydrogen or a methyl radical and R4 is hydrogen or a methyl radical.

**6.** Benzylamine conforming to Claim 1, characterized in that at least one of R1 and R5 is a substituted phenyl group, and R3 and R4, whether identical or different, are hydrogen or a lower alkyl radical, but R3 and R4 are not both hydrogen.

**7.** Process for the preparation of benzylamines conforming to formula (I), characterized in that it consists of the following:
- to obtain a benzylamine of formula (I.1), corresponding to formula (I), in which R3 and R4 are hydrogen, hydrolysis of an isocyanate with formula:

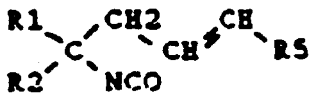

(II.1)

- to obtain a benzylamine of formula (I.2), corresponding to formula (I) in which R3 is a methyl radical and R4 is hydrogen,

i) reduction of an isocyanate of formula (II.1) using a metal hydride, or

ii) acylation of a benzylamine (I.1) with formic acid in the presence of carbonyl-diimidazole, to give the intermediate compound

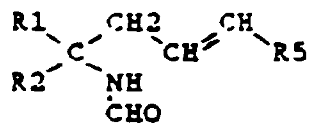

followed by reduction of this intermediate compound with a metal hydride,

- to obtain a benzylamine of formula (I.2), corresponding to formula (I) in which R3 is other than hydrogen and R4 is hydrogen

i) alkylation of a benzylamine of formula (I.1) using a halide of formula Z1R3 in which R3 is other than hydrogen and Z1 is chlorine, bromine or iodine, or

ii) in a first stage, acylation of a benzylamine (I.1) using an acylating agent of formula (R6-CO)nZ2, in which R6 is the immediately lower homologue of R3 (R3 = -CH2R6), and Z2 is either hydroxyl, or bromine or chlorine when n = 1, and is oxygen when n = 2, to obtain an intermediate carboxamide of formula

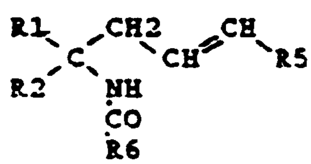

(II.2.1)

which is reduced by a metal hydride in a second stage,

- to obtain a benzylamine of formula (I.3) in which both R3 and R4 are methyl radicals, demethylation of a benzylamine (I.1) by reaction with formaldehyde and formic acid in accordance with the Eschweiler-Clarke reaction, or with formaldehyde in the presence of a reducing agent,

- to obtain a benzylamine of formula (I.3), corresponding to formula (I), in which R3 is not hydrogen and R4 is neither hydrogen nor a methyl radical,

i) in a first stage, acylation of a benzylamine of formula (I.2) in which R3 is other than hydrogen, using an acylating agent of formula R7COZ5 in which R7 is the immediately lower homologue of R4 (R4 = -CH2R7) and Z5 is bromine or chlorine, to obtain a carboxamide disubstituted on the nitrogen atom, of formula

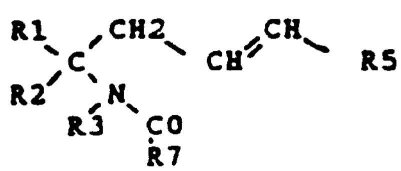

(II.2)

which is reduced in a second stage using a metal hydride,

ii) or reaction of an organomagnesium reagent of formula R2MgZ3, where Z5 is a halogen, with a nitrile of formula

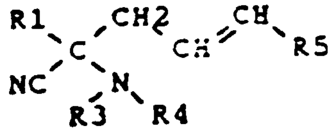

(II.3)

- to obtain a benzylamine of formula (I.3) corresponding to formula (I) in which R3 is not hydrogen and R4 is a methyl radical, N-methylation of a benzylamine of formula (I.2), in which R3 is a lower alkyl or alkenyl radical, using formic aldehyde in the presence of a reducing agent such as a metal or organometallic hydride;
- to obtain an addition salt with an acid, salification of the benzylamine with an acid, and to obtain an optically active form, resolution of a racemic.

8. Process for the preparation of a medicament, notably a psychotropic product, characterized in that it consists of mixing a benzylamine or one of its salts, as defined in Claims 1 to 5, together with a pharmaceutical excipient.

9. Process for the preparation of a medicament that is psychotropic and active upon the urinary tract, characterized in that it consists in mixing a benzylamine or one of its salts, as defined in Claim 6, together with a pharmaceutical excipient.

10. Use of a benzylamine as defined in Claims 1 to 5, or one of its therapeutically acceptable salts, to obtain a psychotropic medicament.

11. Use of a benzylamine as defined in Claim 6, or one of its therapeutically acceptable salts, to obtain a medicament that is psychotropic and active upon the urinary tract.

12. Intermediate compounds of the following formulae:

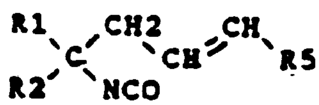

(II.1)

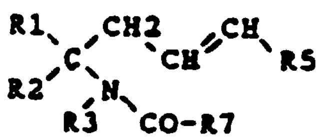

(II.2)

or

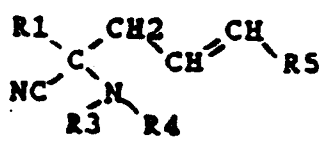

or

(II.3)

in which R1 to R5 are as indicated in Claim 1, R3 and R4 are other than hydrogen in the case of the nitriles (II.3), and R7 is the immediately lower homologue of R4.

13. Process for the preparation of the intermediate compounds of Claim 12, characterized in that it consists of the following:
- to prepare the compounds (II.1), reaction of a starting compound of formula (VIII) R1-CH2-W in which R1 is as defined for the benzylamines (I) and W is a nitrile (-CN) or carboxy (-COOH) radical, with an alkyl halide of formula R2Z6, R2 being as defined for the compounds (I) and Z6 being a halogen, to obtain an acid of formula (VI) when W is a carboxy radical

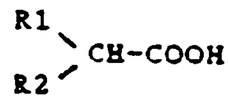

and, when W is a nitrile, a nitrile of formula (VII)

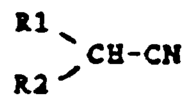

that is hydrolysed to the acid of formula VI, followed by alkylation of the acid (VI) by an alkenyl halide (V) of formula Z7-CH2-CH=CH-R5 in which Z7 is a halogen and R5 is as defined for the benzylamines (I), to obtain an unsaturated chain acid (III) that is transformed to an isocyanate (II.1) by a Curtius reaction,

- to prepare the compounds (II.2),

    i) acylation of a benzylamine with formula

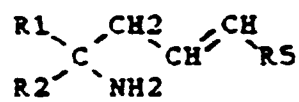

(I.1)

    by means of a reagent with formula (R6CO)nZ2, in which R6 is the carbonated homologue directly below R3 (R3 = -CH2-R6) and in which Z2 is a halogen or hydroxyl when n = 1 and Z2 is oxygen when n = 2, to obtain an intermediate carboxamide (II.2.1),

    ii) acylation of a benzylamine with formula

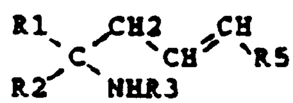

    by an acylation agent with formula R7CO Z5, in which R7 is a lower alkyl or alkenyl radical and Z5 is bromine or chlorine, to obtain an intermediate carboxamide (II.2.2),

- to prepare the compounds (II.3), reaction of an aldehyde of formula (XI) R1-CHO with an amine of formula R3-NH-R4 and an alkali metal cyanide to give an aminonitrile with formula

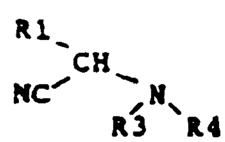

(X)

in which R4 and R3 are as defined for the benzylamines (I) but not hydrogen, and then alkylation of the aminonitrile (X) by an alkenyl halide (V).